# EUROPEAN PATENT APPLICATION

(11) **EP 4 656 648 A1**
(43) Date of publication of application: **03.12.2025**
(21) Application number: 24382585.8
(22) Date of filing: 31.05.2024
(51) Int. Cl.: C07H 15/12, A61P 31/00, A61K 39/00, A61P 35/00

(54) **IMMUNOMODULATORY THIOUREA AND UREA CARBOHYDRATE COMPOUNDS AND USES THEREOF**

(71) Applicant: Consejo Superior De Investigaciones Científicas, 41013 Sevilla (ES); National University Corporation Hokkaido University, Hokkaido 060-0808 (JP); National University Corporation Kanazawa University, Kanazawa-shi, Ishikawa 920-1192 (JP); Universidad de Sevilla, 41013 Sevilla (ES); Academia Sinica, Taipei City 11529 (TW)
(72) Inventor: García Fernández, José Manuel, Sevilla (ES); Parejas Barranco, Almudena, Sevilla (ES); Ortiz Mellet, María del Carmen, Sevilla (ES); González Cuesta, Manuel, Sevilla (ES); Maenaka, Katsumi, Sapporo (JP); Guillén Poza, Pablo Adrián, Sapporo (JP); Furukawa, Atsushi, Kanazawa (JP); Chang, Ya-Jen, Taipei (TW); Chuan-Ying Lai, Alan, Taipei (TW)
(74) Representative: Pons IP

(57) **Abstract**

The present invention relates to new immunomodulatory thiourea and urea carbohydrate compounds of formula I, pharmaceutical compositions comprising them and the use thereof, mainly as vaccine adjuvants, in the modulation of immune response in a subject, in the treatment and/or prophylaxis of infectious diseases and in the treatment of cancer.

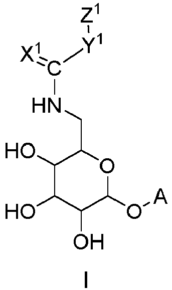

## Description

The present invention relates to new immunomodulatory thiourea and urea carbohydrate compounds, pharmaceutical compositions comprising then and the use thereof, for example, as vaccine adjuvants. Therefore, the present invention belongs to the medicine field.

### BACKGROUND ART

The macrophage-inducible C-type lectin (Mincle) is a transmembrane C-type lectin receptor (CLR) expressed on immune system cells such as macrophages and dendritic cells (DCs). The activation of Mincle can initiate a signaling cascade, leading to the production of pro-inflammatory cytokines, chemokines and growth factors, thereby leading to an immunostimulating effect. In this context, the ligand of Mincle is regarded as a promising adjuvant to be co-administered with vaccine antigens. Over the past decade, many glycolipids have been isolated or synthesized as Mincle activators. Representative members are α,α'-trehalose glycolipids, which are a diverse family of long-chain fatty acid diesters of the disaccharide α,α'-trehalose. Among this class of compounds are the trehalose dimycolates (TDMs), originally isolated from the cell wall of *M. tuberculosis.* TDMs and other trehalose diesters (TDEs) and trehalose monoesters (TMEs) stimulate the innate and immune response upon binding to Mincle. Yet, most of them are far too reactogenic for human vaccine applications. For instance, TDM is highly toxic so cannot be used as a therapeutic agent. TDM also comprises a complex mixture of compounds and is difficult to synthesize.

Accordingly, alternative Mincle agonists are needed to advance new vaccines and therapeutics in the areas of infection disease, as well as autoimmunity and cancer, preferably compounds with high activity and low toxicity that are simple to prepare in high purity.

The C-Type Lectin Receptor (CLR) family contains many diverse members that have different substrates, adaptor proteins, downstream signaling pathways, and cell type expression profiles. While some members of this family are purely phagocytic receptors, most are known to activate intracellular signaling networks that induce functional changes within the cell, such as modulation of transcription, endocytosis/phagocytosis, and/or cell adhesion and migration. One of the best characterized of these signaling cascade-inducing receptors is Mincle (CLEC4e).

Mincle must be coupled to an immunoreceptor tyrosine-based activation motif (ITAM)-containing adaptor molecule, the γ subunit of the immunoglobulin Fc receptor (FcRy), to initiate downstream signaling, and ligand binding is Ca²⁺-dependent. However, the ligands for the Mincle receptor are distinct from those for other CLR receptors such as Dectin-1 and Dectin-2, and include the mycobacterial glycolipid trehalose-6,6'-dimycolate (TDM), its synthetic analog trehalose dibehenate (TDB), and many α-mannose-containing lipids found in various fungi and *Candida* strains. These ligands can also be found in many pathogenic organisms including *M tuberculosis,* S. *mansoni,* and *Trubrum.* Additionally, the use of TDB and subsequent Mincle-induced signaling was shown to be necessary for the Th1/Th17 adjuvanting properties in some pre-clinical vaccine models.

Using an approach described herein for the identification of novel Micle agonists, that uses a nuclear factor of activated T-cell (NFAT)-driven green florescent protein (GFP) reporter strain that expresses Mincle and FcRy, new biologically active compounds have been identified that may serve as a basis for a new class of Th1/Th17-inducing adjuvants.

Carbohydrate derivatives bearing thiourea or urea groups at their primary positions have been previously reported for different purposes, but no evidence of a biological activity involving a mechanism of action implying Mincle agonism has been neither shown nor suggested. For instance, trehalose derivatives connected through urea or thiourea groups were prepared to investigate their conformational and supramolecular properties (Rodriguez-Lucena et al., J. Org. Chem. 2008, 73, 2967-2979, and J. Org. Chem. 2009, 74, 2997-3008) and as core platforms to build polycationic compounds for nucleic acid delivery (Jiménez-Blanco et al. Chem. Commun., 2016, 52, 10117-10120; Carbajo-Gordillo et al. Biomacromolecules 2020, 21, 5173-5188, and Macromol. Rapid Commun. 2022, 2200145). 6,6'-Dideoxy-6,6'-di-(*N*',*N*'-dioctylureido)-α,α'-trehalose has been synthesized and assayed as an inhibitor of the *Mycobacterium* mycolyltransferase ag85 in search for new antibiotics, but it was discarded in view of its poor activity. 6,6'-Dideoxy-6,6'-di-(*N*',*N*'-methylureido)sucrose (Almquist and Reist, J. Med. Chem. 1977, 20, 1426-1450) and methyl 6-deoxy-6-*N*'-(2-chloroethyl)ureido-α-D-glucopyranoside (Sosnovsky and Rao, J. Pharm. Sci. 1991, 80, 693-699) have also been reported as synthetic intermediates of nitrosourea derivatives with anticancer activity.

The present disclosure encompasses an insight that certain mono and oligosaccharides bearing different substituents at their primary positions connected through thiourea or urea groups are surprisingly useful as Mincle activators and bear strong potential for particular therapeutic applications.

### SUMMARY OF THE INVENTION

The inventors of the present invention have found that the compounds of formula I are capable of acting as agonists of the Mincle receptor in immune system cells and may represent a new class of Th1/Th17-inducing adjuvants for vaccines targeting viral, bacterial and fungal pathogens causing a significant burden of disease in humans, certain cancers, and autoimmune diseases. In addition, the compounds of the present invention are capable of eliciting an inflammatory response and of acting as agonists of the Mincle receptor in immune system cells *in vitro* and *in vivo.*

Then, it is described herein a class of compounds that may act as Th1 and/or Th17-inducing adjuvants, which may be useful for targeting diseases of significant medical burden for which no effective vaccine is available. The compounds may also be useful in the treatment of cancer and autoimmune diseases.

The first aspect of the invention refers to a compound of formula I (compound of the invention): 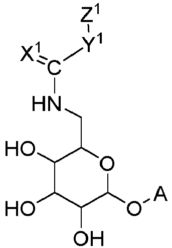 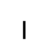 or a pharmaceutically acceptable salt thereof, wherein:
A is A¹, A², A³ or A⁴;
A¹ is selected from C₁-C₂₀ aliphatic, C₃-C₂₀ cycloaliphatic, C₅-C₁₄ aryl, 3- to 14-membered heterocyclyl comprising 1 to 3 heteroatoms selected from N, O, and S, wherein each A¹ is optionally substituted with one or more A^{a};
each A^{a} is independently selected from halogen, -N₃, -A^{b}, -OA^{b}, -SA^{b}, -NHA^{b}, -C(O)-A^{b}, -OC(O)A^{b}, -NHC(O)A^{b}, -C(O)NHA^{b}, -NHC(O)NHA^{b} and -NHC(S)NHA^{b};
each A^{b} is selected from H, C₁-C₂₀ aliphatic, C₃-C₂₀ cycloaliphatic, C₅-C₁₄ aryl, 3- to 14-membered heterocyclyl comprising 1 to 3 heteroatoms selected from N, O, and S and 4- to 14-membered heteroaryl comprising 1 to 3 heteroatoms selected from N, O, and S;
A², A³ and A⁴ are: 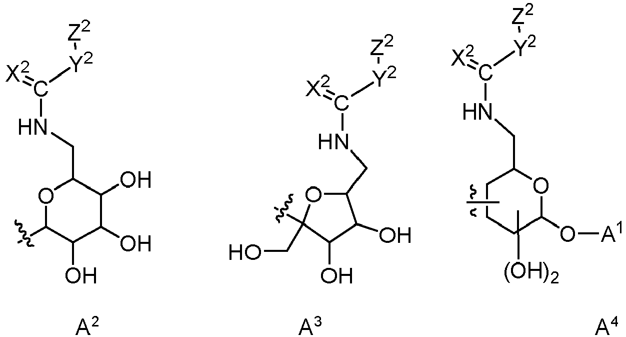
X¹ and X² are independently selected from S, and O;
Y' and Y² are independently selected from -N(R^{a})-, -N(R^{a})-C(=O)- and -N(R^{a})-S(=O)₂,
each R^{a} is independently selected from H, C₁-C₂₅ aliphatic, C₃-C₂₀ cycloaliphatic, C₅-C₁₄ aryl, 3- to 14-membered heterocyclyl comprising 1 to 3 heteroatoms selected from N, O, and S, wherein each R^{a} is optionally substituted with one or more R^{b};
each R^{b} is independently selected from halogen, -N₃, -R^{c}, -OR^{c}, -SR^{c}, -NHR^{c}, -C(O)-R^{c}, -OC(O)R^{c}, -NHC(O)R^{c}, -C(O)NHR^{c}, -NHC(O)NHR^{c} and NHC(S)NHR^{c};
each R^{c} is independently selected from H or optionally substituted C₁-C₂₀ aliphatic, C₃-C₂₀ cycloaliphatic, C₅-C₁₄ aryl, 3- to 14-membered heterocyclyl comprising 1 to 3 heteroatoms selected from N, O, and S, and 4- to 14-membered heteroaryl comprising 1 to 3 heteroatoms selected from N, O, and S;
Z¹ and Z² are independently selected from C₄-C₂₅ aliphatic, C₃-C₂₀ cycloaliphatic, C₅-C₁₄ aryl, 3- to 14-membered heterocyclyl comprising 1 to 3 heteroatoms selected from N, O, and S, wherein Z¹ and Z² are optionally independently substituted with one or more Z³;
each Z³ is independently selected from halogen, -N₃, -R^{d}, -OR^{d}, -OC(O)R^{d}, -SR^{e}, - NHR^{e}, -C(O)-R^{e}, -NHC(O)R^{e}, -C(O)NHR^{e}, -NHC(O)NHR^{e} and NHC(S)NHR^{e};
each R^{d} is independently selected from optionally substituted C₂-C₂₀ aliphatic, C₃-C₂₀ cycloaliphatic, C₅-C₁₄ aryl, 3- to 14-membered heterocyclyl comprising 1 to 3 heteroatoms selected from N, O, and S, and 4- to 14-membered heteroaryl comprising 1 to 3 heteroatoms selected from N, O, and S;
each R^{e} is independently selected from H or optionally substituted C₁-C₂₀ aliphatic, C₃-C₂₀ cycloaliphatic, C₅-C₁₄ aryl, 3- to 14-membered heterocyclyl comprising 1 to 3 heteroatoms selected from N, O, and S, and 4- to 14-membered heteroaryl comprising 1 to 3 heteroatoms selected from N, O, and S;
for use as vaccine adjuvant.

Also, the present invention refers to compounds of formula I 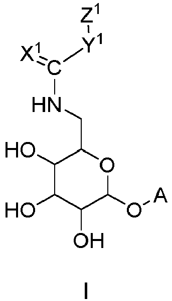 or a pharmaceutically acceptable salt thereof, wherein
A, X¹, Y¹ and Z¹ are as descried above, provided that:
when A is A²,each X¹ and X² is O, each Y¹ and Y² is -N(R^{a})-, and each R^{a} is H, then each Z¹ and Z² is not selected from -(CH₂)₇CH₃ and -(CH₂)₈CH₃, and
when A is Me, X¹ is S, Y' is -N(R^{a})-, and R^{a} is H, then Z¹ is selected from C₆-C₂₅ aliphatic, C₃-C₂₀ cycloaliphatic, C₅-C₁₄ aryl, 3- to 14-membered heterocyclyl comprising 1 to 3 heteroatoms selected from N, O, and S, wherein Z¹ is optionally substituted with one or more Z³.

Also, the present invention refers to methods of preparing a compound of formula I, to pharmaceutical compositions comprising the compounds of formula I, and to medical uses thereof.

### DETAILED DESCRIPTION OF THE INVENTION

In a first aspect, the present invention relates to compounds of formula I 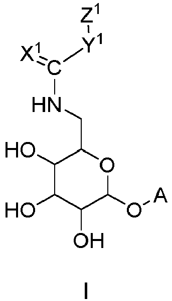 or a pharmaceutically acceptable salt thereof, as descried above for use as vaccine adjuvant.

In some embodiments, A is A¹, and the compound is of formula la: 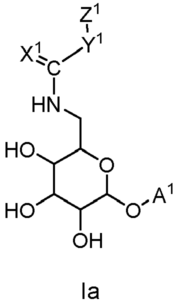 or a pharmaceutically acceptable salt thereof, wherein A¹, X¹, Y¹, and Z¹ are as described previously.

In some embodiments, A¹ in a compound of formula Ia is Me (Me and -CH₃ both refer to a methyl group and are used interchangeably in the present invention.), and the compound is of formula laa: 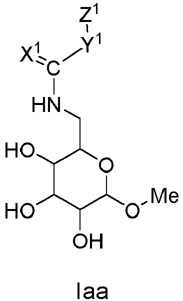 or a pharmaceutically acceptable salt thereof, wherein X¹, Y¹, and Z¹ are as described previously.

In some embodiments, A is A², and the compound is of formula Ib: 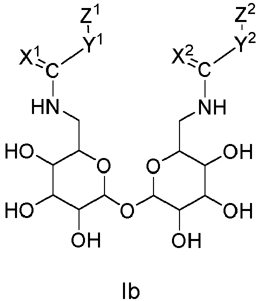 or a pharmaceutically acceptable salt thereof, wherein X¹, X², Y¹, Y², Z¹, and Z² are as described previously, both singly and in combination, provided that when each X¹ and X² is O, each Y¹ and Y² is -N(R^{a})-, and each R^{a} is H, then each Z¹ and Z² is not -(CH₂)₇CH₃.

In some embodiments, A is A³, and the compound is of formula Ic: 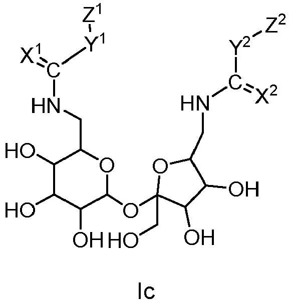 or a pharmaceutically acceptable salt thereof, wherein X¹, X², Y¹, Y², Z¹, and Z² are as described previously, both singly and in combination.

In some embodiments, A is A⁴, and an oligosaccharide is of formula Id: 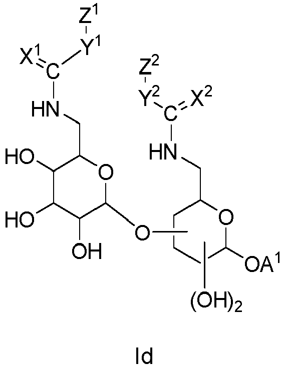 or a pharmaceutically acceptable salt thereof, wherein X¹, X², Y¹, Y², Z¹, Z² and A¹ are as described previously, both singly and in combination.

In some embodiments, A¹ in a compound of formula Id is Me, and the compound is of formula Ida: 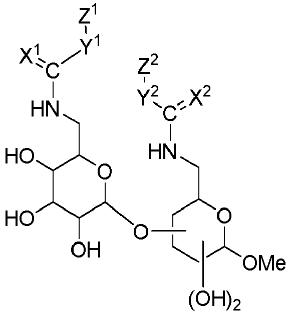 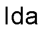 or a pharmaceutically acceptable salt thereof, wherein X¹, X², Y¹, Y², Z¹, and Z² are as described previously, both singly and in combination.

As described generally herein X¹ and X² are each independently selected from S, and O. In some embodiments, X¹ and X² are S. In some embodiments, X¹ and X² are O.

As described generally herein Y¹ and Y² are each independently selected from -N(R^{a})-, -N(R^{a})-C(=O)- and -N(R^{a})-S(=O)₂-. In some embodiments, each of Y¹ and Y² is - N(R^{a})-, and each R^{a} is independently selected from H, C₁-C₂₅ aliphatic, C₃-C₂₀ cycloaliphatic, C₅-C₁₄ aryl, and 3- to 14-membered heterocyclyl comprising 1 to 3 heteroatoms selected from N, O, and S, wherein each R^{a} is optionally substituted with one or more R^{b}, R^{b} as described previously.

In some embodiments, each of Y¹ and Y² is -N(R^{a})-C(=O)-, and each R^{a} is independently selected from H, C₁-C₂₅ aliphatic, C₃-C₂₀ cycloaliphatic, C₅-C₁₄ aryl, and 3- to 14-membered heterocyclyl comprising 1 to 3 heteroatoms selected from N, O, and S, wherein each R^{a} is optionally substituted with one or more R^{b}, R^{b} as described previously.

In some embodiments, each of Y¹ and Y² is -N(R^{a})-S(=O)₂-, and each R^{a} is independently selected from H, C₁-C₂₅ aliphatic, C₃-C₂₀ cycloaliphatic, C₅-C₁₄ aryl, and 3- to 14-membered heterocyclyl comprising 1 to 3 heteroatoms selected from N, O, and S, wherein each R^{a} is optionally substituted with one or more R^{b}, R^{b} as described previously.

As described generally herein R^{a} is each independently selected from H, C₁-C₂₅ aliphatic, C₃-C₂₀ cycloaliphatic, C₅-C₁₄ aryl, 3- to 14-membered heterocyclyl comprising 1 to 3 heteroatoms selected from N, O, and S, wherein each R^{a} is optionally substituted with one or more R^{b}. In some embodiments, each R^{a} is H. In some embodiments, R^{a} is each independently selected from H, C₁-C₂₅ aliphatic, C₃-C₂₀ cycloaliphatic, C₅-C₁₄ aryl, 3- to 14-membered heterocyclyl comprising 1 to 3 heteroatoms selected from N, O, and S, wherein each R^{a} is optionally substituted with one or more R^{b}. In some embodiments, R^{a} is each independently selected from C₁-C₂₅ aliphatic, wherein each R^{a} is optionally substituted with one or more R^{b}. In some embodiments, each R^{a} is Me, R^{b} is as described previously.

As described generally herein R^{b} is each independently selected from halogen, -N₃, - R^{c}, -OR^{c}, -SR^{c}, -NHR^{c}, -C(O)-R^{c}, -OC(O)R^{c}, -NHC(O)R^{c}, -C(O)NHR^{c}, -NHC(O)NHR^{c} and NHC(S)NHR^{c}. In some embodiments, R^{b} is each independently selected from - R^{c}, R^{c} as described previously.

As described generally herein R^{c} is each independently selected from H or optionally substituted C₁-C₂₀ aliphatic, C₃-C₂₀ cycloaliphatic, C₅-C₁₄ aryl, 3- to 14-membered heterocyclyl comprising 1 to 3 heteroatoms selected from N, O, and S, and 4- to 14-membered heteroaryl comprising 1 to 3 heteroatoms selected from N, O, and S. In some embodiments, is each independently selected from H or optionally substituted C₁-C₂₀ aliphatic. In some embodiments, R^{c} is each independently selected from H or optionally substituted C₁-C₂₀ aliphatic. In some embodiments, R^{c} is each independently selected from optionally substituted C₁-C₂₀ aliphatic.

As described generally herein, Z¹ and Z² are each independently selected from C₄-C₂₅ aliphatic, C₃-C₂₀ cycloaliphatic, C₅-C₁₄ aryl, 3- to 14-membered heterocyclyl comprising 1 to 3 heteroatoms selected from N, O, and S, wherein Z¹ and Z² are optionally independently substituted with one or more Z³. In some embodiments, Z¹ and Z² are each independently selected from C₄-C₂₅ aliphatic, wherein Z¹ and Z² are optionally independently substituted with one or more Z³, Z³ as described previously.

As described generally herein Z³ is each independently selected from halogen, -N₃, - R^{d}, -OR^{d}, -OC(O)R^{d}, -SR^{e}, -NHR^{e}, -C(O)-R^{e}, -NHC(O)R^{e}, -C(O)NHR^{e}, -NHC(O)NHR^{e} and NHC(S)NHR^{e}. In some embodiments, Z³ is each independently selected from optionally substituted -R^{d}, -OR^{d}, -OC(O)R^{d} and -C(O)-R^{e}; R^{d} and R^{e} as described previously.

As described generally herein R^{d} is each independently selected from optionally substituted C₂-C₂₀ aliphatic, C₃-C₂₀ cycloaliphatic, C₅-C₁₄ aryl, 3- to 14-membered heterocyclyl comprising 1 to 3 heteroatoms selected from N, O, and S, and 4- to 14-membered heteroaryl comprising 1 to 3 heteroatoms selected from N, O, and S. In some embodiments, R^{d} is each independently selected from optionally substituted C₂-C₂₀ aliphatic.

As described generally herein R^{e} is each independently selected from H or optionally substituted C₁-C₂₀ aliphatic, C₃-C₂₀ cycloaliphatic, C₅-C₁₄ aryl, 3- to 14-membered heterocyclyl comprising 1 to 3 heteroatoms selected from N, O, and S, and 4- to 14-membered heteroaryl comprising 1 to 3 heteroatoms selected from N, O, and S. In some embodiments, R^{e} is each independently selected from H and optionally substituted C₁-C₂₀ aliphatic.

The present invention includes all the described embodiments alone or in combination.

For example, in a preferred embodiment of compound of formula I, A is A², X¹ and X² are both S, Y¹ and Y² are both -N(R^{a})-, the R^{a} are identical and Z¹ and Z² are identical. In a more preferred embodiment, A is A², X¹ and X² are both S, Y¹ and Y² are both - N(R^{a})-, the R^{a} are identical and Z¹ and Z² are identical, wherein R^{a} is H and Z¹ and Z² are C₄-C₂₅ aliphatic.

In another more preferred embodiment, A is A², X¹ and X² are both S, Y¹ and Y² are both -N(R^{a})-, the R^{a} are identical and Z¹ and Z² are identical, wherein R^{a} is C₁-C₂₅ aliphatic and Z¹ and Z² are C₄-C₂₅ aliphatic.

In a preferred embodiment of compound of formula I, A is A², X¹ and X² are both O, Y¹ and Y² are both-N(R^{a})-, the R^{a} are identical and Z¹ and Z² are identical.

In a more preferred embodiment, A is A², X¹ and X² are both O, Y¹ and Y² are both - N(R^{a})-, the R^{a} are identical and Z¹ and Z² are identical, wherein R^{a} is H and Z¹ and Z² are C₄-C₂₅ aliphatic.

In another more preferred embodiment, A is A², X¹ and X² are both O, Y¹ and Y² are both -N(R^{a})-, the R^{a} are identical and Z¹ and Z² are identical, wherein R^{a} is C₁-C₂₅ aliphatic and Z¹ and Z² are C₄-C₂₅ aliphatic.

In a preferred embodiment, A is A¹ and A¹ is Me, X¹ is S, Y' is -N(R^{a})- and Z¹ is C₄-C₂₅ aliphatic, wherein R^{a} is H or C₁-C₂₅ aliphatic.

In a preferred embodiment, A is A¹' and A¹ is Me, X¹ is O, Y' is -N(R^{a})- and Z¹ is C₄-C₂₅ aliphatic, wherein R^{a} is H or C₁-C₂₅ aliphatic-

In another preferred embodiment, A is A³, X¹ and X² are both S, Y¹ and Y² are both - N(R^{a})-, the R^{a} are identical and Z¹ and Z² are identical.

In a more preferred embodiment, A is A³, X¹ and X² are both S, Y¹ and Y² are both - N(R^{a})-, the R^{a} are identical and Z¹ and Z² are identical, wherein R^{a} is H and Z¹ and Z² are C₄-C₂₅ aliphatic.

In a more preferred embodiment, A is A³, X¹ and X² are both S, Y¹ and Y² are both - N(R^{a})-, the R^{a} are identical and Z¹ and Z² are identical, wherein R^{a} is C₁-C₂₅ aliphatic and Z¹ and Z² are C₄-C₂₅ aliphatic.

In another preferred embodiment, A is A³, X¹ and X² are both O, Y¹ and Y² are both - N(R^{a})-, the R^{a} are identical and Z¹ and Z² are identical.

In a more preferred embodiment, A is A³, X¹ and X² are both O, Y¹ and Y² are both - N(R^{a})-, the R^{a} are identical and Z¹ and Z² are identical, wherein R^{a} is H and Z¹ and Z² are C₄-C₂₅ aliphatic.

In a more preferred embodiment, A is A³, X¹ and X² are both O, Y¹ and Y² are both - N(R^{a})-, the R^{a} are identical and Z¹ and Z² are identical, wherein R^{a} is C₁-C₂₅ aliphatic and Z¹ and Z² are C₄-C₂₅ aliphatic.

In another preferred embodiment, A is A⁴, wherein A¹ is Me, X¹ and X² are both S, Y¹ and Y² are both -N(R^{a})-, the R^{a} are identical and Z¹ and Z² are identical.

In a more preferred embodiment, A is A⁴, wherein A¹ is Me, X¹ and X² are both S, Y¹ and Y² are both -N(R^{a})-, the R^{a} are identical and Z¹ and Z² are identical, wherein R^{a} is H and Z¹ and Z² are C₄-C₂₅ aliphatic.

In a more preferred embodiment, A is A⁴, wherein A¹ is Me, X¹ and X² are both S, Y¹ and Y² are both -N(R^{a})-, the R^{a} are identical and Z¹ and Z² are identical, wherein R^{a} is C₁-C₂₅ aliphatic and Z¹ and Z² are C₄-C₂₅ aliphatic.

In another preferred embodiment, A is A⁴, wherein A¹ is Me, X¹ and X² are both O, Y¹ and Y² are both -N(R^{a})-, the R^{a} are identical and Z¹ and Z² are identical.

In a more preferred embodiment, A is A⁴, wherein A¹ is Me, X¹ and X² are both O, Y¹ and Y² are both -N(R^{a})-, the R^{a} are identical and Z¹ and Z² are identical, wherein R^{a} is H and Z¹ and Z² are C₄-C₂₅ aliphatic.

In a more preferred embodiment, A is A⁴, wherein A¹ is Me, X¹ and X² are both O, Y¹ and Y² are both -N(R^{a})-, the R^{a} are identical and Z¹ and Z² are identical, wherein R^{a} is C₁-C₂₅ aliphatic and Z¹ and Z² are C₄-C₂₅ aliphatic.

In some embodiments, the present disclosure provides a compound for use of Table 1.

| **Name** | **Structure** | **Formula Mw (g/mol)** |
|---|---|---|
| TT1 | 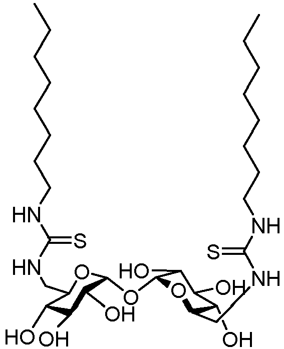 | C₃₀H₅₈N₄O₉S₂ 682.93 |
| TT2 | 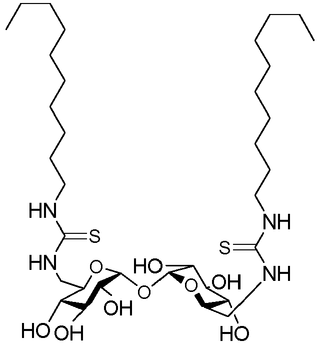 | C₃₄H₆₆N₄O₉S₂ 739.04 |
| TT3 | 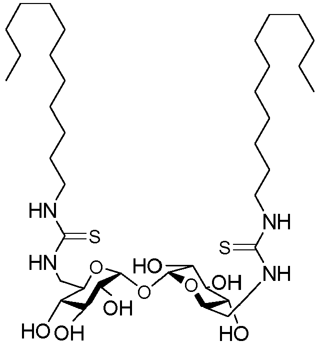 | C₃₈H₇₄N₄O₉S₂ 795.14 |
| TT4 | 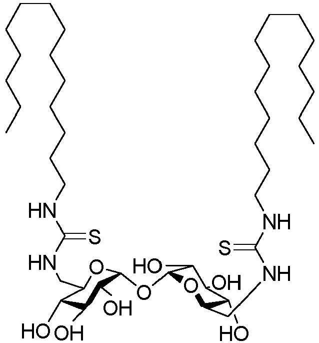 | C₄₂H₈₂N₄O₉S₂ 851.25 |
| TT5 | 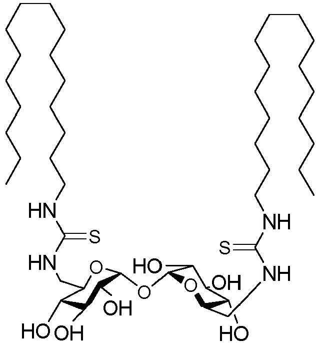 | C₄₆H₉₀N₄O₉S₂ 907.36 |
| TT6 | 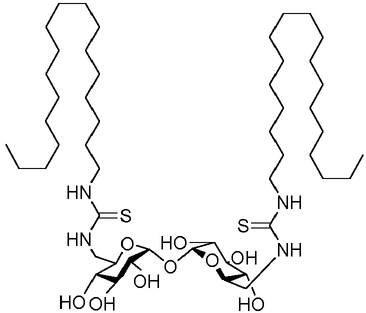 | C₅₀H₉₈N₄O₉S₂ 963.47 |
| TT7 | 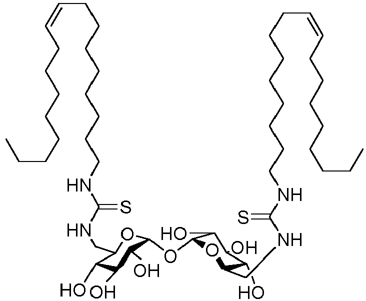 | C₅₀H₉₄N₄O₉S₂ 959.44 |
| TT8 | 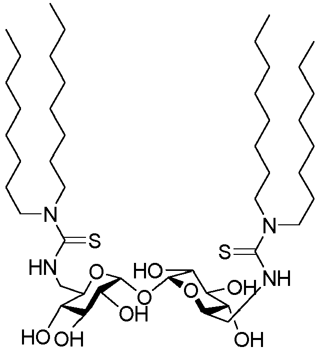 | C₄₆H₉₀N₄O₉S₂ 907.36 |
| TT9 | 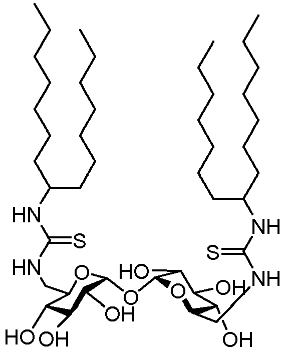 | C₄₄H₈₆N₄O₉S₂ 879.31 |
| TT10 | 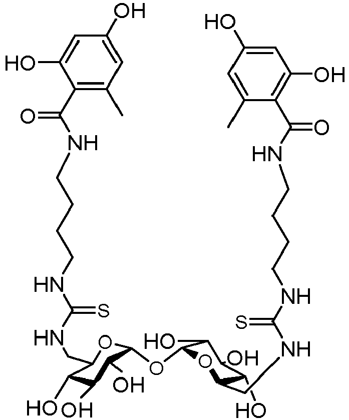 | C₃₈H₅₆N₆O₁₅S₂ 901.01 |
| TT11 | 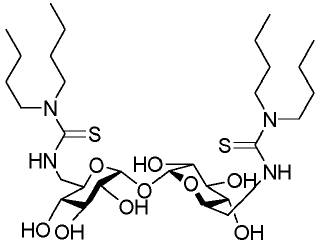 | C₃₀H₅₈N₄O₉S₂ 682.93 |
| TT12 | 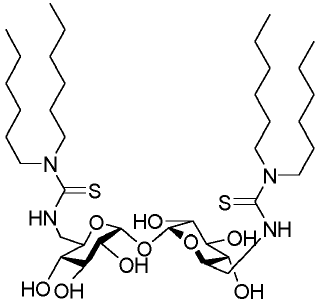 | C₃₈H₇₄N₄O₉S₂ 795.14 |
| TT13 | 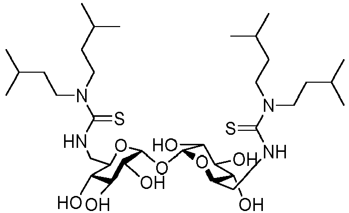 | C₃₄H₆₆N₄O₉S₂ 739.04 |
| TT14 | 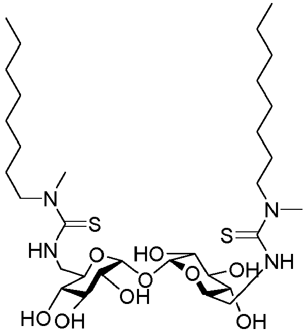 | C₃₂H₆₂N₄O₉S₂ 710.98 |
| TT15 | 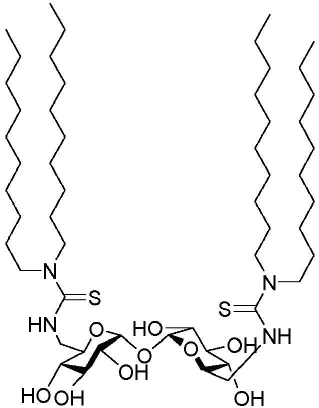 | C₅₄H₁₀₆N₄O₉S₂ 1019.58 |
| TU2 | 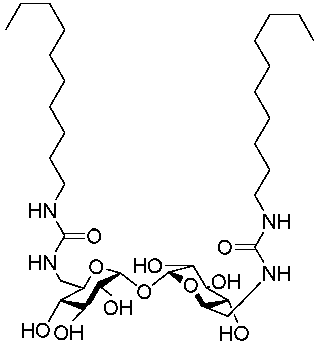 | C₃₄H₆₆N₄O₁₁ 706.91 |
| TU3 | 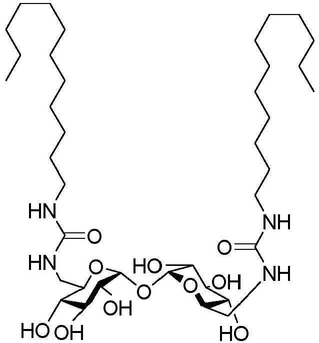 | C₃₈H₇₄N₄O₁₁ 763.02 |
| TU5 | 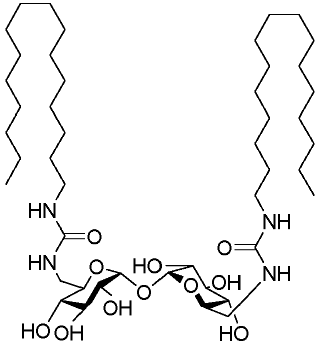 | C₄₆H₉₀N₄O₁₁ 875.24 |
| TU8 | 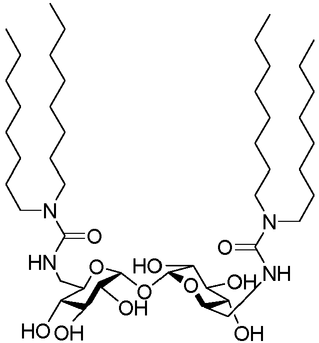 | C₄₆H₉₀N₄O₁₁ 875.24 |
| TU11 | 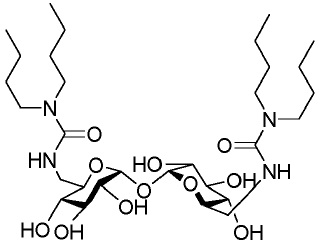 | C₃₀H₅₈N₄O₁₁ 650.81 |
| TU12 | 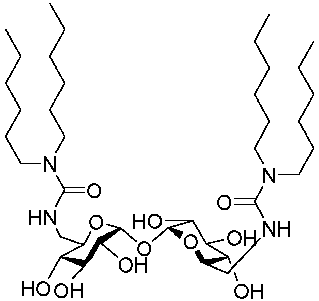 | C₃₈H₇₄N₄O₁₁ 763.02 |
| TU13 | 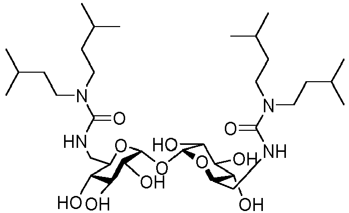 | C₃₄H₆₆N₄O₁₁ 706.91 |
| TU14 | 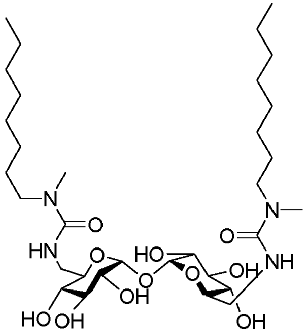 | C₃₂H₆₂N₄O₁₁ 678.86 |
| TU15 | 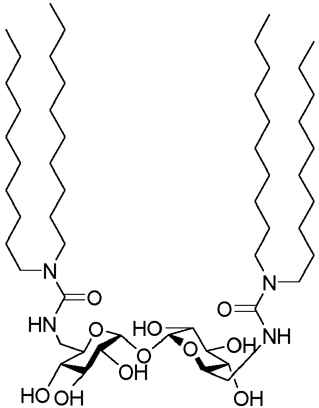 | C₅₄H₁₀₆N₄O₁₁ 987.45 |
| GT1 | 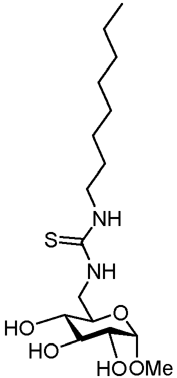 | C₁₆H₃₂N₂O₅S 364.50 |
| GT5 | 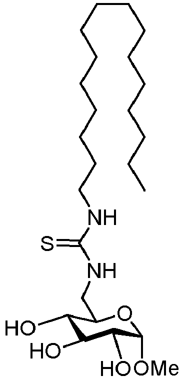 | C₂₄H₄₈N₂O₅S 476.72 |
| GT8 | 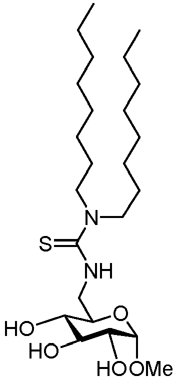 | C₂₄H₄₈N₂O₅S 476.72 |
| GU5 | 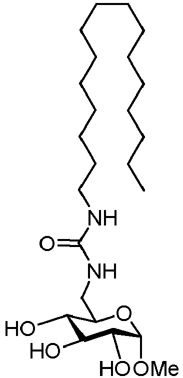 | C₂₄H₄₈N₂O₆ 460.66 |
| GU8 | 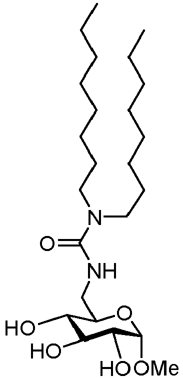 | C₂₄H₄₈N₂O₆ 460.66 |
| ST1 | 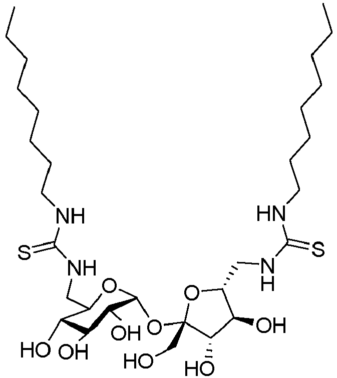 | C₃₀H₅₈N₄O₉S₂ 364.50 |
| ST5 | 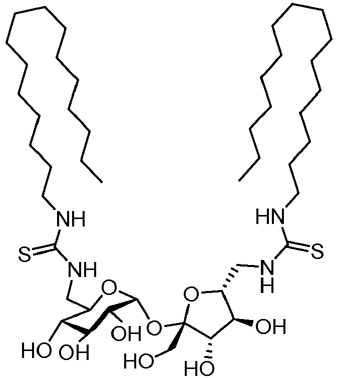 | C₄₆H₉₀N₄O₉S₂ 959.44 |
| ST8 | 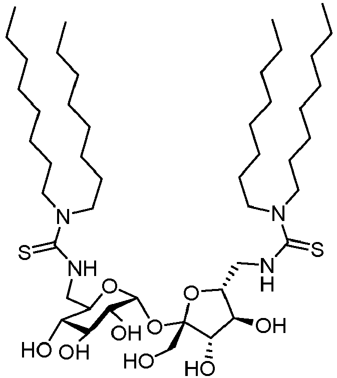 | C₄₆H₉₀N₄O₉S₂ 959.44 |
| SU5 | 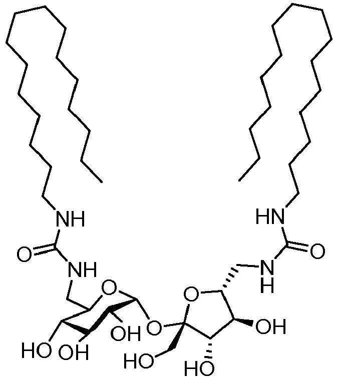 | C₄₆H₉₀N₄O₁₁ 875.24 |
| SU8 | 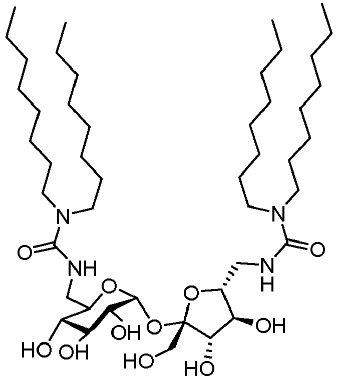 | C₄₆H₉₀N₄O₁₁ 875.24 |
| MT5 | 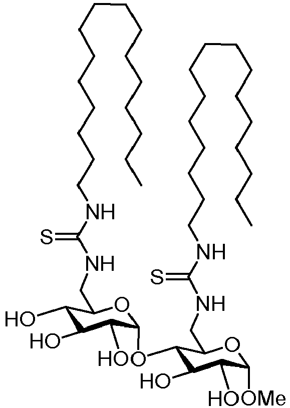 | C₄₆H₉₀N₄O₉S₂ 959.44 |
| MT8 | 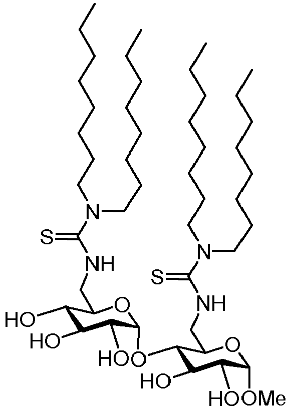 | C₄₆H₉₀N₄O₉S₂ 959.44 |
| MU5 | 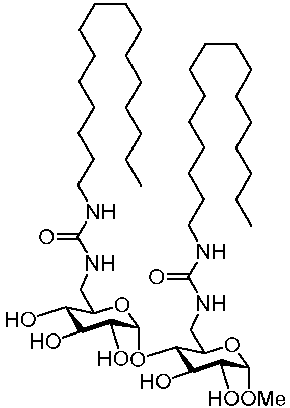 | C₄₆H₉₀N₄O₁₁ 875.24 |
| MU8 | 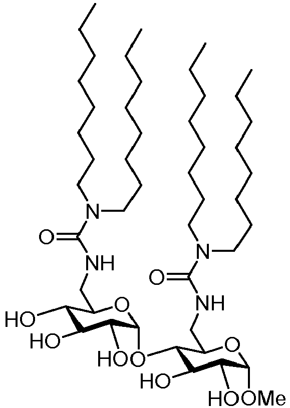 | C₄₆H₉₀N₄O₁₁ 875.24 |

The compounds of formula I of the present invention may contain one or more asymmetric centers and can thus occur as racemates and racemic mixtures, single enantiomers, diastereomeric mixtures and individual diastereomers. Additional asymmetric centers may be present depending upon the nature of the various substituents on the molecule. Each such asymmetric center will independently produce two optical isomers and it is intended that all of the possible optical isomers and diastereomers in mixtures and as pure or partially purified compounds are included within the ambit of this invention. Any formulas, structures or names of compounds described in this specification that do not specify a particular stereochemistry are meant to encompass any and all existing isomers as described above and mixtures thereof in any proportion. When stereochemistry is specified, the invention is meant to encompass that particular isomer in pure form or as part of a mixture with other isomers in any proportion.

Accordingly, the compounds of formula I of the invention may contain chiral centers or double bonds, In the case that contains chiral centers, those may indistinctly have the (R) or (S) configuration. In the case that contains double bonds, those may indistinctly have the (Z) or (E) configuration. The terms "(R)" or "(S)" and "(Z)" or "(E)" are used herein as defined in IUPAC 1974 Recommendations for Section E, Fundamental Stereochemistry, in Pure Appl. Chem., 1976, 45, 13-30. The disclosure contemplates various stereoisomers and mixtures thereof and these are specifically included within the scope of this disclosure. Stereoisomers include enantiomers and diastereomers, and mixtures of enantiomers and diastereomers. Individual stereoisomers of the compounds may be prepared synthetically from commercially available starting materials, which contain asymmetric or chiral centers, or by preparation of racemic mixtures followed by methods of resolution well-known to those of ordinary skill in the art. These methods of resolution are exemplified by (i) attachment of a mixture of enantiomers to a chiral auxiliary, separation of the resulting mixture of diastereomers by recrystallization or chromatography and optional liberation of the optically pure product from the auxiliary as described in Furniss, Hannaford, Smith, and Tatchell, "Vogel's Textbook of Practical Organic Chemistry," 5th edition (1989), Longman Scientific & Technical, Essex CM20 2JE, England (or more recent versions thereof), or (ii) direct separation of the mixture of optical enantiomers on chiral chromatographic columns, or (iii) fractional recrystallization methods.

It should be understood that the compound may possess tautomeric forms, as well as geometric isomer, and that these also constitute embodiments of the disclosure.

The present disclosure also includes an isotopically-labelled compound, which is identical to those recited in formula I, but for the fact that one or more atoms are replaced by an atom having an atomic mass or mass number different from the atomic mass or mass number usually found in nature. Examples of isotopes suitable for inclusion in the compounds of the disclosure are hydrogen, nitrogen, oxygen, sulfur, fluorine and chlorine, such as, but not limited to ²H, ³H, ¹¹C, ¹³C, ¹⁴C, ¹⁵N, ¹⁸O, ¹⁷O, ³⁵S, ¹⁸F, and ³⁶Cl, respectively. Substitution with heavier isotopes such as deuterium, i.e. ²H, can afford certain therapeutic advantages resulting from greater metabolic stability, for example increased in vivo half-life or reduced dosage requirements and, hence, may be preferred in some circumstances. The compound may incorporate positron-emitting isotopes for medical imaging tomography (PET) studies for determining the distribution of receptors. Suitable positron-emitting isotopes that can be incorporated in compounds of formula I are ¹¹C, ¹³C, ¹⁵O, and ¹⁸F. Isotopically-labelled compounds of formula I can be generally prepared by conventional techniques known to those skilled in the art or by processes analogous to those described in the accompanying Examples using appropriate isotopically-labelled reagents in place of non-isotopically-labelled reagent.

The invention also related to compounds of formula I as defined above for the preparation of a vaccine.

Also, the invention refers to a method for treating a subject by administrating a vaccine, said vaccine comprising a compound of formula I as defined above as vaccine adjuvant.

### Compounds

Another aspect of the present invention refers to compounds of formula (I), this formula as described above in the first aspect of the invention (including all the specific embodiment described and the compounds of Table 1): 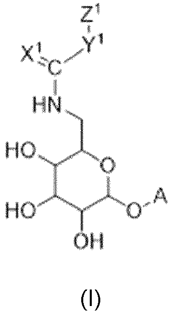 provided that when A is A², each X¹ and X² is O, each Y¹ and Y² is -N(R^{a})-, and each R^{a} is H, then each Z¹ and Z² is not selected from -(CH₂)₇CH₃ and -(CH₂)₈CH₃, and provided that when A is A¹, A¹ is Me, X¹ is S, Y' is -N(R^{a})-, and R^{a} is H, then Z¹ is selected from C₆-C₂₅ aliphatic, C₃-C₂₀ cycloaliphatic, C₅-C₁₄ aryl, 3- to 14-membered heterocyclyl comprising 1 to 3 heteroatoms selected from N, O, and S, wherein Z¹ is optionally substituted with one or more Z³.

Another aspect of the present invention refers to compounds of formula (I), said formula as described in the first aspect of the invention, provided that when A is A², each X¹ and X² is O, each Y¹ and Y² is -N(R^{a})-, and each R^{a} is H, then each Z¹ and Z² is not selected from -(CH₂)₇CH₃ and -(CH₂)₈CH₃, and provided that when A is A¹, A¹ is Me, X¹ is S, Y' is -N(R^{a})-, and R^{a} is H, then Z¹ is selected from C₆-C₂₅ aliphatic, C₃-C₂₀ cycloaliphatic, C₅-C₁₄ aryl, 3- to 14-membered heterocyclyl comprising 1 to 3 heteroatoms selected from N, O, and S, wherein Z¹ is optionally substituted with one or more Z³, or a pharmaceutical composition comprising said compound, for use as a medicament.

Another aspect of the present invention refers to compounds of formula (I) , said formula as described in the first aspect of the invention, provided that when A is A², each X¹ and X² is O, each Y¹ and Y² is -N(R^{a})-, and each R^{a} is H, then each Z¹ and Z² is not selected from -(CH₂)₇CH₃ and -(CH₂)₈CH₃, and provided that when A is A¹, A¹ is Me, X¹ is S, Y' is -N(R^{a})-, and R^{a} is H, then Z¹ is selected from C₆-C₂₅ aliphatic, C₃-C₂₀ cycloaliphatic, C₅-C₁₄ aryl, 3- to 14-membered heterocyclyl comprising 1 to 3 heteroatoms selected from N, O, and S, wherein Z¹ is optionally substituted with one or more Z³, or a pharmaceutical composition comprising said compound, for use in the modulation of immune response in a subject or in the treatment and/or in the prophylaxis of infectious diseases or in the treatment of cancer and also for use as vaccine adjuvants.

### Methods of Preparing Compounds of formula I

Compounds of formula I may be prepared by synthetic processes or by metabolic processes. Preparation of the compounds by metabolic processes includes those occurring in the human or animal body *(in vivo)* or processes occurring *in vitro.*

Another aspect of the present invention refers to a method of preparing a compound of formula I: 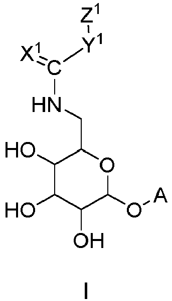 comprising contacting a compound of formula II: 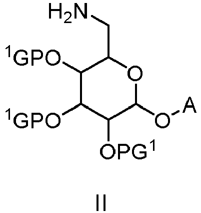 with a compound of formula III

X¹=C=N-Z¹ III

wherein A, X¹, Y¹, and Z¹ are as described previously in the first aspect of the invention, both singly and in combination; and
each PG¹ (PG¹ can be different within the same molecule) is independently selected from H or from a suitable oxygen protecting group that is removed in a next step

As described herein, the present disclosure also provides, among other things, an alternative method of preparing a compound of formula I: 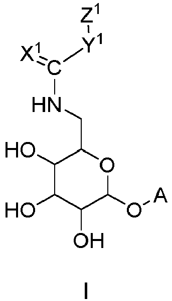 comprising contacting a compound of formula IV: 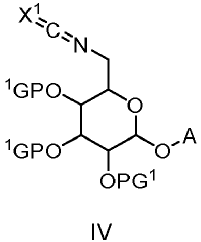 with a compound of formula V

HY¹-Z¹ V

wherein A, X¹, Y¹, and Z¹ are as described as described previously in the first aspect of the invention, both singly and in combination; and
each PG¹ is independently selected from a suitable oxygen protecting group that is removed in a next step.

Suitable oxygen protecting groups and the methods for protecting and deprotecting different substituents using such suitable protecting groups are well known to those skilled in the art; examples of which can be found in PGM Wuts and TW Greene, in Greene's book titled Protective Groups in Organic Synthesis (4th ed.), John Wiley & Sons, NY (2006), which is incorporated herein by reference in its entirety.

In some embodiments, each PG¹ is independently selected from a suitable oxygen protecting group selected from acetyl, benzoyl, benzyl, and trimethylsilyl.

Certain compounds of formula II, III, IV and V may be commercially available. Others may be synthesized according to known methods. The compounds and intermediates may be isolated and purified by methods well-known to those skilled in the art of organic synthesis. Examples of conventional methods for isolating and purifying compounds can include, but are not limited to, chromatography on solid supports such as silica gel, alumina, or silica derivatized with alkylsilane groups, by recrystallization at high or low temperature with an optional pretreatment with activated carbon, thin-layer chromatography, distillation at various pressures, sublimation under vacuum, and trituration, as described for instance in "Vogel's Textbook of Practical Organic Chemistry," 5th edition (1989), by Furniss, Hannaford, Smith, and Tatchell, pub. Longman Scientific & Technical, Essex CM20 2JE, England.

Reaction conditions and reaction times for each individual step can vary depending on the particular reactants employed and substituents present in the reactants used. Specific procedures are provided in the Examples section. Reactions can be worked up in the conventional manner, e.g. by eliminating the solvent from the residue and further purified according to methodologies generally known in the art such as, but not limited to, crystallization, distillation, extraction, trituration and chromatography. Unless otherwise described, the starting materials and reagents are either commercially available or can be prepared by one skilled in the art from commercially available materials using methods described in the chemical literature.

Starting materials, if not commercially available, can be prepared by procedures selected from standard organic chemical techniques, techniques that are analogous to the synthesis of known, structurally similar compounds, or techniques that are analogous to the above described schemes or the procedures described in the synthetic examples section.

Reaction conditions and reaction times for each individual step can vary depending on the particular reactants employed and substituents present in the reactants used. Specific procedures are provided in the Examples section. Reactions can be worked up in the conventional manner, e.g. by eliminating the solvent from the residue and further purified according to methodologies generally known in the art such as, but not limited to, crystallization, distillation, extraction, trituration and chromatography. Unless otherwise described, the starting materials and reagents are either commercially available or can be prepared by one skilled in the art from commercially available materials using methods described in the chemical literature.

It can be appreciated that the synthetic schemes and specific examples as described are illustrative and are not to be read as limiting the scope of the disclosure as it is defined in the appended claims. All alternatives, modifications, and equivalents of the synthetic methods and specific examples are included within the scope of the claims.

### Compositions

The disclosed compounds may be incorporated into pharmaceutical compositions, adjuvant compositions, and vaccine compositions that may be suitable for administration to a subject (such as a patient, which may be a human or non-human).

Then, another aspect of the invention refers to a pharmaceutical composition comprising a therapeutically or prophylactically effective amount of a compound of formula I with the provisos above indicated.

### a. Pharmaceutical Compositions

The disclosed compounds may be incorporated into pharmaceutical compositions. The pharmaceutical compositions may include a "therapeutically effective amount" or a "prophylactically effective amount" of the agent. A "therapeutically effective amount" refers to an amount effective, at dosages and for periods of time necessary, to achieve the desired therapeutic result. A therapeutically effective amount of the composition may be determined by a person skilled in the art and may vary according to factors such as the disease state, age, sex, and weight of the individual, and the ability of the composition to elicit a desired response in the individual. A therapeutically effective amount is also one in which any toxic or detrimental effects of a compound of the invention (e.g., a compound of formula I) are outweighed by the therapeutically beneficial effects. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

For example, a therapeutically effective amount of a compound of formula I, may be about 0.001 mg/kg (Kg refers to Kg of the subject to whom the composition is administered) to about 1000 mg/Kg.

The pharmaceutical compositions and formulations may include pharmaceutically acceptable carriers. The term "pharmaceutically acceptable carrier," as used herein, means a non-toxic, inert solid, semi-solid or liquid filler, diluent, encapsulating material or formulation auxiliary of any type. Some examples of materials which can serve as pharmaceutically acceptable carriers are sugars such as, but not limited to, lactose, glucose and sucrose; starches such as, but not limited to, com starch and potato starch; cellulose and its derivatives such as, but not limited to, sodium carboxymethyl cellulose, ethyl cellulose and cellulose acetate; powdered tragacanth; malt; gelatin; talc; excipients such as, but not limited to, cocoa butter and suppository waxes; oils such as, but not limited to, peanut oil, cottonseed oil, safflower oil, sesame oil, olive oil, com oil and soybean oil; glycols; such as propylene glycol; esters such as, but not limited to, ethyl oleate and ethyl laurate; agar; buffering agents such as, but not limited to, magnesium hydroxide and aluminum hydroxide; alginic acid; pyrogen-free water; isotonic saline; Ringer's solution; ethyl alcohol, and phosphate buffer solutions, as well as other non-toxic compatible lubricants such as, but not limited to, sodium lauryl sulfate and magnesium stearate, as well as coloring agents, releasing agents, coating agents, sweetening, flavoring and perfuming agents, preservatives and antioxidants can also be present in the composition, according to the judgment of the formulator. Preferably, the carriers include at least one of diluents, lubricants, binders, disintegrants, colorants, flavors, sweeteners, antioxidants, preservatives, glidants, solvents, suspending agents, wetting agents, surfactants and combinations thereof.

### b. Adjuvant Compositions and Vaccine Compositions

The compounds may be incorporated into adjuvant compositions and vaccine compositions. The vaccine compositions may further include an antigen. Suitable antigens include microbial pathogens, bacteria, viruses, proteins, glycoproteins lipoproteins, peptides, glycopeptides, lipopeptides, toxoids, carbohydrates, and tumor-specific antigens. Mixtures of two or more antigens may be employed.

The adjuvant and vaccine compositions may include an "effective amount" of the disclosed compound. In the context of an adjuvant or vaccine composition, an "effective amount" refers to an amount effective, at dosages and for periods of time necessary, to achieve the desired result (e.g., to potentiate an immune response to one or more antigens). The immune response can be measured, for example, by measuring antibody titers against an antigen, assessing the ability of a vaccine containing the compound to immunize a host in response to a disease or antigen challenge, etc. For example, administering an "effective amount" of a compound or composition to a subject increases one or more antibody titers by 10% or more over a nonimmune control, by 20% or more over a nonimmune control, by 30% or more over a nonimmune control, by 40% or more over a nonimmune control, by 50% or more over a nonimmune control, by 50% or more over a nonimmune control, by 70% or more over a nonimmune control, by 80% or more over a nonimmune control, by 90% or more over a nonimmune control, or by 100% or more over a nonimmune control (% are wt.%).

Vaccine preparation is a well-developed art and general guidance in the preparation and formulation of vaccines is readily available from any of a variety of sources. One such example is New Trends and Developments in Vaccines, edited by Voller et al., University Park Press, Baltimore, Md, U.S.A. 1978.

The vaccine compositions of the present disclosure may also contain other compounds, which may be biologically active or inactive. For example, one or more immunogenic portions of other tumor antigens may be present, either incorporated into a fusion polypeptide or as a separate compound, within the vaccine composition. Polypeptides may, but need not, be conjugated to other macromolecules as described, for example, within U.S. Pat. Nos. 4,372,945 and 4,474,757. Vaccine compositions may generally be used for prophylactic and therapeutic purposes.

In one embodiment, the antigen in a vaccine composition is a peptide, polypeptide, or immunogenic portion thereof. An "immunogenic portion," as used herein is a portion of a protein that is recognized (i.e., specifically bound) by a B cell and/or T cell surface antigen receptor.

Such immunogenic portions generally comprise at least 5 amino acid residues, more preferably at least 10, and still more preferably at least 20 amino acid residues of an antigenic protein or a variant thereof.

In another embodiment, a compound or adjuvant composition described herein may be used in the preparation of DNA-based vaccine compositions. Illustrative vaccines of this type contain DNA encoding one or more polypeptide antigens, such that the antigen is generated *in situ.* The DNA may be present within any of a variety of delivery systems known to those of ordinary skill in the art, including nucleic acid expression systems, bacteria and viral expression systems. Numerous gene delivery techniques are well known in the art, such as those described by Rolland, Crit. Rev. Therap. Drug Carrier Systems 15:143-198, 1998, and references cited therein.

In another embodiment, a compound or adjuvant composition described herein may be used in the preparation of RNA-based vaccine compositions.

In some embodiments, an RNA amenable to technologies described herein is a single-stranded RNA. In some embodiments, an RNA as disclosed herein is a linear RNA. In some embodiments, a single-stranded RNA is a non-coding RNA in that its nucleotide sequence does not include an open reading frame (or complement thereof). In some embodiments, a single-stranded RNA has a nucleotide sequence that encodes (or is the complement of a sequence that encodes) a polypeptide or a plurality of polypeptides (e.g., epitopes) of the present disclosure.

In some embodiments, an RNA is or comprises an siRNA, an miRNA, or other non-coding RNA.

In some embodiments, a relevant RNA includes at least one open reading frame (ORF) (e.g., is an mRNA); in some embodiments, a relevant RNA includes a single ORF; in some embodiments, a relevant RNA includes more than one ORF.

In some embodiments, an RNA includes unmodified uridine residues; an RNA that includes only unmodified uridine residues may be referred to as a "uRNA". In some embodiments, an RNA includes one or more modified uridine residues; in some embodiments, such an RNA (e.g., an RNA including entirely modified uridine residues) is referred to as a "modRNA". In some embodiments, an RNA may be a self-amplifying RNA (saRNA). In some embodiments, an RNA may be a trans-amplifying RNA (taRNA) (see, for example, WO2017/162461).

In some embodiments, a complex described herein comprises modRNA, saRNA, taRNA, or uRNA. In some embodiments, a complex comprises modRNA. In some embodiments, a complex comprises saRNA. In some embodiments, a complex comprises taRNA. In some embodiments, a complex comprises uRNA.

Moreover, it will be apparent that a vaccine may contain pharmaceutically acceptable salts of the desired polynucleotide, polypeptide and/or carbohydrate antigens. For example, such salts may be prepared from pharmaceutically acceptable non-toxic bases, including organic bases (e.g., salts of primary, secondary and tertiary amines and basic amino acids) and inorganic bases (e.g., sodium, potassium, lithium, ammonium, calcium and magnesium salts).

The adjuvant system may exhibit strong adjuvant effects when administered over a wide range of dosages and a wide range of ratios.

The amount of antigen in each vaccine dose is generally selected as an amount which induces an immunoprotective response without significant adverse side effects in typical vaccines. Such amount will vary depending upon which specific immunogen is employed and how it is presented. Generally, it is expected that each dose will comprise about 1-1000 µg of protein, most typically about 2-100 µg, preferably about 5-50 µg. Of course, the dosage administered may be dependent upon the age, weight, kind of concurrent treatment, if any, and nature of the antigen administered.

The immunogenic activity of a given amount of a vaccine composition can be readily determined, for example by monitoring the increase in titer of antibody against the antigen used in the vaccine composition (Dalsgaard, K. Acta Veterinia Scandinavica 69:1-40 (1978)). Another common method involves injecting CD-I mice intradermally with various amounts of a vaccine composition, later harvesting sera from the mice and testing for anti-immunogen antibody, e.g., by ELISA. These and other similar approaches will be apparent to the skilled artisan.

The antigen can be derived and/or isolated from essentially any desired source depending on the infectious disease, autoimmune disease, condition, cancer, pathogen, or a disease that is to be treated with a given vaccine composition.

In one embodiment, the antigen may be covalently bonded to an adjuvant such as the compound of formula I to produce a discrete molecule which may exhibit an enhanced adjuvanting effect on the antigen, which may be greater than the adjuvanting effect attainable in the absence of such covalent bonding, as in a mixture of components (i.e., the antigen and a compound of formula (I)). The covalent bonding can be achieved by reaction through functional groups; for example, in the case of the compound of formula I through a carboxylic acid group, a hydroxyl group or an aldehyde functionality. A further enhanced adjuvanting effect may be attained for such covalently-bonded antigen by incorporating a mineral salt adjuvant with such compounds. The mineral salt adjuvant preferably comprises aluminum hydroxide or aluminum phosphate, although other known mineral salt adjuvants, such as calcium phosphate, zinc hydroxide or calcium hydroxide, may be used.

The adjuvant may include other polynucleotides and/or polypeptides. It will be apparent that a vaccine may contain pharmaceutically acceptable salts of the polynucleotides and polypeptides provided herein. Such salts may be prepared from pharmaceutically acceptable non-toxic bases, including organic bases (e.g., salts of primary, secondary and tertiary amines and basic amino acids) and inorganic bases (e.g., sodium, potassium, lithium, ammonium, calcium and magnesium salts).

The vaccine compositions may be formulated for any appropriate manner of administration, and thus administered, including for example, topical, oral, nasal, intravenous, intravaginal, epicutaneous, sublingual, intracranial, intradermal, intraperitoneal, subcutaneous, intramuscular administration, or via inhalation. For parenteral administration, such as subcutaneous injection, the carrier preferably comprises water, saline, alcohol, a fat, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed.

The vaccine compositions may also comprise buffers (e.g., neutral buffered saline or phosphate buffered saline), carbohydrates (e.g., glucose, mannose, sucrose or dextrans), mannitol, proteins, polypeptides or amino acids such as glycine, antioxidants, bacteriostats, chelating agents such as EDTA or glutathione, adjuvants (e.g., aluminum hydroxide), solutes that render the formulation isotonic, hypotonic or weakly hypertonic with the blood of a recipient, suspending agents, thickening agents and/or preservatives. Alternatively, vaccine compositions may be formulated as a lyophilisate. Compounds may also be encapsulated within liposomes using well known technology.

The vaccine compositions may also comprise other adjuvants or immunoeffectors. Suitable adjuvants are commercially available as, for example, Freund's Incomplete Adjuvant and Complete Adjuvant (Difeo Laboratories, Detroit, Mich.); Merck Adjuvant 65 (Merck and Company, Inc., Rahway, NJ.); AS-2 (SmithKline Beecham); mineral salts (for example, aluminum, silica, kaolin, and carbon); aluminum salts such as aluminum hydroxide gel (alum), AlK(SO₄)₂, AlNa(SO₄)₂, AlNH₄(SO₄)₂, and Al(OH)₃; salts of calcium (e.g., Ca₃(PO₄)₂) , iron or zinc; an insoluble suspension of acylated tyrosine; acylated sugars; cationically or anionically derivatized polysaccharides; polynucleotides (for example, poly IC and poly AU acids); polyphosphazenes; cyanoacrylates; polymerase-(DL-lactide-co-glycoside); biodegradable microspheres; liposomes; lipid A and its derivatives; monophosphoryl lipid A; wax D from Mycobacterium tuberculosis, as well as substances found in Corynebacterium parvum, Bordetella pertussis, and members of the genus Brucella); bovine serum albumin; diphtheria toxoid; tetanus toxoid; edestin; keyhole-limpet hemocyanin; Pseudomonal Toxin A; choleragenoid; cholera toxin; pertussis toxin; viral proteins; and Quil A Aminoalkyl glucosamine phosphate compounds can also be used (see, e.g., WO 98/50399, U.S. Pat. No. 6,113,918 (which issued from U.S. Ser. No. 08/853,826), and U.S. Ser. No. 09/074,720). In addition, adjuvants such as cytokines (e.g., GM-CSF or interleukin-2, -7, or -12), interferons, or tumor necrosis factor, may also be used as adjuvants. Protein and polypeptide adjuvants may be obtained from natural or recombinant sources according to methods well known to those skilled in the art. When obtained from recombinant sources, the adjuvant may comprise a protein fragment comprising at least the immunostimulatory portion of the molecule. Other known immunostimulatory macromolecules which can be used include are, but not limited to, polysaccharides, tRNA, non-metabolizable synthetic polymers such as polyvinylamine, polymethacrylic acid, polyvinylpyrrolidone, mixed polycondensates (with relatively high molecular weight) of 4',4-diaminodiphenylmethane-3,3'-dicarboxylic acid and 4-nitro-2- aminobenzoic acid (See, Sela, M., Science 166: 1365-1374 (1969)) or glycolipids, lipids or carbohydrates.

Within the vaccine compositions provided herein, the adjuvant composition is preferably designed to induce an immune response predominantly of the Th1 and/or Th17-type. High levels of Th1 and/or Th17-type cytokines (e.g., IL-6, IL-1 , IL-23, and TNFα) may favor the induction of cell mediated immune responses to an administered antigen. Following administration of a vaccine as provided herein, a patient may support an immune response that includes Th1- and/or Th17-type responses.

In some embodiments, in which a response is predominantly Th1 and/or Th17-type, the level of Th1 and/or Th17-type cytokines will increase to a greater extent than the level of other cytokines. The levels of these cytokines may be readily assessed using standard assays. For a review of the families of cytokines, see, Mosmann and Coffman, Ann. Rev. Immunol. 1989, 7: 145-173.

The compositions described herein may be administered as part of a sustained release formulation (i.e., a formulation such as a capsule, sponge or gel (composed of polysaccharides, for example) that effects a slow release of compound following administration). Such formulations may generally be prepared using well known technology (see, e.g., Coombes et al., Vaccine 14:1429-1438, 1996) and administered by, for example, oral, rectal or subcutaneous implantation, or by implantation at the desired target site. Sustained-release formulations may contain a polypeptide, polynucleotide or antibody dispersed in a carrier matrix and/or contained within a reservoir surrounded by a rate controlling membrane. Carriers for use within such formulations are biocompatible, and may also be biodegradable; preferably the formulation provides a relatively constant level of active component release. Such carriers include microparticles of poly(lactide-co-glycolide), polyacrylate, latex, starch, cellulose, dextran and the like. Other delayed-release carriers include supramolecular biovectors, which comprise a non-liquid hydrophilic core (e.g., a cross-linked polysaccharide or oligosaccharide) and, optionally, an external layer comprising an amphiphilic compound, such as a phospholipid (see, e.g., U.S. Pat. No. 5,151,254 and PCT applications WO 94/20078, WO/94/23701 and WO 96/06638). The amount of active compound contained within a sustained release formulation will vary depending upon the site of implantation, the rate and expected duration of release and the nature of the condition to be treated or prevented.

Any of a variety of known delivery vehicles may be employed within pharmaceutical compositions and vaccines to facilitate production of an antigen-specific immune response that targets cells. Delivery vehicles include antigen presenting cells (APCs), such as dendritic cells, macrophages, B cells, monocytes and other cells that may be engineered to be efficient APCs. Such cells may, but need not, be genetically modified to increase the capacity for presenting the antigen, to improve activation and/or maintenance of the T cell response, to have anti-target effects per se and/or to be immunologically compatible with the receiver (i.e., matched HLA haplotype). APCs may generally be isolated from any of a variety of biological fluids and organs, including tumor and peritumoral tissues, and may be autologous, allogeneic, syngeneic or xenogeneic cells.

In one embodiment, the vaccine composition may comprise a liposome vesicle comprising the compound of formula I. Liposomes are generally produced from phospholipids or other lipid substances. Procedures for the preparation of liposomes are well known to those of skill in the art. Any lipid capable of forming vesicles that comprises the compound of formula I can be employed. For clinical application, it is desirable that the lipid be non-toxic, physiologically acceptable, and metabolizable. Common bilayer forming lipids having clinical potential are phospholipids, fatty acids, sphingolipids, glycosphingolipids, and steroids. Glycerol containing phospholipids are the most commonly used component of liposome formulations having clinical utility. One commonly used example is phosphatidylcholine or lecithin. The steroid cholesterol and its derivatives are often included as components of liposomal membranes. The tendency of liposomes to aggregate and fuse can be controlled by the inclusion of small amounts of acidic or basic lipids in the formulation. The properties of liposomes containing phospholipids are determined by the chemistry of the phospholipid. Important considerations are the hydrocarbon chain length, degree of unsaturation of the hydrocarbon chain, degree of branching of the hydrocarbon chain, and temperature of the system.

Multilamellar liposomes can be created by depositing a mixture of lipids as a thin film by evaporation under reduced pressure followed by dispersion with an excess volume of aqueous buffer containing the antigen with or without organic solvents. Another method is to mix the aqueous phase containing the antigen with small unilamellar liposomes followed by lyophilization. The multilamellar liposomes are formed when the lyophilized product is rehydrated, usually with a small amount of distilled water. The small unilamellar liposomes to be used in this process are produced by dispersing the lipids in an aqueous medium followed by a mechanical means of dispersion such as sonication, use of a high pressure device, or a solvent injection method. Large and intermediate sized unilamellar liposomes can also be produced by conventional techniques including detergent dialysis, extrusion through small pore size membranes under high pressure, freeze thawing followed by slow swelling, dehydration followed by rehydration and dilution, or dialysis of lipids in the presence of chaotropic ions. The size of the liposomes can be made more uniform by fractionation procedures such as centrifugation or size exclusion chromatography, homogenization, or capillary pore membrane extrusion.

### Compounds/compositions for use

The disclosed compounds with the provisos above indicated and compositions may be used in various methods, including methods for modulating an immune response in a subject, methods of inducing or enhancing immunogenicity of an antigen in a subject, and related methods. The disclosed compounds and compositions may also be used in methods of treating cancer and related methods. The disclosed compounds and compositions may also be used in methods of treatment and prevention of an autoimmune disorder or an infectious disease.

The compounds and compositions of the invention may be used in methods of treating an immune disease. The term "immune disease", as used herein, refers to any disease in which the immune system is compromised, altered or subverted, and that can therefore be treated with immunotherapy approaches, i.e., therapeutic strategies targeting innate immune sensors. In the case of the compounds and compositions of the invention, the targeted innate immune sensor is Mincle, as supported by the exemplary embodiments hereinafter. By targeting Mincle, the compounds and compositions of the invention behave as immune modulators as defined in the review article by E. C. Patin, S. J. Orr and U. E. Schaible (2017) Macrophage Inducible C-Type Lectin As a Multifunctional Player in Immunity, Front. Immunol. 8:861. Accordingly, they may be used as adjuvants in various methods for vaccine formulation and may also counter regulate pro-inflammatory signalling pathways. Examples of immune diseases that can be treated with immunotherapy approaches that target Mincle, according to the invention, are infections, cancers, and autoimmune diseases.

The present invention also relates to the use of compounds of formula I with the provisos above indicated for the preparation of a medicament. More preferably a medicament for the treatment and/or prevention of cancer, an autoimmune disorder or an infectious disease.

The present invention also relates to methods for the treatment and/or prevention of cancer, an autoimmune disorder or an infectious disease comprising administering a compound of formula I with the provisos above indicated to a subject.

### a. Modulating Immune Response

The disclosed compounds and compositions may be used in methods of modulating the immune response in a subject, comprising administering to the subject an effective amount of a compound described herein, an adjuvant composition described herein, or an immunomodulatory composition described herein.

In some embodiments, disclosed compounds and compositions may be used in a method of inducing an enhanced immune response in a subject. In some embodiments, the enhanced immune response is an immune response of a Th1 and/or Th17-type. In some embodiments, administration of the compound or composition may induce a Th1 and/or Th17-type immune response and not a Th2-type immune response.

The enhanced immune response may be induced by co-administering the compound or composition with an antigen. Suitable antigens include microbial pathogens, bacteria, viruses, proteins, glycoproteins lipoproteins, peptides, glycopeptides, lipopeptides, toxoids, carbohydrates, and tumor-specific antigens. Protein antigens may be codified by DNA or RNA. Mixtures of two or more antigens may be employed.

In some embodiments, the disclosed compounds and compositions may be administered as a monotherapy.

Compounds and compositions described herein are useful in the treatment and prophylaxis in a subject of diseases, disorders, and conditions described herein. In some embodiments, a disease, disorder or condition is an infectious disease, cancer or an autoimmune disease.

### b. Inducing or Enhancing Immunogenicity of an Antigen for the Treatment and Profilaxis of Infectious Diseases

The disclosed compounds and compositions may be used in methods of inducing or enhancing immunogenicity of an antigen in a subject for the treatment and profilaxis of an infectious disease, comprising administering to the subject a vaccine composition comprising the antigen and an adjuvant composition comprising an effective amount of a compound or composition described herein.

Suitable antigens include microbial pathogens, bacteria, viruses, proteins, glycoproteins lipoproteins, peptides, glycopeplides, lipopeptides, toxoids, carbohydrates, and tumor-specific antigens. Protein antigens may be codified by DNA or RNA. Mixtures of two or more antigens may be employed.

In some embodiments, an infectious disease is caused by or associated with a viral pathogen. In some embodiments, a viral pathogen is of a family selected from poxviridae, rhabdoviridae, filoviridae, paramyxoviridae, hepadnaviridae, coronaviridae, caliciviridae, picornaviridae, reoviridae, retroviridae, and orthomyxoviridae. In some embodiments, an infectious disease is caused by or associated with a virus selected from SARS-CoV-2, influenza, Crimean-Congo Hemorhhagic Fever (CCHF), Ebola virus, Lassa virus, Marburg virus, HIV, Nipah virus, and MERS-CoV.

In some embodiments, an infectious disease is caused by or associated with a bacterial pathogen. In some embodiments, a bacterial pathogen is of a species selected from Actinomyces israelii, bacillus antracis, Bacteroides fragilis, Bordetella pertussis, Borrelia burgdorferi, Borrelia garinii, Borrelia afzelii, Borrelia recurrentis, Brucella abortus, Brucella canis, Brucella melitensis, Brucella suis, Campolobacter jejuni, Chlamydia pneumoniae, Chlamydia trachomatis, Chlamydophila psittaci, Clostridium botulinum, Clostridium difficile, Clostridium perfringens, Clostridium tetani, Corynebacterium idphteriae, Ehrlichia canis, Ehrlichia chaffeensis, Enterococcus faecalis, Enterococcus faecium, Escherichia coli, Francisella tularensis, Haemophilus influenzae, Helicobacter pylori, Klebsiella pneumoniae, Legionella pneumophila, Leptospira, Listeria monocytogenes, Mycobacterium leprae, Mycobacterium tuberculosis, Mycoplasma pneumoniae, Neisseria gonorrhoeae, Neisseria meningitidis, Pseudomonas aeruginosa, Nocardia asteroids, Rickettsia ricektssii, Salmonella typhi, Salmonella typhimurium, Shigella sonnei, Shigella dysenteriae, Staphylococcus aureus, Staphylococcus epidermidis, Staphylococcus saprophyticus, Streptococcus agalactiae, Streptococcus pneumoniae, Streptococcus pyogenes, Streptococcus viridans, Treponema pallidum, Vibrio cholerae, and Yersinia pestis.

In some embodiments, an infectious disease is caused by or associated with a parasite. In some embodiments, a parasite is of a family selected from Plasmodium, Leishmania, Cryptosporidium, Entamoeba, Trypanosomas, Schistosomes, Ascaris, Echinococcus and Taeniidae.

### c. Treatment of Cancer and Related Methods

The disclosed compounds and compositions may be used in methods of treating cancer, or in methods of reducing or inhibiting the proliferation of cancer cells, the methods comprising administering to a subject in need thereof a therapeutically effective amount of a compound or a composition described herein.

The methods can be used with any cancer cell or in a subject having any type of cancer, for example those described by the National Cancer Institute. In some embodiments, a cancer is selected from-the groups of digestive/gastrointestinal cancers, eye cancers, musculoskeletal cancers, neurologic cancers, genitourinary cancers, head and neck cancers, hematologic/blood cell cancer, lung cancers, respiratory cancers, skin cancers, AIDS-related malignancies, unusual cancers of childhood, cancers of unknown primary site, and metastases of the aforementioned cancers.

### d. Treatment of Autoimmune Diseases

The disclosed compounds and compositions may be used in methods of treating an autoimmune disease, the methods comprising administering to a subject in need thereof a therapeutically effective amount of a compound or a composition described herein.

In some embodiments, an autoimmune disease is selected from addison disease, celiac disease, rheumatoid arthritis, lupus, inflammatory bowel disease, dermatomyositis, multiple sclerosis, diabetes, guillain-barre syndrome, chronic inflammatory demyelinating polyneuropathy, psoriasis, pernicious anemia, graves' disease, hashimoto's thyroiditis, myasthenia gravis, and vasculitis sjörgen syndrome.

### Kits

In another aspect, the disclosure provides kits comprising at least one disclosed compound of formula I or a pharmaceutically acceptable salt thereof, or a composition comprising said compound or a pharmaceutically acceptable salt thereof, and one or more of:
(a) at least one antigen; and
(b) instructions for administering the compound or composition.

In some embodiments, the at least one disclosed compound and the at least one antigen are co-formulated. In some embodiments, the at least one disclosed compound and the at least one antigen are co-packaged. The kits can also comprise compounds and/or products co- packaged, co-formulated, and/or co-delivered with other components. For example, a drug manufacturer, a drug reseller, a physician, a compounding shop, or a pharmacist can provide a kit comprising a disclosed compound and/or product and another component for delivery to a patient.

The disclosed kits can be employed in connection with disclosed methods of use.

The kits may further include information, instructions, or both that use of the kit will provide increased immunity against certain pathogens in mammals (particularly humans). The information and instructions may be in the form of words, pictures, or both, and the like. In addition, or in the alternative, the kit may include the compound, a composition, or both; and information, instructions, or both, regarding methods of administration of compound, or of the composition, preferably with the benefit of treating or preventing medical conditions in mammals (e.g., humans).

The compounds and processes of the disclosure may be better understood by reference to the examples of the invention, which are intended as an illustration of and not a limitation upon the scope of the disclosure.

### Definitions

Unless otherwise defined, all technical and scientific terms used herein have the same meaning as commonly understood by one of ordinary skill in the art. In case of conflict, the present document, including definitions, will control. Preferred methods and materials are described hereinafter, although methods and materials similar or equivalent to those described herein can be used in practice or testing. All publications, patents and other references are incorporated by reference in their entirety. The materials, methods and examples disclosed herein are illustrative and not intended to be limiting.

The terms "comprise(s)", "include/s)", having", "has", "can", "contain(s)" and variants thereof, as used herein, are intended to be open-ended transitional phrases, terms or words that do not preclude the possibility of additional acts or structures. The present disclosure also contemplates other embodiments "comprising", "consisting of" and "consisting essentially of" the embodiments or elements presented herein, whether explicitly set forth or not.

As used herein the singular forms "a", "and", and "the" include plural referents unless the context clearly dictates otherwise. For example, "a compound" refers to one or more of such compounds as known to those skilled in the art.

Definitions of specific functional groups and chemical terms are described in more detail below. For purposes of this disclosure, the chemical elements are identified in accordance with the Periodic Table of Elements, CAS version, in Handbook of Chemistry and Physics, 75^{th} Ed., inside cover, and specific functional groups are generally defined as described therein. Additionally, general principles of organic chemistry, as well as specific functional moieties and reactivity , are described in Organic Chemistry, Thomas Sorrell, University Science Books, Sausalito, 1999; Smith and March March's Advanced Organic Chemistry, 5th Edition, John Wiely & Sons, Inc., New York, 2002; Larock, Comprehensive Organic Transformations, VCH Publishers, Inc., New York, 1989; Carruthers, Some Modern Methods of Organic Synthesis, 3rd Edition, Cambridge University Press, Cambridge, 1987; the entire contents of which are incorporated herein by reference.

Unless otherwise stated, structures depicted herein are meant to include all stereoisomeric (e.g., enantiomeric or diastereomeric) forms of the structure, as well as all geometric or conformational isomeric forms of the structure. For example, the R and S configurations of each stereocenter are contemplated as part of the disclosure. Therefore, single stereochemical isomers, as well as enantiomeric, diastereomeric, and geometric (or conformational) mixtures of provided compounds are within the scope of the disclosure. For example, in some cases, Table 1 show one or more stereoisomers of a compound, and unless otherwise indicated, represents each stereoisomer alone and/or as a mixture. Unless otherwise stated, all tautomeric forms of provided compounds are within the scope of the disclosure.

Unless otherwise indicated, structures depicted herein are meant to include compounds that differ only in the presence of one or more isotopically enriched atoms. For example, compounds having the present structures including replacement of hydrogen by deuterium or tritium, or replacement of a carbon by ¹³C- or ¹⁴C-enriched carbon are within the scope of this disclosure.

***Compound:*** Throughout this application, it is contemplated that the term "compound" or "compounds" refers to the compounds discussed herein and includes precursors and derivatives of the compounds and pharmaceutically acceptable salts of the compounds, precursors, and derivatives.

***About** or **approximately:*** As used herein, the term "approximately" or "about," as applied to one or more values of interest, refers to a value that is similar to a stated reference value. In general, those skilled in the art, familiar within the context, will appreciate the relevant degree of variance encompassed by "about" or "approximately" in that context. For example, in some embodiments, the term "approximately" or "about" may encompass a range of values that are within (i.e., ±) 25%, 20%, 19%, 18%, 17%, 16%, 15%, 14%, 13%, 12%, 11%, 10%, 9%, 8%, 7%, 6%, 5%, 4%, 3%, 2%, 1%, or less of the referred value.

***Administering:*** As used herein, the term "administering" or "administration" typically refers to the administration of a composition to a subject to achieve delivery of an agent that is, or is included in, a composition to a target site or a site to be treated. Those of ordinary skill in the art will be aware of a variety of routes that may, in appropriate circumstances, be utilized for administration to a subject, for example a human. For example, in some embodiments, administration may be ocular, oral, parenteral, topical, *etc.* In some particular embodiments, administration may be bronchial (*e.g*., by bronchial instillation), buccal, dermal (which may be or comprise, for example, one or more of topical to the dermis, intradermal, interdermal, transdermal, *etc*.), enteral, intra-arterial, intradermal, intragastric, intramedullary, intramuscular, intranasal, intraperitoneal, intrathecal, intravenous, intraventricular, within a specific organ (*e.g*., intrahepatic), mucosal, nasal, oral, rectal, subcutaneous, sublingual, topical, tracheal (*e.g.*, by intratracheal instillation), vaginal, vitreal, *etc.* In some embodiments, administration may be parenteral. In some embodiments, administration may be oral. In some particular embodiments, administration may be intravenous. In some particular embodiments, administration may be subcutaneous. In some embodiments, administration may involve only a single dose. In some embodiments, administration may involve application of a fixed number of doses. In some embodiments, administration may involve dosing that is intermittent (*e.g*., a plurality of doses separated in time) and/or periodic (*e.g*., individual doses separated by a common period of time) dosing. In some embodiments, administration may involve continuous dosing (*e.g*., perfusion) for at least a selected period of time. In some embodiments, administration may comprise a prime-and-boost protocol. A prime-and-boost protocol can include administration of a first dose of a pharmaceutical composition (e.g., an immunogenic composition, e.g., a vaccine) followed by, after an interval of time, administration of a second or subsequent dose of a pharmaceutical composition (e.g., an immunogenic composition, e.g., a vaccine). In the case of an immunogenic composition, a prime-and-boost protocol can result in an increased immune response in a patient.

***Aliphatic:*** The term "aliphatic" refers to a straight-chain (i.e., unbranched) or branched, substituted or unsubstituted hydrocarbon chain that is completely saturated or that contains one or more units of unsaturation, or a monocyclic hydrocarbon or bicyclic hydrocarbon that is completely saturated or that contains one or more units of unsaturation, but which is not aromatic (also referred to herein as "cycloaliphatic"), that has a single point or more than one points of attachment to the rest of the molecule. As used herein, it is understood that an aliphatic group can also be bivalent (e.g., encompass a bivalent hydrocarbon chain that is saturated or contains one or more units of unsaturation, such as, for example, -CH₂-, -CH₂-CH₂-, -CH₂-CH₂-CH₂-, and so on). In some embodiments, 1-20 aliphatic carbon atoms (e.g., C₁₋₂₀). In some embodiments, aliphatic groups contain 2-20 aliphatic carbon atoms (e.g., C₂₋₂₀). In other embodiments, aliphatic groups contain 4-20 aliphatic carbon atoms (e.g., C₄₋₂₀). In still other embodiments, aliphatic groups contain 1-25 aliphatic carbon atoms (e.g., C₁₋₂₅). In some embodiments, "cycloaliphatic" refers to a monocyclic or bicyclic C₃₋₂₀ hydrocarbon that is completely saturated or that contains one or more units of unsaturation, but which is not aromatic, that has a single point or more than one points of attachment to the rest of the molecule. Suitable aliphatic groups include, but are not limited to, linear or branched, substituted or unsubstituted alkyl, alkenyl, or alkynyl groups and hybrids thereof.

***Alkyl:*** The term "alkyl", used alone or as part of a larger moiety, refers to a saturated, optionally substituted straight or branched chain hydrocarbon group having (unless otherwise specified) 1-20, 2-20, 4-20, 1-25 carbon atoms (e.g., C₁₋₂₀, C₂₋₂₀, C₄₋₂₀, C₁₋₂₅). Exemplary alkyl groups include methyl, ethyl, propyl, butyl, pentyl, hexyl, heptyl octyl, decyl, dodecyl, tetradecyl, hexadecyl and octadecyl.

***Alkenyl:*** The term "alkenyl", used alone or as part of a larger moiety, refers to an optionally substituted straight or branched chain or cyclic hydrocarbon group having at least one double bond and having (unless otherwise specified) 2-20, 2-20, 4-20, 2-25 carbon atoms (e.g., C₂₋₂₀, C₂₋₂₀, C₄₋₂₀, C₂₋₂₅). The term "cycloalkenyl" refers to an optionally substituted non-aromatic monocyclic or multicyclic ring system containing at least one carbon-carbon double bond and having about 3 to about 20 carbon atoms. Exemplary monocyclic cycloalkenyl rings include cyclopentenyl, cyclohexenyl, and cycloheptenyl.

***Alkynyl:*** The term "alkynyl", used alone or as part of a larger moiety, refers to an optionally substituted straight or branched chain hydrocarbon group having at least one triple bond and having (unless otherwise specified) 2-20, 2-20, 4-20, 2-25 carbon atoms (e.g., C₂₋₂₀, C₂₋₂₀, C₄₋₂₀, C₂₋₂₅). Exemplary alkynyl groups include ethynyl, propynyl, butynyl, pentynyl, hexynyl, and heptynyl.

***Analog:*** As used herein, the term "analog" refers to a substance that shares one or more particular structural features, elements, components, or moieties with a reference substance. Typically, an "analog" shows significant structural similarity with the reference substance, for example sharing a core or consensus structure, but also differs in certain discrete ways. In some embodiments, an analog is a substance that can be generated from the reference substance, e.g., by chemical manipulation of the reference substance. In some embodiments, an analog is a substance that can be generated through performance of a synthetic process substantially similar to (e.g., sharing a plurality of steps with) one that generates the reference substance. In some embodiments, an analog is or can be generated through performance of a synthetic process different from that used to generate the reference substance.

***Aryl:*** The term "aryl" refers to monocyclic and bicyclic ring systems having a total of five to fourteen ring members (e.g., C₅-C₁₄), wherein at least one ring in the system is aromatic and wherein each ring in the system contains three to seven ring members. In some embodiments, an "aryl" group contains between six and twelve total ring members (e.g., C₆-C₁₂). The term "aryl" may be used interchangeably with the term "aryl ring". In certain embodiments of the present invention, "aryl" refers to an aromatic ring system which includes, but not limited to, phenyl, biphenyl, naphthyl, anthracyl and the like, which may bear one or more substituents. Unless otherwise specified, "aryl" groups are hydrocarbons. In some embodiments, an "aryl" ring system is an aromatic ring (e.g., phenyl) that is fused to a non-aromatic ring (e.g., cycloalkyl). Examples of aryl rings include that are fused include 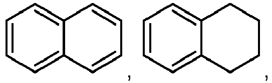 and

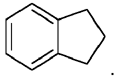

***Associated:*** Two events or entities are "associated" with one another, as that term is used herein, if the presence, level and/or form of one is correlated with that of the other. For example, a particular entity (e.g., polypeptide, genetic signature, metabolite, microbe, etc) is considered to be associated with a particular disease, disorder, or condition, if its presence, level and/or form correlates with incidence of and/or susceptibility to the disease, disorder, or condition (e.g., across a relevant population). In some embodiments, two or more entities are physically "associated" with one another if they interact, directly or indirectly, so that they are and/or remain in physical proximity with one another. In some embodiments, two or more entities that are physically associated with one another are covalently linked to one another; in some embodiments, two or more entities that are physically associated with one another are not covalently linked to one another but are non-covalently associated, for example by means of hydrogen bonds, van der Waals interaction, hydrophobic interactions, magnetism, and combinations thereof.

***Biological sample:*** As used herein, the term "biological sample" typically refers to a sample obtained or derived from a biological source (e.g., a tissue or organism or cell culture) of interest, as described herein. In some embodiments, a source of interest comprises an organism, such as an animal or human. In some embodiments, a biological sample is or comprises biological tissue or fluid. In some embodiments, a biological sample may be or comprise bone marrow; blood; blood cells; ascites; tissue or fine needle biopsy samples; cell-containing body fluids; free floating nucleic acids; sputum; saliva; urine; cerebrospinal fluid, peritoneal fluid; pleural fluid; feces; lymph; gynecological fluids; skin swabs; vaginal swabs; oral swabs; nasal swabs; washings or lavages such as a ductal lavages or broncheoalveolar lavages; aspirates; scrapings; bone marrow specimens; tissue biopsy specimens; surgical specimens; feces, other body fluids (e.g., sperm, sweat, tears), secretions, and/or excretions; and/or cells therefrom, etc. In some embodiments, a biological sample is or comprises cells obtained from an individual. In some embodiments, obtained cells are or include cells from an individual from whom the sample is obtained. In some embodiments, a sample is a "primary sample" obtained directly from a source of interest by any appropriate means. For example, in some embodiments, a primary biological sample is obtained by methods selected from the group consisting of biopsy (e.g., fine needle aspiration or tissue biopsy), surgery, collection of body fluid (e.g., blood, lymph, feces etc.), etc. In some embodiments, as will be clear from context, the term "sample" refers to a preparation that is obtained by processing (e.g., by removing one or more components of and/or by adding one or more agents to) a primary sample. For example, filtering using a semi-permeable membrane. Such a "processed sample" may comprise, for example, nucleic acids or proteins extracted from a sample or obtained by subjecting a primary sample to techniques such as amplification or reverse transcription of mRNA, isolation and/or purification of certain components, etc.

*Carrier:* As used herein, the term "carrier" refers to a diluent, adjuvant, excipient, or vehicle with which a composition is administered. In some exemplary embodiments, carriers can include sterile liquids, such as, for example, water and oils, including oils of petroleum, animal, vegetable or synthetic origin, such as, for example, peanut oil, soybean oil, mineral oil, sesame oil and the like. In some embodiments, carriers are or include one or more solid components.

***Comparable:*** As used herein, the term "comparable" refers to two or more agents, entities, situations, sets of conditions, etc., that may not be identical to one another but that are sufficiently similar to permit comparison therebetween so that one skilled in the art will appreciate that conclusions may reasonably be drawn based on differences or similarities observed. In some embodiments, comparable sets of conditions, circumstances, individuals, or populations are characterized by a plurality of substantially identical features and one or a small number of varied features. Those of ordinary skill in the art will understand, in context, what degree of identity is required in any given circumstance for two or more such agents, entities, situations, sets of conditions, etc. to be considered comparable. For example, those of ordinary skill in the art will appreciate that sets of circumstances, individuals, or populations are comparable to one another when characterized by a sufficient number and type of substantially identical features to warrant a reasonable conclusion that differences in results obtained or phenomena observed under or with different sets of circumstances, individuals, or populations are caused by or indicative of the variation in those features that are varied.

***Composition:*** Those skilled in the art will appreciate that the term "composition" may be used to refer to a discrete physical entity that comprises one or more specified components. In general, unless otherwise specified, a composition may be of any form - e.g., gas, gel, liquid, solid, etc.

***Cycloaliphatic:*** As used herein, the term "cycloaliphatic" refers to a monocyclic C₃₋₈ hydrocarbon or a bicyclic C₇₋₁₀ hydrocarbon that is completely saturated or that contains one or more units of unsaturation, but which is not aromatic, that has a single point or more than one points of attachment to the rest of the molecule.

***Cycloalkyl:*** As used herein, the term "cycloalkyl" refers to an optionally substituted saturated ring monocyclic or polycyclic system of about 3 to about 10 ring carbon atoms. Exemplary monocyclic cycloalkyl rings include cyclopropyl, cyclobutyl, cyclopentyl, cyclohexyl, and cycloheptyl.

***Excipient:*** As used herein, the term "excipient" refers to a non-therapeutic agent that may be included in a pharmaceutical composition, for example, to provide or contribute to a desired consistency or stabilizing effect. Suitable pharmaceutical excipients include, for example, starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like.

***Halogen:*** The term "halogen" or "halo", as used herein, means Cl, Br, I or F.

***Heteroaliphatic:*** The term "heteroaliphatic" or "heteroaliphatic group", as used herein, denotes an optionally substituted hydrocarbon moiety having, in addition to carbon atoms, from one to five heteroatoms, that may be straight-chain (*i.e.,* unbranched), branched, or cyclic ("heterocyclic") and may be completely saturated or may contain one or more units of unsaturation, but which is not aromatic. The term "heteroatom" refers to nitrogen, oxygen, or sulfur, and includes any oxidized form of nitrogen or sulfur, and any quaternized form of a basic nitrogen. The term "nitrogen" also includes a substituted nitrogen. Unless otherwise specified, heteroaliphatic groups contain 1-10 carbon atoms wherein 1-3 carbon atoms are optionally and independently replaced with heteroatoms selected from oxygen, nitrogen, and sulfur. In some embodiments, heteroaliphatic groups contain 1-4 carbon atoms, wherein 1-2 carbon atoms are optionally and independently replaced with heteroatoms selected from oxygen, nitrogen, and sulfur. In yet other embodiments, heteroaliphatic groups contain 1-3 carbon atoms, wherein 1 carbon atom is optionally and independently replaced with a heteroatom selected from oxygen, nitrogen, and sulfur. Suitable heteroaliphatic groups include, but are not limited to, linear or branched, heteroalkyl, heteroalkenyl, and heteroalkynyl groups. For example, a 1 to 10 atom heteroaliphatic group includes the following exemplary groups: -O-CH₃, -CH₂-O-CH₃, -O-CH₂-CH₂-O-CH₂-CH₂-O-CH₃, and the like.

***Heteroaryl:*** The terms "heteroaryl" and "heteroar-", used alone or as part of a larger moiety, e.g., "heteroaralkyl", or "heteroaralkoxy", refer to monocyclic or bicyclic ring groups having 5 to 12 ring atoms (e.g., 5- to 6- membered monocyclic heteroaryl or 9- to 12-membered bicyclic heteroaryl); having 6, 10, or 14 π-electrons shared in a cyclic array; and having, in addition to carbon atoms, from one to five heteroatoms. The term "heteroatom" refers to nitrogen, oxygen, or sulfur, and includes any oxidized form of nitrogen or sulfur, and any quaternized form of a basic nitrogen. Heteroaryl groups include, without limitation, thienyl, furanyl, pyrrolyl, imidazolyl, pyrazolyl, triazolyl, tetrazolyl, oxazolyl, isoxazolyl, oxadiazolyl, thiazolyl, isothiazolyl, thiadiazolyl, pyridyl, pyridazinyl, pyrimidinyl, pyrazinyl, indolizinyl, purinyl, naphthyridinyl, pteridinyl, imidazo[1,2-a]pyrimidinyl, imidazo[1,2-a]pyridyl, imidazo[4,5-b]pyridyl, imidazo[4,5-c]pyridyl, pyrrolopyridyl, pyrrolopyrazinyl, thienopyrimidinyl, triazolopyridyl, and benzoisoxazolyl. The terms "heteroaryl" and "heteroar-", as used herein, also include groups in which a heteroaromatic ring is fused to one or more aryl, cycloaliphatic, or heterocyclyl rings, where the radical or point of attachment is on the heteroaromatic ring (i.e., a bicyclic heteroaryl ring having 1 to 3 heteroatoms). Nonlimiting examples include indolyl, isoindolyl, benzothienyl, benzofuranyl, dibenzofuranyl, indazolyl, benzimidazolyl, benzotriazolyl, benzothiazolyl, benzothiadiazolyl, benzoxazolyl, quinolyl, isoquinolyl, cinnolinyl, phthalazinyl, quinazolinyl, quinoxalinyl, 4*H*-quinolizinyl, carbazolyl, acridinyl, phenazinyl, phenothiazinyl, phenoxazinyl, tetrahydroquinolinyl, tetrahydroisoquinolinyl, pyrido[2,3-b]-1,4-oxazin-3(4H)-one, 4H-thieno[3,2-b]pyrrole, and benzoisoxazolyl. A heteroaryl group may be mono- or bicyclic. The term "heteroaryl" may be used interchangeably with the terms "heteroaryl ring", "heteroaryl group", or "heteroaromatic", any of which terms include rings that are optionally substituted. The term "heteroaralkyl" refers to an alkyl group substituted by a heteroaryl, wherein the alkyl and heteroaryl portions independently are optionally substituted.

***Heteroatom:*** The term "heteroatom" as used herein refers to nitrogen, oxygen, or sulfur, and includes any oxidized form of nitrogen or sulfur, and any quaternized form of a basic nitrogen.

*Heterocycle:* As used herein, the terms "heterocycle", "heterocyclyl", "heterocyclic radical", and "heterocyclic ring" are used interchangeably and refer to a stable 3- to 8-membered monocyclic, a 7- to 12-membered bicyclic, or a 10- to 16-membered polycyclic heterocyclic moiety that is either saturated or partially unsaturated, and having, in addition to carbon atoms, one or more, such as one to four, heteroatoms, as defined above. When used in reference to a ring atom of a heterocycle, the term "nitrogen" includes a substituted nitrogen. As an example, in a saturated or partially unsaturated ring having 0-3 heteroatoms selected from oxygen, sulfur or nitrogen, the nitrogen may be N (as in 3,4-dihydro-2H-pyrrolyl), NH (as in pyrrolidinyl), or NR⁺ (as in N-substituted pyrrolidinyl). A heterocyclic ring can be attached to its pendant group at any heteroatom or carbon atom that results in a stable structure and any of the ring atoms can be optionally substituted. Examples of such saturated or partially unsaturated heterocyclic radicals include, without limitation, azetidinyl, oxetanyl, tetrahydrofuranyl, tetrahydrothienyl, pyrrolidinyl, piperidinyl, decahydroquinolinyl, oxazolidinyl, piperazinyl, dioxanyl, dioxolanyl, diazepinyl, oxazepinyl, thiazepinyl, morpholinyl, and thiamorpholinyl. A heterocyclyl group may be mono-, bi-, tri-, or polycyclic, preferably mono-, bi-, or tricyclic, more preferably mono- or bicyclic. The term "heterocyclylalkyl" refers to an alkyl group substituted by a heterocyclyl, wherein the alkyl and heterocyclyl portions independently are optionally substituted. A bicyclic heterocyclic ring also includes groups in which the heterocyclic ring is fused to one or more aryl rings. Exemplary bicyclic heterocyclic groups include indolinyl, isoindolinyl, benzodioxolyl, 1,3-dihydroisobenzofuranyl, 2,3-dihydrobenzofuranyl, tetrahydroquinolinyl, 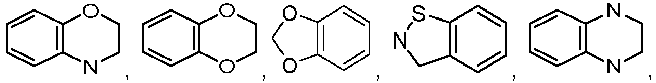 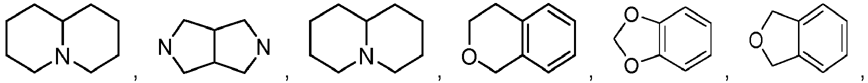 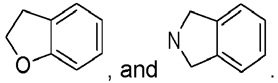 A bicyclic heterocyclic ring can also be a spirocyclic ring system (e.g., 7- to 11-membered spirocyclic fused heterocyclic ring having, in addition to carbon atoms, one or more heteroatoms as defined above (e.g., one, two, three or four heteroatoms)). A bicyclic heterocyclic ring can also be a bridged ring system (e.g., 7- to 11-membered bridged heterocyclic ring having one, two, or three bridging atoms.

***Immune response:*** The term "immune response" includes any response associate with immunity including, but not limited to, increases in cytokine expression, production or secretion (e.g., IL-1, IL-6, IL-7, TNFα expression, production or secretion).

The phrase "modulating an immune response" or "modulation of an immune response" or the term "immunomodulation" refers to the induction of an immune response or the modification of the immune system status. It includes upregulation, potentiating, stimulating, enhancing or increasing an immune response, as well as downregulation, counteracting, depleting or decreasing an immune response, as defined herein.

***Nucleic acid*/ *Polynucleotide:*** As used herein, the term "nucleic acid" refers to a polymer of at least 10 nucleotides or more. In some embodiments, a nucleic acid is or comprises DNA. In some embodiments, a nucleic acid is or comprises RNA. In some embodiments, a nucleic acid is or comprises peptide nucleic acid (PNA). In some embodiments, a nucleic acid is or comprises a single stranded nucleic acid. In some embodiments, a nucleic acid is or comprises a double-stranded nucleic acid. In some embodiments, a nucleic acid comprises both single and double-stranded portions. In some embodiments, a nucleic acid comprises a backbone that comprises one or more phosphodiester linkages. In some embodiments, a nucleic acid comprises a backbone that comprises both phosphodiester and non-phosphodiester linkages. For example, in some embodiments, a nucleic acid may comprise a backbone that comprises one or more phosphorothioate or 5'-N-phosphoramidite linkages and/or one or more peptide bonds, *e.g.,* as in a "peptide nucleic acid". In some embodiments, a nucleic acid comprises one or more, or all, natural residues (*e.g.,* adenine, cytosine, deoxyadenosine, deoxycytidine, deoxyguanosine, deoxythymidine, guanine, thymine, uracil). In some embodiments, a nucleic acid comprises on or more, or all, non-natural residues. In some embodiments, a non-natural residue comprises a nucleoside analog (*e.g.,* 2-aminoadenosine, 2-thiothymidine, inosine, pyrrolo-pyrimidine, 3 - methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5 -propynyl-cytidine, C5-methylcytidine, 2-aminoadenosine, 7-deazaadenosine, 7-deazaguanosine, 8-oxoadenosine, 8-oxoguanosine, 6-O-methylguanine, 2-thiocytidine, methylated bases, intercalated bases, and combinations thereof). In some embodiments, a non-natural residue comprises one or more modified sugars (*e.g.,* 2'-fluororibose, ribose, 2'-deoxyribose, arabinose, and hexose) as compared to those in natural residues. In some embodiments, a nucleic acid has a nucleotide sequence that encodes a functional gene product such as an RNA or polypeptide. In some embodiments, a nucleic acid has a nucleotide sequence that comprises one or more introns. In some embodiments, a nucleic acid may be prepared by isolation from a natural source, enzymatic synthesis (*e.g.,* by polymerization based on a complementary template, *e.g., in vivo* or *in vitro,* reproduction in a recombinant cell or system, or chemical synthesis.

***Nucleic acid particle:*** A "nucleic acid particle" can be used to deliver nucleic acid to a target site of interest (e.g., cell, tissue, organ, and the like). A nucleic acid particle may be formed from at least one cationic or cationically ionizable lipid or lipid-like material, at least one cationic polymer such as protamine, or a mixture thereof and nucleic acid. Nucleic acid particles include lipid nanoparticle (LNP)-based and lipoplex (LPX)-based formulations.

***Nucleotide:*** As used herein, the term "nucleotide" refers to its art-recognized meaning. When a number of nucleotides is used as an indication of size, *e.g.,* of a polynucleotide, a certain number of nucleotides refers to the number of nucleotides on a single strand, *e.g.,* of a polynucleotide.

***Oral:*** The phrases "oral administration" and "administered orally" as used herein have their art-understood meaning referring to administration by mouth of a compound or composition.

***Parenteral:*** The phrases "parenteral administration" and "administered parenterally" as used herein have their art-understood meaning referring to modes of administration other than enteral and topical administration, usually by injection, and include, without limitation, intravenous, intramuscular, intra-arterial, intrathecal, intracapsular, intraorbital, intracardiac, intradermal, intraperitoneal, transtracheal, subcutaneous, subcuticular, intraarticulare, subcapsular, subarachnoid, intraspinal, and intrasternal injection and infusion.

***Partially unsaturated:*** As used herein, the term "partially unsaturated" refers to a ring moiety that includes at least one double or triple bond between ring atoms. The term "partially unsaturated" is intended to encompass rings having multiple sites of unsaturation, but is not intended to include aromatic (e.g., aryl or heteroaryl) moieties, as herein defined.

***Patient*** or ***subject:*** As used herein, the term "patient" or "subject" refers to any organism to which a provided composition is or may be administered, *e.g.,* for experimental, diagnostic, prophylactic, cosmetic, and/or therapeutic purposes. Typical patients or subjects include animals (*e.g.,* mammals such as mice, rats, rabbits, non-human primates, and/or humans). In some embodiments, a patient is a human. In some embodiments, a patient or a subject is suffering from or susceptible to one or more disorders or conditions. In some embodiments, a patient or subject displays one or more symptoms of a disorder or condition. In some embodiments, a patient or subject has been diagnosed with one or more disorders or conditions. In some embodiments, a patient or a subject is receiving or has received certain therapy to diagnose and/or to treat a disease, disorder, or condition.

***Pharmaceutical composition:*** As used herein, the term "pharmaceutical composition" refers to an active agent, formulated together with one or more pharmaceutically acceptable carriers. In some embodiments, the active agent is present in unit dose amount appropriate for administration in a therapeutic or dosing regimen that shows a statistically significant probability of achieving a predetermined therapeutic effect when administered to a relevant population. In some embodiments, pharmaceutical compositions may be specially formulated for administration in solid or liquid form, including those adapted for the following: oral administration, for example, drenches (aqueous or non-aqueous solutions or suspensions), tablets, *e.g.,* those targeted for buccal, sublingual, and systemic absorption, boluses, powders, granules, pastes for application to the tongue; parenteral administration, for example, by subcutaneous, intramuscular, intravenous or epidural injection as, for example, a sterile solution or suspension, or sustained-release formulation; topical application, for example, as a cream, ointment, or a controlled-release patch or spray applied to the skin, lungs, or oral cavity; intravaginally or intrarectally, for example, as a pessary, cream, or foam; sublingually; ocularly; transdermally; or nasally, pulmonary, and to other mucosal surfaces.

***Pharmaceutically acceptable:*** As used herein, the phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

***Pharmaceutically acceptable carrier*** As used herein, the term "pharmaceutically acceptable carrier" means a pharmaceutically-acceptable material, composition or vehicle, such as a liquid or solid filler, diluent, excipient, or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; pH buffered solutions; polyesters, polycarbonates and/or polyanhydrides; and other non-toxic compatible substances employed in pharmaceutical formulations. ***Pharmaceutically acceptable salt:*** The term "pharmaceutically acceptable salt" refers to salts or zwitterions of the compounds which are water or oil-soluble or dispersible, suitable for treatment of disorders without undue toxicity, irritation, and allergic response, commensurate with a reasonable benefit/risk ratio and effective for their intended use; *i.e.,* salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. The salts may be prepared during the final isolation and purification of the compounds or separately by reacting an amino group of the compounds with a suitable acid. For example, a compound may be dissolved in a suitable solvent, such as but not limited to methanol and water and treated with at least one equivalent of an acid, like hydrochloric acid. The resulting salt may precipitate out and be isolated by filtration and dried under reduced pressure. Alternatively, the solvent and excess acid may be removed under reduced pressure to provide a salt.

For example, S. M. Berge, et al. describes pharmaceutically acceptable salts in detail in J. Pharmaceutical Sciences, 66: 1-19 (1977). In some embodiments, pharmaceutically acceptable salts include, but are not limited to, nontoxic acid addition salts, which are salts of an amino group formed with inorganic acids such as hydrochloric acid, hydrobromic acid, phosphoric acid, sulfuric acid and perchloric acid or with organic acids such as acetic acid, maleic acid, tartaric acid, citric acid, succinic acid or malonic acid or by using other methods used in the art such as ion exchange. In some embodiments, pharmaceutically acceptable salts include, but are not limited to, adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, *p*-toluenesulfonate, undecanoate, valerate salts, and the like. Representative alkali or alkaline earth metal salts include sodium, lithium, potassium, calcium, magnesium, and the like. In some embodiments, pharmaceutically acceptable salts include, when appropriate, nontoxic ammonium, quaternary ammonium, and amine cations formed using counterions such as halide, hydroxide, carboxylate, sulfate, phosphate, nitrate, alkyl having from 1 to 6 carbon atoms, sulfonate and aryl sulfonate.

Basic addition salts may be prepared during the final isolation and purification of the disclosed compounds by reaction of a carboxyl group with a suitable base such as the hydroxide, carbonate, or bicarbonate of a metal cation such as lithium, sodium, potassium, calcium, magnesium, or aluminum, or an organic primary, secondary, or tertiary amine. Quaternary amine salts can be prepared, such as those derived from methylamine, dimethylamine, trimethylamine, triethylamine, diethylamine, ethylamine, tributylamine, pyridine, N,N-dimethylaniline, N- methylpiperidine, N-methylmorpholine, dicyclohexylamine, procaine, dibenzylamine, N,N-dibenzylphenethylamine, 1-ephenamine and N,N'-dibenzylethylenediamine, ethylenediamine, ethanolamine, diethanolamine, piperidine, piperazine, and the like. ***Polycyclic:*** As used herein, the term "polycyclic" refers to a saturated or unsaturated ring system having two or more rings (for example, heterocyclyl rings, heteroaryl rings, cycloalkyl rings, or aryl rings), having between 7 and 20 atoms, in which one or more carbon atoms are common to two adjacent rings. For example, in some embodiments, a polycyclic ring system refers to a saturated or unsaturated ring system having three or more rings (for example, heterocyclyl rings, heteroaryl rings, cycloalkyl rings, or aryl rings), having between 14 and 20 atoms, in which one or more carbon atoms are common to two adjacent rings. The rings in a polycyclic ring system may be fused (i.e., bicyclic or tricyclic), spirocyclic, or a combination thereof.

***Polypeptide:*** The term "polypeptide", as used herein, typically has its art-recognized meaning of a polymer of at least three amino acids or more. Those of ordinary skill in the art will appreciate that the term "polypeptide" is intended to be sufficiently general as to encompass not only polypeptides having a complete sequence recited herein, but also to encompass polypeptides that represent functional, biologically active, or characteristic fragments, portions or domains (*e.g.,* fragments, portions, or domains retaining at least one activity) of such complete polypeptides. In some embodiments, polypeptides may contain L-amino acids, D-amino acids, or both and/or may contain any of a variety of amino acid modifications or analogs known in the art. Useful modifications include, *e.g.,* terminal acetylation, amidation, methylation, *etc.* In some embodiments, polypeptides may comprise natural amino acids, non-natural amino acids, synthetic amino acids, and combinations thereof (*e.g.,* may be or comprise peptidomimetics).

***Prevent*** or ***prevention:*** As used herein, the terms "prevent" or "prevention", when used in connection with the occurrence of a disease, disorder, and/or condition, refer to reducing the risk of developing the disease, disorder and/or condition and/or to delaying onset of one or more characteristics or symptoms of the disease, disorder or condition. Prevention may be considered complete when onset of a disease, disorder or condition has been delayed for a predefined period of time.

***Reference:*** As used herein describes a standard or control relative to which a comparison is performed. For example, in some embodiments, an agent, animal, individual, population, sample, sequence or value of interest is compared with a reference or control agent, animal, individual, population, sample, sequence or value.

In some embodiments, a reference or control is tested and/or determined substantially simultaneously with the testing or determination of interest. In some embodiments, a reference or control is a historical reference or control, optionally embodied in a tangible medium. Typically, as would be understood by those skilled in the art, a reference or control is determined or characterized under comparable conditions or circumstances to those under assessment. Those skilled in the art will appreciate when sufficient similarities are present to justify reliance on and/or comparison to a particular possible reference or control.

***Sample:*** As used herein, the term "sample" typically refers to an aliquot of material obtained or derived from a source of interest. In some embodiments, a source of interest is a biological or environmental source. In some embodiments, a source of interest may be or comprise a cell, tissue, or organism, such as a microbe, a plant, or an animal (e.g., a human). In some embodiments, a source of interest is or comprises biological tissue or fluid. In some embodiments, a source of interest may be or comprise a preparation generated in a production run. In some embodiments, a sample is a "primary sample" obtained directly from a source of interest by any appropriate means. In some embodiments, as will be clear from context, the term "sample" refers to a preparation that is obtained by processing (e.g., by removing one or more components of and/or by adding one or more agents to) a primary sample. ***Substituted*** or ***optionally substituted:*** As described herein, compounds of the invention may contain "optionally substituted" moieties. In general, the term "substituted," whether preceded by the term "optionally" or not, means that one or more hydrogens of the designated moiety are replaced with a suitable substituent. "Substituted" applies to one or more hydrogens that are either explicit or implicit from the structure (e.g., 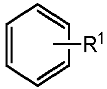 refers to at least 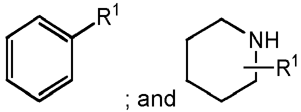 refers to at least 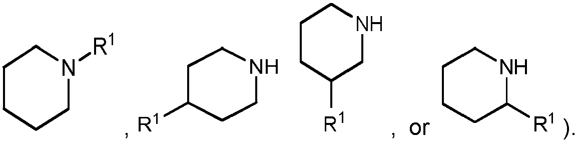 Unless otherwise indicated, an "optionally substituted" group may have a suitable substituent at each substitutable position of the group, and when more than one position in any given structure may be substituted with more than one substituent selected from a specified group, the substituent may be either the same or different at every position.

Combinations of substituents envisioned by this invention are preferably those that result in the formation of stable or chemically feasible compounds. The term "stable," as used herein, refers to compounds that are not substantially altered when subjected to conditions to allow for their production, detection, and, in certain embodiments, their recovery, purification, and use for one or more of the purposes provided herein. Groups described as being "substituted" preferably have between 1 and 4 substituents, more preferably 1 or 2 substituents. Groups described as being "optionally substituted" may be unsubstituted or be "substituted" as described above. Suitable monovalent substituents on a substitutable carbon atom of an "optionally substituted" group are independently halogen; -NO₂; -CN; -N₃; optionally substituted alkyl cycloalkyl, alkenyl alkynyl aryl or heteroaryl; -OR°; -SR°; NR°₂; -C(O)R°; - C(S)R°; -C(O)OR°; -C(S)SR°; -C(S)NR°₂; -C(NH)NR°₂; -OC(O)R°; -SC(O)R°; - N(R°)C(O)R°; OC(S)R°; -SC(S)R°; -N(R°)C(S)R°; OC(O)OR°; -SC(O)OR°; - N(R°)C(O)OR°; OC(S)SR°; -SC(S)SR°; -N(R°)C(S)SR°; OC(O)NR°₂; -SC(O)NR°₂; -N(R°)C(O)NR°₂; OC(S)NR°₂; -SC(S)NR°₂; -N(R°)C(S)NR°₂; -OSiR°₃, -S(O)₂NR°₂; S(O)₂NR°₂; -P(O)₂R°; P(O)R°₂; OP(O)R°₂; -OP(O)(OR°)₂; SiR°₃, wherein each R° is independently hydrogen or optionally substituted alkyl, cycloalkyl, alkenyl, alkynyl, aryl or heteroaryl.

Suitable divalent substituents on a saturated carbon atom of an "optionally substituted" group include the following: =O ("oxo"), =S, =NNR°₂, =NNHC(O)R°, =NNHC(O)OR°, =NNHS(O)₂R°, =NR°, or =NOR°, wherein. each R° is independently hydrogen or optionally substituted alkyl; cycloalkyl, alkenyl; alkynyl; aryl or heteroaryl. ***Small molecule:*** As used herein, the term "small molecule" means a low molecular weight organic and/or inorganic compound. In general, a "small molecule" is a molecule that is less than about 5 kilodaltons (kD) in size. In some embodiments, a small molecule is less than about 4 kD, 3 kD, about 2 kD, or about 1 kD. In some embodiments, the small molecule is less than about 800 daltons (D), about 600 D, about 500 D, about 400 D, about 300 D, about 200 D, or about 100 D. In some embodiments, a small molecule is not a polymer.

Those of ordinary skill in the art, reading the present disclosure, will appreciate that certain small molecule compounds described herein, including, for example, oligosaccharide compounds described herein, may be provided and/or utilized in any of a variety of forms such as, for example, crystal forms (e.g., polymorphs, solvates, etc), salt forms, protected forms, pro-drug forms, ester forms, isomeric forms (e.g., optical and/or structural isomers), isotopic forms, etc.

Those of ordinary skill in the art will appreciate that certain small molecule compounds (e.g., oligosaccharide compounds described herein) have structures that can exist in one or more steroisomeric forms. In some embodiments, such a small molecule may be utilized in accordance with the present disclosure in the form of an individual enantiomer, diastereomer or geometric isomer, or may be in the form of a mixture of stereoisomers; in some embodiments, such a small molecule may be utilized in accordance with the present disclosure in a racemic mixture form.

Those of skill in the art will appreciate that certain small molecule compounds (e.g., oligosaccharide compounds described herein) have structures that can exist in one or more tautomeric forms. In some embodiments, such a small molecule may be utilized in accordance with the present disclosure in the form of an individual tautomer, or in a form that interconverts between tautomeric forms.

Those of skill in the art will appreciate that certain small molecule compounds (e.g., oligosaccharide compounds described herein) have structures that permit isotopic substitution (e.g., ²H or ³H for H; ¹¹C, ¹³C or ¹⁴C for ¹²C; ¹³N or ¹⁵N for ¹⁴N; ¹⁷O or ¹⁸O for ¹⁶O; ³⁶Cl for ³⁵Cl or ³⁷Cl; ¹⁸F for ¹⁹F; ¹³¹I for ¹²⁷I; etc.). In some embodiments, such a small molecule may be utilized in accordance with the present disclosure in one or more isotopically modified forms, or mixtures thereof.

In some embodiments, reference to a particular small molecule compound (e.g., mono saccharide and oligosaccharide compounds described herein) may relate to a specific form of that compound. In some embodiments, a particular small molecule compound may be provided and/or utilized in a salt form (e.g., in an acid-addition or base-addition salt form, depending on the compound); in some such embodiments, the salt form may be a pharmaceutically acceptable salt form.

In some embodiments, where a small molecule compound is one that exists or is found in nature, that compound may be provided and/or utilized in accordance in the present disclosure in a form different from that in which it exists or is found in nature. Those of ordinary skill in the art will appreciate that, in some embodiments, a preparation of a particular small molecule compound (e.g., an oligosaccharide compound described herein) that contains an absolute or relative amount of the compound, or of a particular form thereof, that is different from the absolute or relative (with respect to another component of the preparation including, for example, another form of the compound) amount of the compound or form that is present in a reference preparation of interest (e.g., in a primary sample from a source of interest such as a biological or environmental source) is distinct from the compound as it exists in the reference preparation or source. Thus, in some embodiments, for example, a preparation of a single stereoisomer of a small molecule compound may be considered to be a different form of the compound than a racemic mixture of the compound; a particular salt of a small molecule compound may be considered to be a different form from another salt form of the compound; a preparation that contains only a form of the compound that contains one conformational isomer ((Z) or (E)) of a double bond may be considered to be a different form of the compound from one that contains the other conformational isomer ((E) or (Z)) of the double bond; a preparation in which one or more atoms is a different isotope than is present in a reference preparation may be considered to be a different form; etc.

Those skilled in the art will further appreciate that, in small molecule structures, the symbol , as used herein, refers to a point of attachment between two atoms.

***Therapeutic agent:*** As used herein, the phrase "therapeutic agent" in general refers to any agent that elicits a desired pharmacological effect when administered to an organism. In some embodiments, an agent is considered to be a therapeutic agent if it demonstrates a statistically significant effect across an appropriate population. In some embodiments, the appropriate population may be a population of model organisms. In some embodiments, an appropriate population may be defined by various criteria, such as a certain age group, gender, genetic background, preexisting clinical conditions, etc. In some embodiments, a therapeutic agent is a substance that can be used to alleviate, ameliorate, relieve, inhibit, prevent, delay onset of, reduce severity of, and/or reduce incidence of one or more symptoms or features of a disease, disorder, and/or condition. In some embodiments, a "therapeutic agent" is an agent that has been or is required to be approved by a government agency before it can be marketed for administration to humans. In some embodiments, a "therapeutic agent" is an agent for which a medical prescription is required for administration to humans.

***Treat:*** As used herein, the terms "treat," "treatment," or "treating" refer to any method used to partially or completely alleviate, ameliorate, relieve, inhibit, prevent, delay onset of, reduce severity of, and/or reduce incidence of one or more symptoms or features of a disease, disorder, and/or condition. Treatment may be administered to a subject who does not exhibit signs of a disease, disorder, and/or condition. In some embodiments, treatment may be administered to a subject who exhibits only early signs of the disease, disorder, and/or condition, for example, for the purpose of decreasing the risk of developing pathology associated with the disease, disorder, and/or condition.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Additional objects, advantages and features of the invention will become apparent to those skilled in the art upon examination of the description or may be learned by practice of the invention. The following examples and drawings are provided by way of illustration and are not intended to be limiting of the present invention.

### DESCRIPTION OF THE DRAWINGS

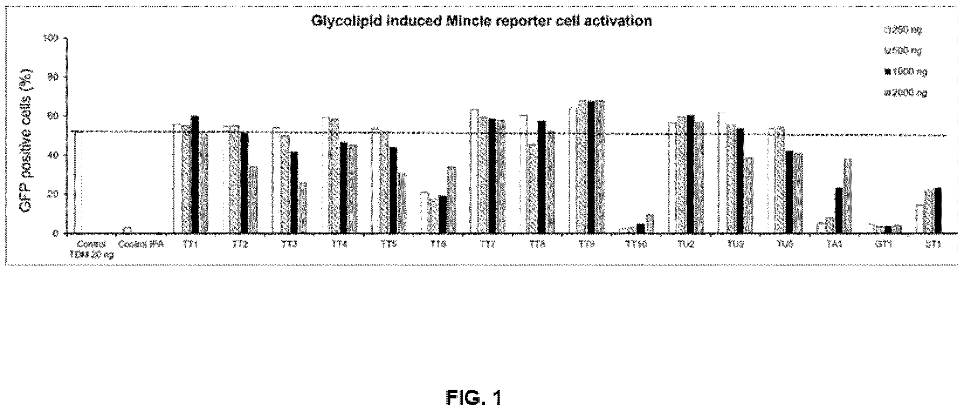

**FIG. 1****. Graph that shows the data obtained in the Mincle agonist glycolipid fast screening evaluation assay for selected compounds of the invention.** A high coating amount, multi-concentration (250 ng, 500 ng, 1000 ng and 2000 ng), fast screening flow cytometry analysis was performed to determine the ability of the following glycolipid structures to induce Mincle mediated cell reporter activation along with GFP expression. Evaluated samples in this assay: TDM (20 ng) as positive control, isopropanol (IPA) as negative control and compounds TT1, TT2, TT3, TT4, TT5, TT6, TT7, TT8, TT9, TT10, TU2, TU3, TU5, TA1, GT1 and ST1.

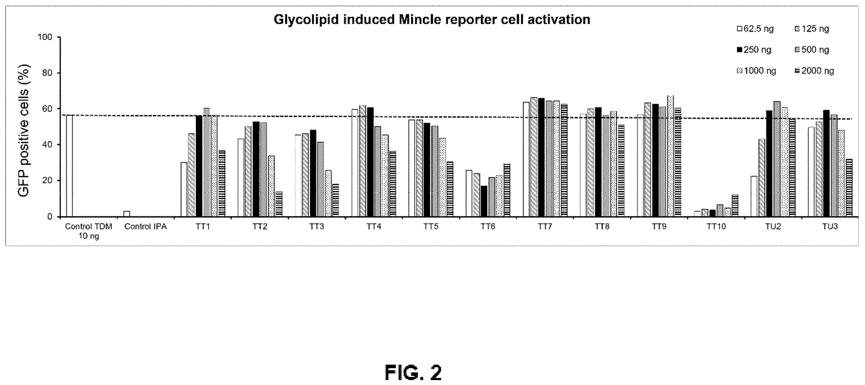

**Figure 2****. Graph that shows the data obtained for selected compounds of the invention in the concentration exploration analysis for GFP expression.** A wide range multi-concentration (62.5 ng, 125 ng, 250 ng, 500 ng, 1000 ng and 2000 ng) array was prepared using selected compounds of the invention, followed by GFP activation flow cytometry analysis. Evaluated samples in this assay: TDM (10 ng) as positive control, IPA as negative control and compounds TT1, TT2, TT3, TT4, TT5, TT6, TT7, TT8, TT9, TT10, TU2 and TU3.

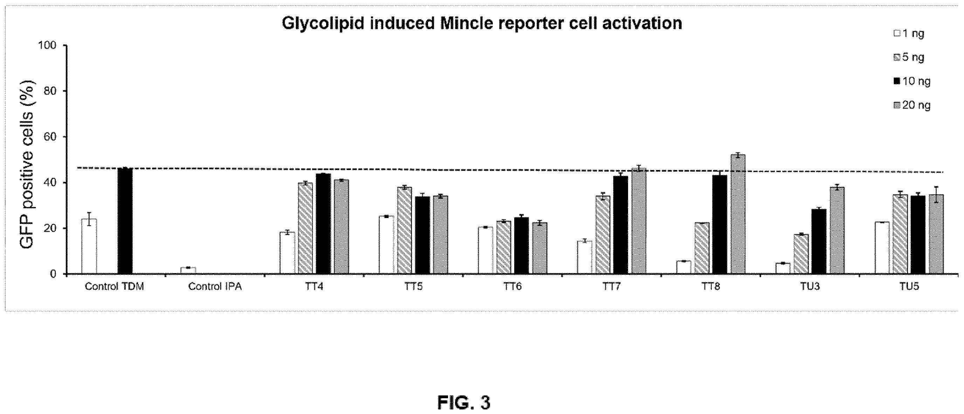

**Figure 3****. Graph that shows the data obtained in the fine structure-Mincle agonistic activity relationship analysis for selected compounds of the invention 01.** The GFP activation capacity of a selected glycolipid collection was evaluated by flow cytometry at control comparable concentrations (1 ng, 5 ng, 10 ng and 20 ng) by triplicate. Evaluated samples in this assay: TDM (1 ng and 10 ng) as positive control, IPA as negative control and compounds TT4, TT5, TT6, TT7, TT8 TU3 and TU5.

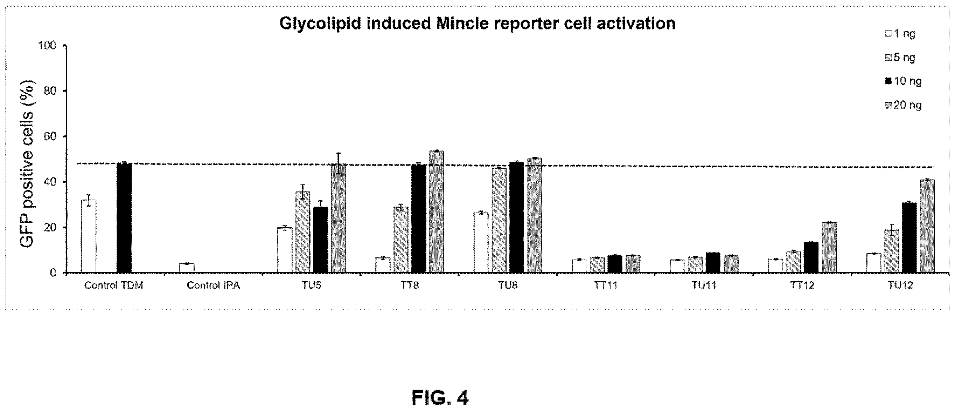

**Figure 4****. Graph that shows the data obtained in the fine structure-Mincle agonistic activity relationship analysis for selected compounds of the invention 02.** The GFP activation capacity of a selected glycolipid collection was evaluated by flow cytometry at control comparable concentrations (1 ng, 5 ng, 10 ng and 20 ng) by triplicate. Evaluated samples in this assay: TDM (1 ng and 10 ng) as positive control, IPA as negative control and compounds TU5, TT8, TU8, TT11, TU11, TT12 and TU 12.

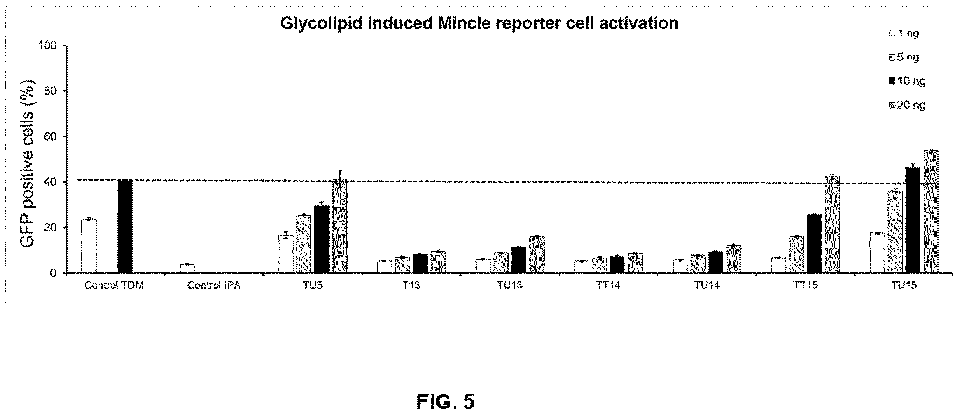

**Figure 5****. Graph that shows the data obtained in the fine structure-Mincle agonistic activity relationship analysis for selected compounds of the invention 03.** The GFP activation capacity of a selected glycolipid collection was evaluated by flow cytometry at control comparable concentrations (1 ng, 5 ng, 10 ng and 20 ng) by triplicate. Evaluated samples in this assay: TDM (1 ng and 10 ng) as positive control, IPA as negative control and compounds TU5, TT13, TU13, TT14, TU14, TT15 and TU15.

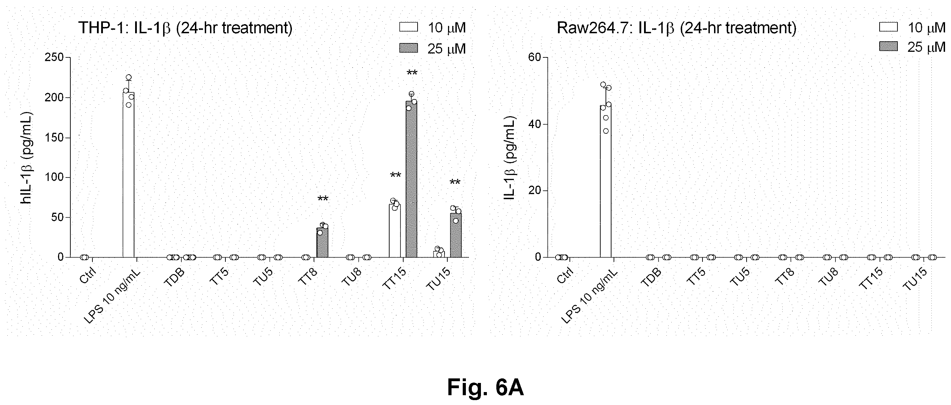

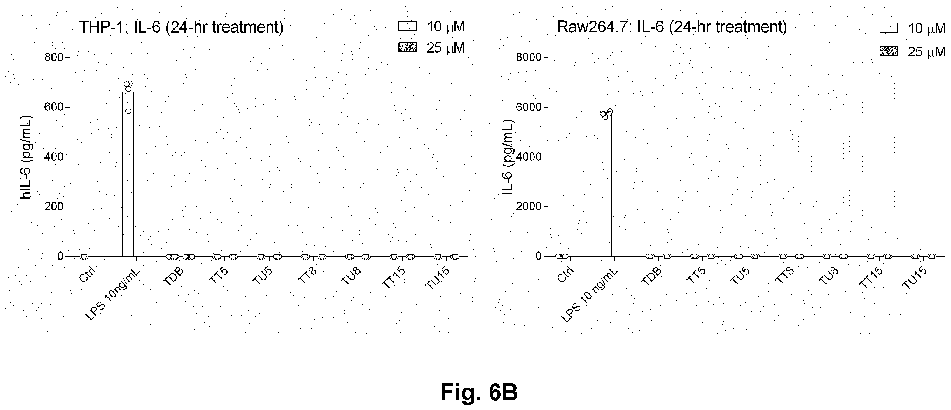

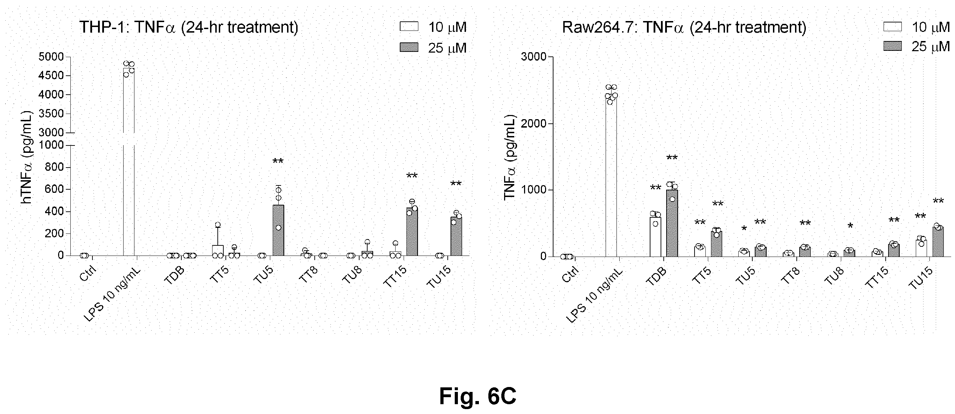

**Figure 6A-C****. Graphs that show the immunostimulant function of selected compounds of the invention in human THP-1 cells and mouse Raw264.7 cells.** THP-1 cells and Raw264.7 cells were treated with selected compounds, and the levels of cytokines IL-1β, IL-6 and TNFα in the culture supernatant were measured. Several compounds demonstrated immunostimulating functions and species-specific discrepancy in reactivity, in which compound TT15 showed the most prominent activity. Data are shown as means ± SEMs of two independent experiments (n = 3-6 each). *P < 0.05; **P < 0.01.

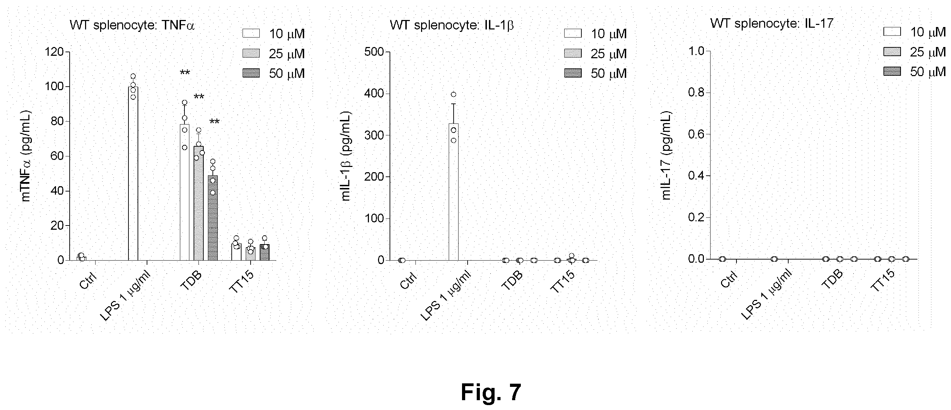

**Figure 7****. Graphs that show the immunostimulant function of TT15 in WT mouse splenocytes.** After 24 h treatment, compound TT15 was shown to induce TNFα, but not IL-1β and IL-17. Data are shown as means ± SEMs of two independent experiments (n = 4 each). *P < 0.05; **P < 0.01.

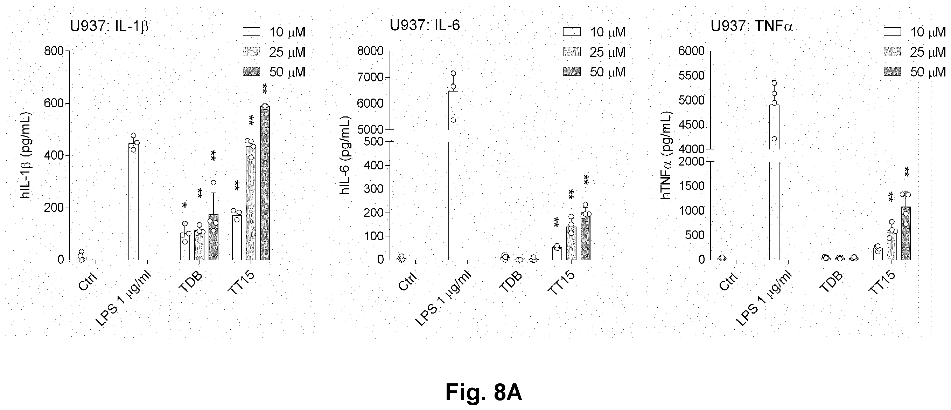

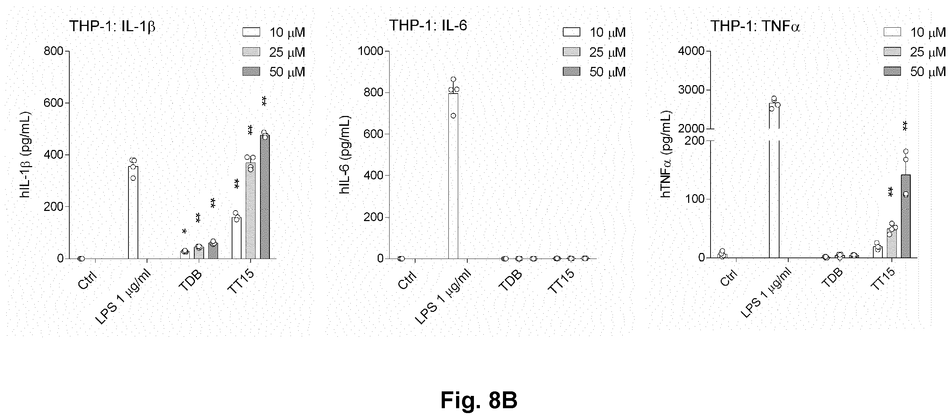

**Figure 8A-B****. Graphs that show the immunostimulant function of TT15 in human U937 and THP-1 cells.** U937 and THP-1 cells were treated with TT-15 for 24 h, and the levels of cytokines IL-1β, IL-6 and TNFα in the culture supernatant were measured. After 24 h treatment, compound TT15 was shown to induce IL-1β and TNFα in both cell lines, as well as IL-6 in the U937 cell line, but not in THP-1 cell line. Data are shown as means ± SEMs of two independent experiments (*n* = 4 each). **P* < 0.05; ***P* < 0.01.

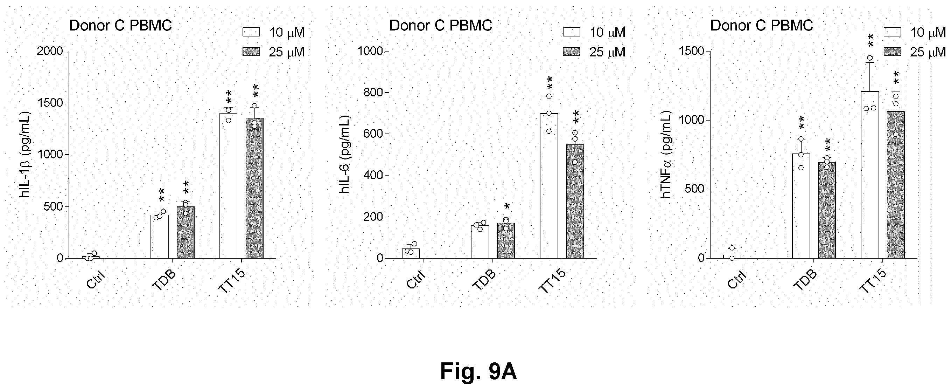

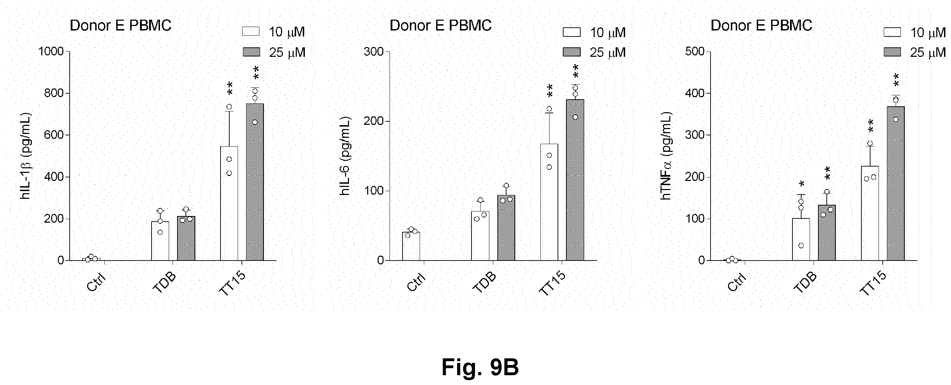

**Figure 9A-B****. Graphs that show the levels of IL-1β, IL-6 and TNFα induced by TT15 in human PBMCs.** PBMCs from two different donors were treated with TT15 for 24 h, and the levels of cytokines IL-1β, IL-6 and TNFα in the culture supernatant were measured. After 24 h treatment, compound TT15 was shown to induce IL-1β, IL-6 and TNFα in human PBMCs. Data are shown as means ± SEMs of two independent experiments (*n* = 3 each). **P* < 0.05; ***P* < 0.01.

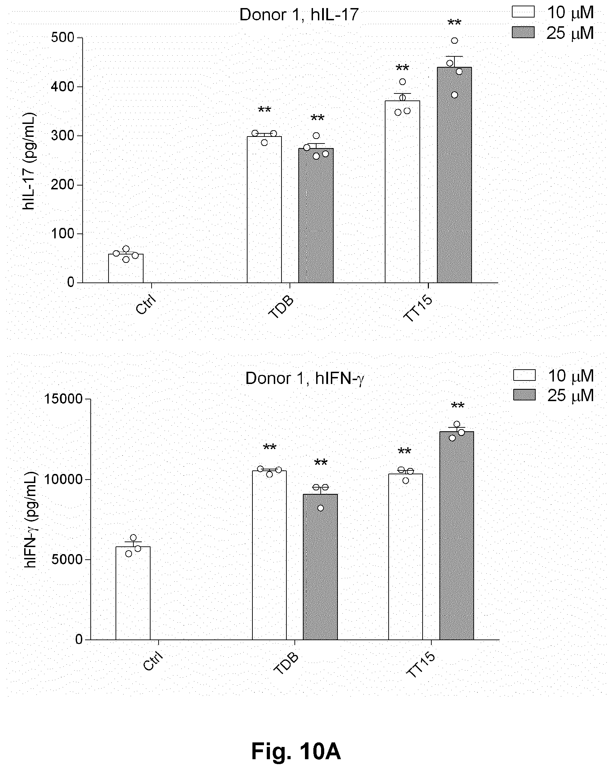

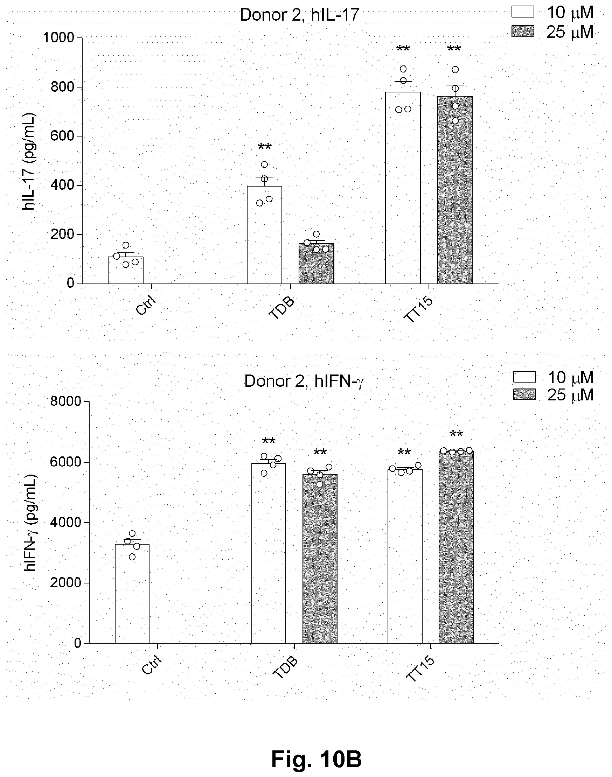

**Figure 10A-B****. Graphs that Show the levels of IL-17 and IFNγ induced by TT15 in human PBMCs.** PBMCs from two different donors were treated with TT15 for 96 h, and the levels of cytokines IL-17 and IFN-γ in the culture supernatant were measured. After 96 h treatment, compound TT15 was shown to induce IL-17 and IFN-γ in human PBMCs. Data are shown as means ± SEMs of two independent experiments (*n* = 3 each). ***P* < 0.01.

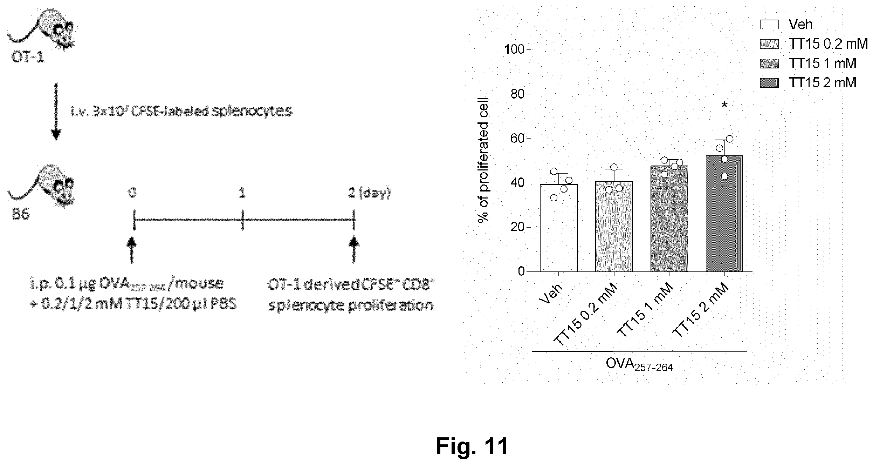

**Figure 11****. Graph that shows OVA-specific CD8⁺ T cell proliferation induced by TT15.** The ability of TT15 to function as an adjuvant was assessed in the OT-1 adoptive transfer mouse model, in which the administration of OVA in combination with TT15 was shown to induce the proliferation of OVA-specific CD8⁺ T cells. Data are shown as means ± SEMs of two independent experiments (n = 3-4 each). *P < 0.05.

### EXAMPLES

The Examples provided herein document and support certain aspects of the present disclosure but are not intended to limit the scope of any claim. The following nonlimiting examples are provided to further illustrate certain teachings provided by the present disclosure. Those of skill in the art, in light of the present application, will appreciate that various changes can be made in the specific embodiments that are illustrated in the present Examples without departing from the spirit and scope of the present teachings.

### Example 1 - Synthetic Examples

All reagents and solvents used in the preparations disclosed in the following examples were purchased from commercial sources and used without further purification, unless otherwise specified. Thin-layer chromatography (TLC) was carried out on aluminum sheets coated with Silica gel 60 F₂₅₄ Merck with visualization by UV light (λ 254 nm) and by charring with 10% ethanolic H₂SO₄, 0.1% ethanolic ninhydrin and heating at 100 °C. Column chromatography was carried out on Silice 60 A.C.C. Chromagel (SDS 70-200 and 35-70 µm). The structure of all compounds described herein, including synthetic intermediates and precursors, was confirmed by ¹H and ¹³C nuclear magnetic resonance (NMR) and mass spectrometry (MS). NMR experiments were performed at 300 (75.5), and 500 (125.7, 202) MHz with Bruker 300 ADVANCE and 500 DRX. 1D TOCSY, 2D COSY, HMQC and HSQC experiments were used to assist on NMR assignments. Mass spectra (High-resolution mass spectra) were carried out on a Bruker Daltonics Esquire6000^{™} (LTQ-Orbitrap XL ETD). The samples were introduced via solid probe heated from 30 to 280 °C. ESI as ionization source (Electrospray Ionization) was used to which methanol was used as solvent. The samples were introduced via direct injection using a Cole-Parmer syringe at a flow rate of 2 □l/min. Ions were scanned between 300 and 3000 Da with a scan speed of 13000 Da/s at unit resolution using resonance ejection at the multipole resonance of one-third of the radio frequency (Ω = 781.25 kHz).

### Methods

Scheme 1 depicts synthesis of a compound of formula I wherein A is A², X¹ and X² are both =S, Y¹ and Y² are both -N(R^{a})-, the R^{a} are identical and Z¹ and Z² are identical.

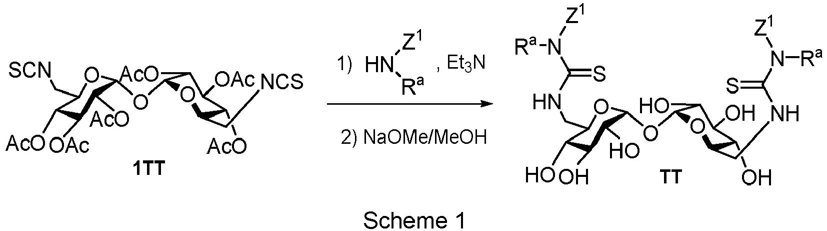

Scheme 2 depicts synthesis of a compound of formula I wherein A is A², X¹ and X² are both =O, Y¹ and Y² are both -NH-, and Z¹ and Z² are identical.

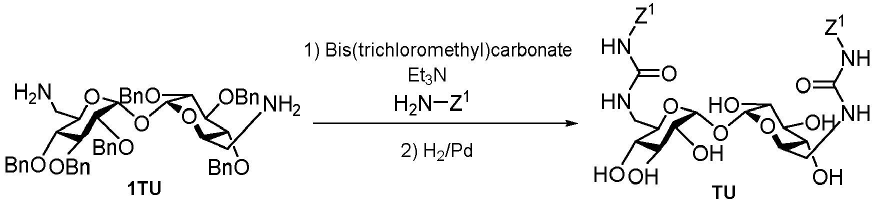

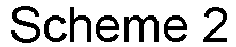

Scheme 3 depicts synthesis of a compound of formula I wherein A is A' and A¹ is Me, X¹ is =S, Y' is -NH-, and Z¹ is as described in classes and subclasses herein.

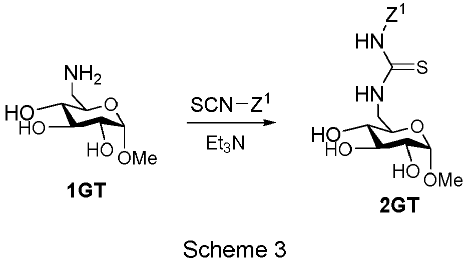

Scheme 4 depicts synthesis of a compound of formula I wherein A is A³, X¹ and X² are both =S, Y¹ and Y² are both -NH-, and Z¹ and Z² are identical.

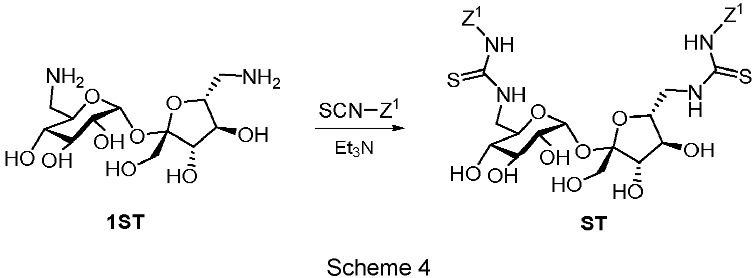

In exemplary embodiments, the synthesis of a compound of formula I wherein Y' and Y² are both -N(R^{a}) the R^{a} are identical and Z¹ and Z² are identical, according to Schemes 1 and 2, or the synthesis of a compound of formula I wherein A is A¹ and A¹ is Me, X¹ is =S, Y' is -NH-, and Z¹ is as described in classes and subclasses herein, comprises the following amine or isothiocyanate reagents:

| Reagent | R^{a} | Z¹ | Code |
|---|---|---|---|
| 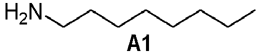 | H | 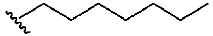 | **1** |
| 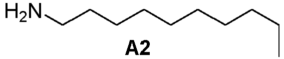 | H | 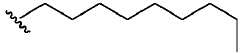 | **2** |
| 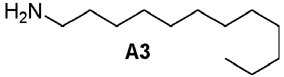 | H | 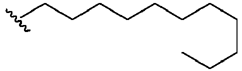 | **3** |
| 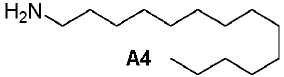 | H | 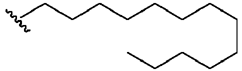 | **4** |
| 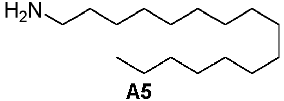 | H | 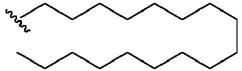 | **5** |
| 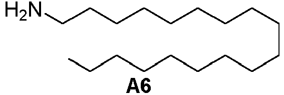 | H | 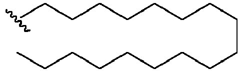 | **6** |
| 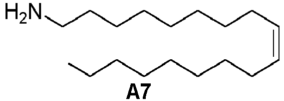 | H | 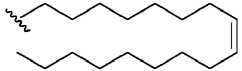 | **7** |
| 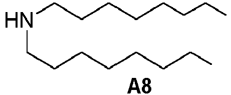 | 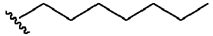 | 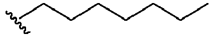 | **8** |
| 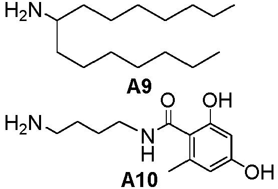 | H | 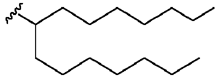 | **9** |
| | H | 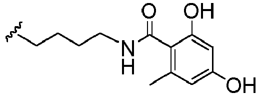 | **10** |
| 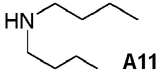 | 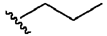 | 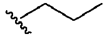 | **11** |
| 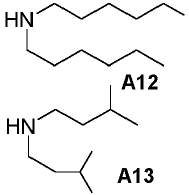 | 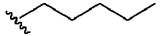 | 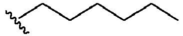 | **12** |
| | 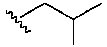 | 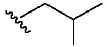 | **13** |
| 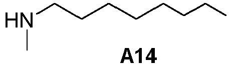 | Me | 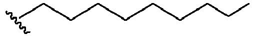 | **14** |
| 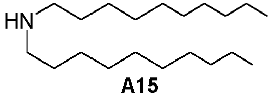 | 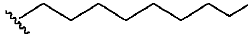 | 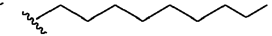 | **15** |
| 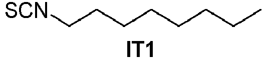 | H | 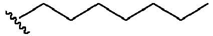 | **1** |

### Materials

In some exemplary embodiments, the following precursors used in the synthesis of specific compounds described herein were prepared according to known methods:
2,3,4,2',3',4'-Hexa-*O*-acetyl-6,6'-dideoxy-6,6'-diisothiocyanato-α,α'-trehalose **(1TT)** was prepared according to the procedure described in the literature: J. M. Garcia Fernández, C. Ortiz Mellet, J. L. Jiménez Blanco, J. Fuentes Mota, A. Gadelle, A. Coste-Sarguet, J. Dafaye, Carbohydr. Res. 1995, 268, 57-71.
2,3,4,2',3',4'-Hexa-*O*-benzyl-6,6'-dideoxy-6,6'-diiamino-α,α'-trehalose **(1TU)** was prepared according to the procedure described in the literature: J. Wang, B. Elchert, Y. Hui, J. Y. Takemoto, M. Bensaci, J. Wennergren, H. Chang, R. Rai, C. T. Chang, Bioorg. Med. Chem. 2004, 12, 6397-6413.
Methyl 6-amino-6-deoxy-α-d-glucopyranoside **(1GT)** was prepared according to the procedure described in the literature: M. V. Reddington, J. Chem. Soc. Perkin Trans. 1998, 1, 143-147.
6,6'-Diamino-6,6'-dideoxysucrose **(1ST)** was prepared according to the procedure described in the literature: J. M. Garcia Fernández, C. Ortiz Mellet, J. L. Jiménez Blanco, J. Fuentes Mota, A. Gadelle, A. Coste-Sarguet, J. Dafaye, Carbohydr. Res. 1995, 268, 57-71.
   As used herein, an arrow followed by a code between parentheses, e.g., "(→ **code)"** indicates the combination of R^{a} substituents (in a compound of formula I wherein Y¹ and Y² are both NR^{a}) and Z¹ and Z² substituents (in a compound of formula I wherein Z¹ and Z² are identical) that the preceding reagent provided during the synthesis. E.g., "octylamine A1 (→**1**)" is intended to refer to the combination of R^{a} and Z¹ substituents as described in the table above that was bonded to a compound through use of octylamine. Accordingly, reagents affording the same combination of substituents, e.g. octylamine (**A1**) and octyl isothiocyanate (**IT1**) share the same code (1)
1-Octyl- (CAS# 111-86-4, **A1**), 1-decyl- (CAS# 2016-57-1, **A2**), 1-dodecyl- (CAS# 124-22-1, **A3**), 1-tetradecyl- (CAS# 2016-42-4, **A4**), 1-hexadecyl- (CAS# 143-27-1, **A5**), 1-octadecyl- (CAS# 124-30-1, **A6**) and 1-oleyl-amine (CAS# 112-90-3, **A7**) were obtained from commercial sources.
N,N-Di-1-octylamine (CAS# 1120-48-5, **A8**) and pentadecan-8-amine (CAS# 18618-64-9, **A9**) were obtained from commercial sources.
N-(4-Aminobutyl)-2,4-dihydroxy-6-methylbenzamide (**A10**, as the corresponding hydrochloride salt) was prepared from commercially available 2,4-dihydroxy-6-methylbenzoic acid (CAS# 480-64-8) and 4-(*tert*-butoxycarbonylamino)butylamine (CAS# 68076-36-8) according to the following scheme (Scheme 5): 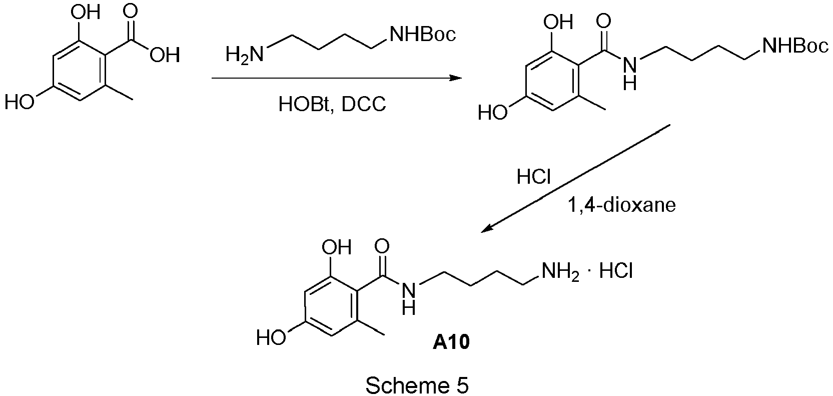
N,N-Di-1-butyl-(CAS# 111-92-2, **A11**), N,N-di-1-hexyl-(CAS# 143-16-8, **A12**), N,N-diisopentyl- (CAS# 544-00-3, **A13**), N-metyl-N-octyl- (CAS# 2439-54-5, **A14**), and N,N-di-1-decyl-amine (CAS# 1120-49-6, **A15**) were obtained from commercial sources.

### General procedure for the synthesis of a compound of formula I according to Scheme 1.

To a solution of 2,3,4,2',3',4'-hexa-*O*-acetyl-6,6'-dideoxy-6,6'-diisothiocyanato-α,α'-trehalose (**1TT**; 100 mg, 0.14 mmol) in DCM (3 mL), the corresponding amine reagent (0.35 mmol) and Et₃N (50 µl, 0.35 mmol) were added. The solution was stirred for 16 h at RT. The reaction mixture was diluted with DCM (5 mL), washed with 1 M HCl (3 × 5 mL) and the organic phase was dried (MgSO4), filtered and concentrated. The crude product was dissolved in MeOH (3 mL) and treated with a solution of 1 M NaOMe in MeOH (84 µL), followed by neutralization with Amberlite^{®} IR120 (H⁺ form), filtration, and purification by column chromatography (100:10:1 to 70:10:1 DCM-MeOH-H₂O).

### General procedure for the synthesis of a compound of formula I according to Scheme 2.

To a solution of 2,3,4,2',3',4'-hexa-*O*-benzyl-6,6'-dideoxy-6,6'-diiamino-α,α'-trehalose (**1TU**; 200 mg, 0.22 mmol) in DCM (5 mL), triphosgene (45 mg, 0.15 mmol) and Et₃N (130 µL, 0.91 mmol) were added at -78 °C and the mixture was stirred for 10 min under Ar atmosphere. Then, the corresponding alkylamine (0.46 mmol) was added at 0 °C and the reaction was stirred for 16 h. The solution was diluted with EtOAc (5 mL), extracted with H₂O (3 × 10 mL) and the organic phase was washed with 1 M HCl (3 × 10 mL), dried (MgSO4), filtered and concentrated. The crude residue was dissolved in degasified MeOH (5 mL), Pd/C (20 mg) was added and the reaction was stirred at RT during 16 h under H₂ atmosphere. The reaction was filtered through a short pad of Celite, concentrate and the resulting residue was purified by column chromatography (70:10:1 DCM-MeOH-H₂O).

### General procedure for the synthesis of a compound of formula I according to Schemes 3 and 4.

To a solution of methyl 6-amino-6-deoxy-α-D-glucopyranoside or 6,6'-diamino-6,6'-dideoxysucrose (**1GT** or **1ST,** respectively; 248 mg, 1.28 mmol) in DMF (7 mL), Et3N (360 µL, 2.56 mmol) and octyl isothiocyanate (0.5 mL, 2.56 mmol) were added and the solution was stirred at RT for 16 h. The solvent was evaporated and the crude residue was purified by column chromatography using the eluent indicated in each case.

### Example 1a - 6,6'-Dideoxy-6,6'-di-(N'-octylthioureido)-α,α'-trehalose (compound TT1).

Synthesized from compound **1TT** and amine **A1** (→ **1**) according to Scheme 1. Yield: 46%. *R_{f}* 0.50 (50:10:1 DCM-MeOH-H₂O). [α]_{D} +27.93 (c 1.0, 1:1 DCM-MeOH). ¹H NMR (500 MHz, CD₃OD): δ = 5.08 (d, 2 H, *J*_{1,2} = 3.8 Hz, H-1), 3.96 (m, 4 H, H-5, H-6a), 3.79 (t, 2 H, *J*_{2,3} = *J*_{3,4} = 9.4 Hz, H-3), 3.62 (bd, 2 H, *J*_{6a,6b} = 12.2 Hz, H-6b), 3.49 (bdd, 6 H, H-2, CH₂N), 3.22 (t, 2 H, *J*_{4',5'} = 9.4 Hz, H-4), 1.58 (m, 4 H, CH₂), 1.34-0.92 (m, 24 H, CH₂, CH₂CH₃). ¹³C NMR (125.7 MHz, CD₃OD): δ = 94.4 (C-1), 72.5 (C-3), 71.8 (C-2), 71.1 (C-4), 70.9 (C-5), 44.3 (CH₂N, C-6), 31.6-13.0 (CH₂, CH₂CH₃). ESIMS: *m*/*z* = 683.43 [M + H]⁺. Anal. calcd. for C₃₀H₅₈N₄O₉S₂: C 52.76, H 8.56, N 8.20, S 9.39. Found C 52.61, H 8.47, N 8.05, S 9.24.

### Example 1b - 6,6'-Dideoxy-6,6'-di-(N'-decylthioureido)-α,α'-trehalose (compound TT2).

Synthesized from compound **1TT** and amine **A2** (→ **2**) according to Scheme 1. Yield: 50%. *R_{f}*0.50 (50:10:1 DCM-MeOH-H₂O). [α]_{D} +18.47 (c 1.0 in 1:1 DCM-MeOH). ¹H NMR (500 MHz, CD₃OD): δ = 4.96 (d, 2 H, *J*_{1,2} = 3.8 Hz, H-1), 3.82 (m, 4 H, H-5, H-6a), 3.69 (t, 2 H, *J*_{2,3} = *J*_{3,4} = 9.4 Hz, H-3), 3.46 (bd, 2 H, *J*_{6a,6b} = 12.2 Hz, H-6b), 3.40 (bdd, 6 H, H-2, CH₂N), 3.16 (t, 2 H, *J*_{4',5'} = 9.4 Hz, H-4), 1.48 (m, 4 H, CH₂), 1.24-0.79 (m, 32 H, CH₂, CH₂CH₃). ¹³C NMR (125.7 MHz, CD₃OD): δ = 94.4 (C-1), 72.4 (C-3), 71.8 (C-2), 71.0 (C-4), 70.9 (C-5), 44.5 (CH₂N, C-6), 31.7-22.4 (CH₂, CH₂CH₃). ESIMS: *m*/*z* = 739.52 [M + H]⁺. Anal. calcd. for C₃₄H₆₆N₄O₉S₂: C 55.26, H 9.00, N 7.58, S 8.68. Found C 55.17, H 8.89, N 7.42, S 8.53.

### Example 1c - 6,6'-Dideoxy-6,6'-di-(N'-dodecylthioureido)-α,α'-trehalose (compound TT3).

Synthesized from compound **1TT** and amine **A3** (→ **3**) according to Scheme 1. Yield: 65%. *R_{f}* 0.60 (50:10:1 DCM-MeOH-H₂O). [α]_{D} +11.02 (c 1.0 in 1:1 DCM-MeOH). ¹H NMR (500 MHz, CD₃OD): δ = 4.96 (d, 2 H, *J*_{1,2} = 3.8 Hz, H-1), 3.82 (m, 4 H, H-5, H-6a), 3.69 (t, 2 H, *J*_{2,3} = *J*_{3,4} = 9.4 Hz, H-3), 3.46 (bd, 2 H, *J*_{6a,6b} = 12.2 Hz, H-6b), 3.40 (bdd, 6 H, H-2, CH₂N), 3.16 (t, 2 H, *J*_{4',5'} = 9.4 Hz, H-4), 1.48 (m, 4 H, CH₂), 1.24-0.79 (m, 40 H, CH₂, CH₂CH₃). ¹³C NMR (125.7 MHz, CD₃OD): δ = 94.4 (C-1), 72.4 (C-3), 71.8 (C-2), 71.0 (C-4), 70.9 (C-5), 44.6 (CH₂N, C-6), 31.7-22.4 (CH₂, CH₂CH₃). ESIMS: *m*/*z* = 795.56 [M + H]⁺. Anal. calcd. for C₃₈H₇₄N₄O₉S₂: C 57.40, H 9.38, N 7.05, S 8.06. Found C 57.48, H 9.46, N 6.85, S 7.87.

### Example 1d- 6,6'-Dideoxy-6,6'-di-(N'-tetradecylthioureido)-α,α'-trehalose (compound TT4).

Synthesized from compound **1TT** and amine **A4** (→ **4)** according to Scheme 1. Yield: 57%. Rr0.40 (60:10:1 DCM-MeOH-H₂O). [α]_{D} +10.77 (c 1.0 in 1:1 DCM-MeOH). ¹H NMR (500 MHz, CD₃OD): δ = 4.96 (d, 2 H, *J*_{1,2} = 3.8 Hz, H-1), 3.82 (m, 4 H, H-5, H-6a), 3.68 (t, 2 H, *J*_{2,3} = *J*_{3,4} = 9.4 Hz, H-3), 3.47 (bd, 2 H, *J*_{6a,6b} = 12.2 Hz, H-6b), 3.39 (bdd, 6 H, H-2, CH₂N), 3.15 (t, 2 H, *J*_{4',5'} = 9.4 Hz, H-4), 1.48 (m, 4 H, CH₂), 1.24-0.79 (m, 48 H, CH₂, CH₂CH₃). ¹³C NMR (125.7 MHz, CD₃OD): δ = 94.4 (C-1), 72.4 (C-3), 71.8 (C-2), 71.0 (C-4), 70.9 (C-5), 44.4 (CH₂N, C-6), 31.7-13.4 (CH₂, CH₂CH₃). ESIMS: *m*/*z* = 851.68 [M + H]⁺. Anal. calcd. for C₄₂H₈₂N₄O₉S₂: C 59.26, H 9.71, N 6.58, S 7.53. Found C 59.37, H 9.80, N 6.43, S 7.38.

### Example 1e - 6,6'-Dideoxy-6,6'-di-(N'-hexadecylthioureido)-α,α'-trehalose (compound TT5).

Synthesized from compound **1TT** and amine **A5** (→ **5)** according to Scheme 1. Yield: 60 %. Rr0.30 (70:10:1 DCM-MeOH-H₂O). [α]_{D} +15.86 (c 1.0 in 1:1 DCM-MeOH). ¹H NMR (500 MHz, CD₃OD): δ = 4.96 (d, 2 H, *J*_{1,2} = 3.8 Hz, H-1), 3.82 (m, 4 H, H-5, H-6a), 3.69 (t, 2 H, *J*_{2,3} = *J*_{3,4} = 9.4 Hz, H-3), 3.47 (bd, 2 H, *J*_{6a,6b} = 12.2 Hz, H-6b), 3.39 (bdd, 6 H, H-2, CH₂N), 3.15 (t, 2 H, *J*_{4',5'} = 9.4 Hz, H-4), 1.50 (m, 4 H, CH₂), 1.24-0.79 (m, 56 H, CH₂, CH₂CH₃). ¹³C NMR (125.7 MHz, CD₃OD): δ = 94.4 (C-1), 72.4 (C-3), 71.8 (C-2), 71.0 (C-4), 70.9 (C-5), 44.4 (CH₂N, C-6), 31.7-13.4 (CH₂, CH₂CH₃). ESIMS: *m*/*z* = 907.71 [M + H]⁺. Anal. calcd. for C₄₆H₉₀N₄O₉S₂: C 60.89, H 10.00, N 6.17, S 7.07. Found C 61.01, H 10.15, N 6.02, S 6.79.

### Example 1f - 6,6'-Dideoxy-6,6'-di-(N'-octadecylthioureido)-α,α'-trehalose (compound TT6).

Synthesized from compound **1TT** and amine **A6** (→ **6**) according to Scheme 1. Yield: 85 mg (63%). *R_{f}* 0.35 (80:10:1 DCM-MeOH-H₂O). [α]_{D} +10.20 (c 1.0 in 1:1 DCM-MeOH). ¹H NMR (500 MHz, CD₃OD): δ = 4.96 (d, 2 H, *J*_{1,2} = 3.8 Hz, H-1), 3.83 (m, 4 H, H-5, H-6a), 3.69 (t, 2 H, *J*_{2,3} = *J*_{3,4} = 9.4 Hz, H-3), 3.47 (bd, 2 H, *J*_{6a,6b} = 12.2 Hz, H-6b), 3.39 (bdd, 6 H, H-2, CH₂N), 3.16 (t, 2 H, *J*_{4',5'} = 9.4 Hz, H-4), 1.48 (m, 4 H, CH₂), 1.24-0.79 (m, 64 H, CH₂, CH₂CH₃). ¹³C NMR (125.7 MHz, CD₃OD): δ = 94.4 (C-1), 72.4 (C-3), 71.8 (C-2), 71.0 (C-4), 70.9 (C-5), 44.4 (CH₂N, C-6), 31.7-13.4 (CH₂, CH₂CH₃). ESIMS: *m*/*z* = 961.94 [M - H]⁻. Anal. calcd. for C₅₀H₉₈N₄O₉S₂: C 62.33, H 10.25, N 5.82, S 6.66. Found C 62.46, H 10.33, N 5.58, S 6.52.

### Example 1g - 6,6'-Dideoxy-6,6'-di-(N'-oleylthioureido)-α,α'-trehalose (compound TT7).

Synthesized from compound **1TT** and amine **A7** (→ **7**) according to Scheme 1. Yield: 101 mg (74%). *R_{f}* 0.60 (60:10:1 DCM-MeOH-H₂O). [α]_{D} +4.72 (c 1.0 in DCM). ¹H NMR (500 MHz, CD₃OD): δ = 5.24 (bt, 4 H, CH=CH), 4.96 (d, 2 H, *J*_{1,2} = 3.8 Hz, H-1), 3.83 (m, 4 H, H-5, H-6a), 3.69 (t, 2 H, *J*_{2,3} = *J*_{3,4} = 9.4 Hz, H-3), 3.47 (bd, 2 H, *J*_{6a,6b} = 12.2 Hz, H-6b), 3.39 (bdd, 6 H, H-2, CH₂N), 3.15 (t, 2 H, *J*_{4',5'} = 9.4 Hz, H-4), 1.93 (m, 4 H, CH₂), 1.49-0.79 (m, 60 H, CH₂, CH₂CH₃). ¹³C NMR (125.7 MHz, CD₃OD): δ = 94.4 (C-1), 72.4 (C-3), 71.8 (C-2), 71.0 (C-4), 70.9 (C-5), 44.4 (CH₂N, C-6), 31.7-13.4 (CH₂, CH₂CH₃). ESIMS: *m*/*z* = 961.94 [M - H]⁻. Anal. calcd. for C₅₀H₉₄N₄O₉S₂: C 62.59, H 9.88, N 5.84, S 6.68. Found C 62.51, H 9.73, N 5.67, S 6.50.

### Example 1h - 6,6'-Dideoxy-6,6'-di-(N',N'-dioctylthioureido)-α,α'-trehalose (compound TT8).

Synthesized from compound **1TT** and amine **A8** (→ **8**) according to Scheme 1. Yield: 52%. *R_{f}* 0.50 (60:10:1 DCM-MeOH-H₂O). [α]_{D} -0.07 (c 1.0 in 1:1 DCM-MeOH). ¹H NMR (500 MHz, CD₃OD): δ = 4.95 (d, 2 H, *J*_{1,2} = 3.8 Hz, H-1), 4.14 (dd, 2 H, *J*_{6a,6b} = 13.5 Hz, *J*_{5,6a} = 4.8 Hz, H-6a), 3.89 (m, 2 H, H-5), 3.69 (t, 2 H, *J*_{2,3} = *J*_{3,4} = 9.4 Hz, H-3), 3.55 (bd, 6 H, H-6b, CH₂N), 3.37 (bdd, 6 H, H-2, CH₂N), 3.14 (t, 2 H, *J*_{4',5'}= 9.4 Hz, H-4), 1.48 (m, 4 H, CH₂), 1.55-0.80 (m, 56 H, CH₂, CH₂CH₃). ¹³C NMR (125.7 MHz, CD₃OD): δ = 180.8 (CS), 94.7 (C-1), 72.4 (C-3), 71.9 (C-2), 71.5 (C-4), 70.7 (C-5), 44.6 (CH₂N, C-6), 31.6-13.4 (CH₂, CH₂CH₃). ESIMS: *m*/*z* = 907.74 [M + H]⁺. Anal. calcd. for C₄₆H₉₀N₄O₉S₂: C 60.89, H 10.00, N 6.17, S 7.07. Found C 60.67, H 9.89, N 5.94, S 6.83.

### Example 1i - 6,6'-Dideoxy-6,6'-di-(N'-pentadec-8-ylthioureido)-α,α'-trehalose (compound TT9).

Synthesized from compound **1TT** and amine **A9** (→ **9**) according to Scheme 1. Yield: 55%. *R_{f}* 0.51 (60:10:1 DCM-MeOH-H₂O). [α]_{D} -0.10 (c 1.0 in 1:1 DCM-MeOH). ¹H NMR (500 MHz, CD₃OD): δ = 4.97 (d, 2 H, *J*_{1,2} = 3.8 Hz, H-1), 3.84 (m, 4 H, H-5, H-6a), 3.69 (t, 2 H, *J*_{2,3} = *J*_{3,4} = 9.4 Hz, H-3), 3.46 (bd, 2 H, *J*_{6a,6b} = 12.2 Hz, H-6b), 3.37 (dd, 2 H, H-2), 3.14 (t, 2 H, *J*_{4',5'} = 9.4 Hz, H-4), 1.38 (m, 8 H, CH₂), 1.24-0.79 (m, 52 H, CH₂, CH₂CH₃). ¹³C NMR (125.7 MHz, CD₃OD): δ = 94.4 (C-1), 72.4 (C-3), 71.8 (C-2), 71.0 (C-4), 70.9 (C-5), 44.4 (CH₂N, C-6), 31.7-13.4 (CH₂, CH₂CH₃). ESIMS: *m*/*z* = 879.71 [M - H]-. Anal. calcd. for C₄₄H₈₆N₄O₉S₂: C 60.10, H 9.86, N 6.37, S 7.29. Found C 59.76, H 9.65, N 6.21, S 7.02.

### Example 1j - 6,6'-Oideoxy-6,6'-di[4-(2,4-hydroxy-6-methylbenzamide)butylthioureido]-α,α'-trehalose (compound TT10).

Synthesized from compound **1TT** and amine **A10** (→ **10**) according to Scheme 1. Yield: 38%. *R_{f}* 0.20 (45:5:3 MeCN-MeOH-H₂O). [α]_{D} +18.02 (c 1.0 in 1:1 DCM-MeOH). ¹H NMR (500 MHz, CD₃OD): δ = 6.22 (s, 4 H, CHₐᵣₒₘ), 5.10 (d, 2 H, *J*_{1,2} = 3.8 Hz, H-1), 3.97 (m, 4 H, H-5, H-6a), 3.81 (t, 2 H, *J*_{2,3} = *J*_{3,4} = 9.4 Hz, H-3), 3.62 (m, 2 H, H-6b), 3.51 (bdd, 6 H, H-2, CH₂N), 3.45 (t, 2 H, *J*_{4',5'} 5.7 Hz, H-4), 3.25 (t, 2 H, CH₂N), 2.27 (s, 3 H, CH₃), 1.70 (bs, 8 H, CH₂). ¹³C NMR (125.7 MHz, CD₃OD): δ = 171.0 (CO), 159.2, 156.2, 137.6, 115.9, 108.2, 99.7 (Cₐᵣₒₘ), 94.2 (C-1), 72.5 (C-3), 71.8 (C-2), 71.1 (C-4, C-5), 46.5 (C-6), 39.6 (CH₂N), 26.0 (CH₂), 18.5 (CH₃). ESIMS: *m*/*z* = 901.36 [M + H]⁺. Anal. calcd. for C₃₈H₅₆N₆O₁₅S₂: C 50.66, H 6.26, N 9.33, S 7.12. Found C 50.37, H 5.98, N 9.07, S 6.84.

### Example 1k - 6,6'-Dideoxy-6,6'-di-(N',N'-dibutylthioureido)-α,α'-trehalose (compound TT11).

Synthesized from compound **1TT** and amine **A11** (→ **11**) according to Scheme 1. Yield: (50%. *R_{f}* 0.40 (100:10:1 DCM-MeOH-H₂O). [α]_{D} +12.7 (c 1.0 in MeOH). ¹H NMR (500 MHz, CD₃OD): δ = 5.08 (d, 2 H, *J*_{1,2} = 3.8 Hz, H-1), 4.22 (dd, 2 H, *J*_{6a,6b} = 13.6 Hz, *J*_{5,6a}= 4.8 Hz, H-6a), 4.04 (m, 2 H, H-5), 3.83 (t, 2 H, *J*_{2,3} = *J*_{3,4} = 9.4 Hz, H-3), 3.70 (dd, 2 H, *J*_{5,6b} = 14 Hz, H-6b), 3.56 (bd, 4 H, CH₂N), 3.48 (dd, 6 H, H-2), 3.24 (t, 2 H, *J*_{4',5'} = 9.4 Hz, H-4), 1.66, 1.38 (m, 8 H, CH₂), 1.00 (t, 6 H, CH₃). ¹³C NMR (125.7 MHz, CD₃OD): δ = 180.1 (CS), 94.7 (C-1), 72.3 (C-3), 72.0 (C-2), 71.6 (C-4), 70.7 (C-5), 50.6 (CH₂N, C-6), 29.1, 19.6 (CH₂), 12.9 (CH₃). ESIMS: *m*/*z =* 907.74 [M + H]⁺. Anal. calcd. for C₃₀H₅₈N₄O₉S₂: C 52.76, H 8.56, N 8.20, S 9.39. Found C 52.61, H 8.39, N 8.04, S 9.21.

### Example 11- 6,6'-Dideoxy-6,6'-di-(N',N'-dihexylthioureido)-α,α'-trehalose (compound TT12).

Synthesized from compound **1TT** and amine **A12** (→ **12)** according to Scheme 1. Yield: 34%. Rr0.55 (80:10:1 DCM-MeOH-H₂O). [α]_{D} +11.9 (c 1.0 in MeOH). ¹H NMR (500 MHz, CD₃OD): δ = 5.08 (d, 2 H, *J*_{1,2} = 3.8 Hz, H-1), 4.22 (dd, 2 H, *J*_{6a,6b} = 14.0 Hz, *J*_{5,6a}= 4.8 Hz, H-6a), 4.05 (m, 2 H, H-5), 3.82 (t, 2 H, *J*_{2,3} = *J*_{3,4} = 9.4 Hz, H-3), 3.69 (dd, 2 H, *J*_{5,6b} = 14 Hz, H-6b), 3.56 (bd, 4 H, CH₂N), 3.49 (dd, 6 H, H-2), 3.24 (t, 2 H, *J*_{4',5'} = 9.4 Hz, H-4), 1.67, 1.37 (m, 16 H, CH₂), 0.96 (t, 6 H, CH₃). ¹³C NMR (125.7 MHz, CD₃OD): δ = 180.1 (CS), 94.8 (C-1), 72.4 (C-3), 72.0 (C-2), 71.6 (C-4), 70.7 (C-5), 50.9 (CH₂N, C-6), 31.4, 26.9, 22.2 (CH₂), 13.0 (CH₃). ESIMS: *m*/*z* = 793.81 [M - H]-. Anal. calcd. for C₃₈H₇₄N₄O₉S₂: C 57.40, H 9.38, N 7.05, S 8.06. Found C 57.18, H 9.15, N 6.89, S 7.78.

### Example 1m - 6,6'-Dideoxy-6,6'-di-(N',N'-diisopentylthioureido)-α,α'-trehalose (compound TT13).

Synthesized from compound **1TT** and amine **A13** (→ **13)** according to Scheme 1. Yield: 57%. *R_{f}* 0.52 (80:10:1 DCM-MeOH-H₂O). [α]_{D} +7.5 (c 1.0 in MeOH). ¹H NMR (500 MHz, CD₃OD): δ = 5.09 (d, 2 H, *J*_{1,2} = 3.8 Hz, H-1), 4.26 (dd, 2 H, *J*_{6a,6b} = 13.6 Hz, *J*_{5,6a}= 4.8 Hz, H-6a), 4.04 (m, 2 H, H-5), 3.83 (t, 2 H, *J*_{2,3} = *J*_{3,4} = 9.4 Hz, H-3), 3.69 (dd, 2 H, *J*_{5,6b} = 14 Hz, H-6b), 3.57 (bd, 4 H, CH₂N), 3.49 (ddd, 6 H, H-2), 3.24 (t, 2 H, *J*_{4',5'} = 9.4 Hz, H-4), 1.58 (m, 12 H, CH₂, CH), 0.99 (d, 24 H, CH₃). ¹³C NMR (125.7 MHz, CD₃OD): δ = 180.0 (CS), 94.8 (C-1), 72.3 (C-3), 72.0 (C-2), 71.5 (C-4), 70.7 (C-5), 49.2 (CH₂N, C-6), 35.7 (CH), 25.9 (CH₂), 21.6, 21.5 (CH₃). ESIMS: *m*/*z* = 739.58 [M + H]⁺. Anal. calcd. for C₃₄H₆₆N₄O₉S₂: C 55.26, H 9.00, N 7.58, S 8.68. Found C 55.07, H 8.90, N 7.45, S 8.46.

### Example 1n - 6,6'-Dideoxy-6,6'-di-(N'-methyl,N'-octylthioureido)-α,α'-trehalose (compound TT14).

Synthesized from compound **1TT** and amine **A14** (→ **14)** according to Scheme 1. Yield: 39%. *R_{f}* 0.51 (80:10:1 DCM-MeOH-H₂O). [α]_{D} +3.4 (c 1.0 in MeOH). ¹H NMR (500 MHz, CD₃OD): δ = 5.09 (d, 2 H, *J*_{1,2} = 3.8 Hz, H-1), 4.15 (dd, 2 H, *J*_{6a,6b} = 14.0 Hz, *J*_{5,6a}= 4.8 Hz, H-6a), 4.04 (m, 2 H, H-5), 3.81 (t, 2 H, *J*_{2,3} = *J*_{3,4} = 9.4 Hz, H-3), 3.71 (dd, 6 H, *J*_{5,6b} = 14 Hz, H-6b, CH₂N), 3.50 (dd, 6 H, H-2), 3.24 (t, 2 H, *J*_{4',5'} = 9.4 Hz, H-4), 3.18 (s, 3 H, NCH₃), 1.65 (m, 4 H, CH₂), 1.36 (m, 20 H, CH₂), 0.94 (t, 6 H, CH₃). ¹³C NMR (125.7 MHz, CD₃OD): δ = 180.8 (CS), 94.8 (C-1), 72.5 (C-3), 71.9 (C-2), 71.6 (C-4), 70.7 (C-5), 53.2 (CH₂N, C-6), 31.1-22.3 (CH₂), 13.0 (CH₃). ESIMS: m/z *=* 711.55 [M + H]⁺. Anal. calcd. for CazHszNaOsSz: C 54.06, H 8.79, N 7.88, S 9.02. Found C 53.76, H 8.53, N 7.59, S 8.68.

### Example 1o - 6,6'-Dideoxy-6,6'-di-(N',N'-didecylthioureido)-α,α'-trehalose (compound TT15).

Synthesized from compound **1TT** and amine **A15** (→ **15**) according to Scheme 1. Yield: 101 mg (70%). *R_{f}* 0.55 (80:10:1 DCM-MeOH-H₂O). [α]_{D} +0.6 (c 1.0 in MeOH). ¹H NMR (500 MHz, CD₃OD): δ = 5.08 (d, 2 H, *J*_{1,2} = 3.8 Hz, H-1), 4.16 (dd, 2 H, *J*_{6a,6b} = 13.6 Hz, *J*_{5,6a}= 4.8 Hz, H-6a), 4.06 (m, 2 H, H-5), 3.82 (t, 2 H, *J*_{2,3} = *J*_{3,4} = 9.4 Hz, H-3), 3.74 (dd, 2 H, *J*_{5,6b} = 14 Hz, H-6b), 3.56 (bd, 4 H, CH₂N), 3.49 (dd, 6 H, H-2), 3.24 (t, 2 H, *J*_{4',5'} = 9.4 Hz, H-4), 1.67 (m, 16 H, CH₂), 1.34 (m, 48 H, CH₂), 0.94 (t, 12 H, CH₃). ¹³C NMR (125.7 MHz, CD₃OD): δ = 180.1 (CS), 94.8 (C-1), 72.4 (C-3), 72.0 (C-2), 71.7 (C-4), 70.6 (C-5), 50.9 (CH₂N, C-6), 31.6-22.3 (CH₂), 13.0 (CH₃). ESIMS: *m*/*z* = 1019.98 [M + H]⁺. Anal. calcd. for C_{S4}H₁₀₆N₄O₉S₂: C 63.61, H 10.48, N 5.50, S 6.29. Found C 63.69, H 10.60, N 5.33, S 6.05.

### Example 1p - 6,6'-Dideoxy-6,6'-di-(N'-decylureido)-α,α'-trehalose (compound TU2).

Synthesized from compound **1TU** and amine **A2** (→ **2**) according to Scheme 2. Yield: 25%. *R_{f}* 0.42 (40:10:1 DCM-MeOH-H₂O). [α]_{D} +32.69 (c 1.0 in 1:1 DCM-MeOH). ¹H NMR (500 MHz, CD₃OD): δ = 4.95 (d, 2 H, *J*_{1,2} = 3.8 Hz, H-1), 3.72 (m, 2 H, H-5), 3.68 (t, 2 H, *J*_{2,3} = *J*_{3,4} = 9.4 Hz, H-3), 3.39 (m, 4 H, *J*_{6a,6b} = 12.2 Hz, H-6a, H-2), 3.20 (dd, 2 H, *J*_{5,6b} = 3.0 Hz, H-6b), 3.12 (t, 2 H, *J*_{4',5'} = 9.4 Hz, H-4), 3.01 (t, 4 H, CH₂N), 1.37 (m, 4 H, CH₂), 1.21-0.80 (m, 38 H, CH₂, CH₂CH₃). ¹³C NMR (125.7 MHz, CD₃OD): δ = 160.3 (CO), 94.0 (C-1), 77.8 (C-3), 72.6 (C-2), 71.9 (C-4), 71.2 (C-5), 40.5 (CH₂N), 39.9 (C-6), 31.7-13.4 (CH₂, CH₂CH₃). ESIMS: *m*/*z* = 707.55 [M + H]⁺. Anal. calcd. for C₃₄H₆₆N₄O₁₁: C 55.77, H 9.41, N 7.93. Found C 55.53, H 9.30, N 7.68.

### Example 1q - 6,6'-Dideoxy-6,6'-di-(N'-dodecylureido)-α,α'-trehalose (compound TU3).

Synthesized from compound **1TU** and amine **A3** (→ **3**) according to Scheme 2. Yield: 26%. *R_{f}* 0.42 (40:10:1 DCM-MeOH-H₂O). [α]_{D} +27.95 (c 1.0 in 1:1 DCM-MeOH). ¹H NMR (500 MHz, CD₃OD): δ = 4.95 (d, 2 H, *J*_{1,2} = 3.8 Hz, H-1), 3.72 (m, 2 H, H-5), 3.68 (t, 2 H, *J*_{2,3} = *J*_{3,4} = 9.4 Hz, H-3), 3.40 (m, 4 H, *J*_{6a,6b} = 12.2 Hz, H-6a, H-2), 3.21 (dd, 2 H, *J*_{5,6b} = 3.0 Hz, H-6b), 3.12 (t, 2 H, *J*_{4',5'} = 9.4 Hz, H-4), 3.01 (t, 4 H, CH₂N), 1.37 (m, 4 H, CH₂), 1.21-0.79 (m, 46 H, CH₂, CH₂CH₃). ¹³C NMR (125.7 MHz, CD₃OD): δ = 160.3 (CO), 94.0 (C-1), 72.6 (C-3), 71.9 (C-2), 71.2 (C-4, C-5), 40.5 (CH₂N), 39.9 (C-6), 31.7-13.4 (CH₂, CH₂CH₃). ESIMS: *m*/*z* = 763.64 [M + H]⁺. Anal. calcd. for C₃₈H₇₄N₄O₁₁. C 59.82, H 9.78, N 7.34. Found C 59.71, H 9.69, N 7.12.

### Example 1r - 6,6'-Dideoxy-6,6'-di-(N'-hexadecylureido)-α,α'-trehalose (compound TU5).

Synthesized from compound **1TU** and amine **A5** (→ **5**) according to Scheme 2. Yield: 29%. *R_{f}* 0.50 (60:10:1 DCM-MeOH-H₂O). [α]_{D} +18.70 (c 1.0 in 1:1 DCM-MeOH). ¹H NMR (500 MHz, CD₃OD): δ = 4.94 (d, 2 H, *J*_{1,2} = 3.8 Hz, H-1), 3.71 (m, 2 H, H-5), 3.68 (t, 2 H, *J*_{2,3} = *J*_{3,4} = 9.4 Hz, H-3), 3.39 (m, 4 H, *J*_{6a,6b} = 12.2 Hz, H-6a, H-2), 3.21 (dd, 2 H, *J*_{5,6b} = 3.0 Hz, H-6b), 3.13 (t, 2 H, *J*_{4',5'} = 9.4 Hz, H-4), 3.01 (t, 4 H, CH₂N), 1.37 (m, 4 H, CH₂), 1.21-0.79 (m, 60 H, CH₂, CH₂CH₃). ¹³C NMR (125.7 MHz, CD₃OD): δ = 160.3 (CO), 94.0 (C-1), 72.6 (C-3), 71.9 (C-2), 71.2 (C-4, C-5), 40.5 (CH₂N), 39.9 (C-6), 31.7-13.5 (CH₂, CH₂CH₃). ESIMS: *m*/*z* = 875.77 [M + H]⁺. Anal. calcd. for C₄₆H₉₀N₄O₁₁: C 63.13, H 10.37, N 6.40. Found C 63.24, H 10.45, N 6.30.

### Example 1s - 6,6'-Oideoxy-6,6'-di-(N',N'-dioctylthioureido)-α,α'-trehalose (compound TU8).

Synthesized from compound **1TT** and amine **A8** (→ **8**) according to Scheme 2. Yield: 59%. *R_{f}* 0.51 (80:10:1 DCM-MeOH-H₂O). [α]_{D} +26.5 (c 1.0 in MeOH). ¹H NMR (500 MHz, CD₃OD): δ = 5.07 (d, 2 H, *J*_{1,2} = 3.8 Hz, H-1), 3.92 (m, 2 H, H-5), 3.81 (t, 2 H, *J*_{2,3} = *J*_{3,4} = 9.4 Hz, H-3), 3.39 (m, 2 H, *J*_{6a,6b} = 14.3 Hz, *J*_{5,6a} = 5.4 Hz, H-6a), 3.48 (dd, 2 H, H-2), 3.41 (dd, 2 H, *J*_{5,6b}= 3.2 Hz, H-6b), 3.24 (m, 10 H, H-4, CH₂N), 1.58 (m, 8 H, CH₂), 1.35-0.94 (m, 52 H, CH₂, CH₂CH₃). ¹³C NMR (125.7 MHz, CD₃OD): δ = 159.0 (CO), 94.2 (C-1), 72.6 (C-3), 72.1 (C-2), 71.9 (C-4), 70.9 (C-5), 41.4 (C-6), 31.6-13.0 (CH₂, CH₂CH₃). ESIMS: *m*/*z* = 920.08 [M + HCO₂]⁻. Anal. calcd. for C₄₆H₉₀N₄O₁₁: C 63.13, H 10.37, N 6.40. Found C 63.24, H 10.47, N 6.28.

### Example 1t - 6,6'-Oideoxy-6,6'-di-(N',N'-dibutylthioureido)-α,α'-trehalose (compound TU11).

Synthesized from compound **1TU** and amine **A11** (→ **11**) according to Scheme 2. Yield: 73 mg (51%). *R_{f}* 0.42 (40:10:1 DCM-MeOH-H₂O). [α]_{D} +24.3 (c 1.0 in MeOH). ¹H NMR (500 MHz, CD₃OD): δ = 5.07 (d, 2 H, *J*_{1,2} = 3.8 Hz, H-1), 3.91 (m, 2 H, H-5), 3.81 (t, 2 H, *J*_{2,3} = *J*_{3,4} = 9.4 Hz, H-3), 3.58 (m, 2 H, *J*_{6a,6b} = 14.3 Hz, *J*_{5,6a} = 5.4 Hz, H-6a), 3.47 (dd, 2 H, H-2), 3.40 (dd, 2 H, *J*_{5,6b}= 3.2 Hz, H-6b), 3.24 (m, 10 H, H-4, CH₂N), 1.57 (m, 8 H, CH₂), 1.37 (m, 8 H, CH₂), 0.98 (t, 6 H, CH₃). ¹³C NMR (125.7 MHz, CD₃OD): δ = 159.0 (CO), 94.2 (C-1), 72.6 (C-3), 72.1 (C-2), 71.9 (C-4), 71.0 (C-5), 41.3 (C-6), 30.2, 19.7 (CH₂), 12.9 (CH₃). ESIMS: *m*/*z* = 695.59 [M + HCO₂]⁻. Anal. calcd. for C₃₀H₅₈N₄O₁₁: C 55.37, H 9.98, N 8.61. Found C 55.13, H 8.81, N 8.37.

### Example 1u - 6,6'-Dideoxy-6,6'-di-(N',N'-dihexylthioureido)-α,α'-trehalose (compound TU12).

Synthesized from compound **1TU** and amine **A12** (→ **12**) according to Scheme 2. Yield: 64 mg (38%). *R_{f}* 0.48 (60:10:1 DCM-MeOH-H₂O). [α]_{D} +10.7 (*c* 1.0 in MeOH). ¹H NMR (500 MHz, CD₃OD): δ = 5.07 (d, 2 H, *J*_{1,2} = 3.8 Hz, H-1), 3.92 (m, 2 H, H-5), 3.81 (t, 2 H, *J*_{2,3} = *J*_{3,4} = 9.4 Hz, H-3), 3.58 (m, 2 H, *J*_{6a,6b} = 14.3 Hz, *J*_{5,6a} = 5.4 Hz, H-6a), 3.48 (dd, 2 H, H-2), 3.40 (dd, 2 H, *J*_{5,6b}= 3.2 Hz, H-6b), 3.25 (m, 10 H, H-4, CH₂N), 1.58 (m, 8 H, CH₂), 1.35-0.94 (m, 36 H, CH₂, CH₂CH₃). ¹³C NMR (125.7 MHz, CD₃OD): δ = 159.0 (CO), 94.2 (C-1), 72.6 (C-3), 72.1 (C-2), 71.9 (C-4), 71.0 (C-5), 41.3 (C-6), 31.4-13.0 (CH₂, CH₂CH₃). ESIMS: *m*/*z* = 763.70 [M + H]⁺. Anal. calcd. for C₃₈H₇₄N₄O₁₁. C 59.82, H 9.78, N 7.34. Found C 59.58, H 9.50, N 7.09.

### Example 1 v - 6,6'-Dideoxy-6,6'-di-(N',N'-diisopentylthioureido)-α,α'-trehalose (compound TU13).

Synthesized from compound **1TU** and amine **A13** (→ **13**) according to Scheme 2. Yield: 98 mg (63%). *R_{f}* 0.52 (60:10:1 DCM-MeOH-H₂O). [α]_{D} +15.9 (c 1.0 in MeOH). ¹H NMR (500 MHz, CD₃OD): δ = 5.07 (d, 2 H, *J*_{1,2} = 3.8 Hz, H-1), 3.92 (m, 2 H, H-5), 3.81 (t, 2 H, *J*_{2,3} = *J*_{3,4} = 9.4 Hz, H-3), 3.59 (m, 2 H, *J*_{6a,6b} = 14.3 Hz, *J*_{5,6a} = 5.4 Hz, H-6a), 3.48 (dd, 2 H, H-2), 3.40 (dd, 2 H, *J*_{5,6b}= 3.2 Hz, H-6b), 3.25 (m, 10 H, H-4, CH₂N), 1.62 (m, 4 H, CH), 1.47 (m, 8 H, CH₂), 0.97 (d, 24 H, CH₃). ¹³C NMR (125.7 MHz, CD₃OD): δ = 158.9 (CO), 94.2 (C-1), 72.6 (C-3), 72.0 (C-2), 71.9 (C-4), 70.9 (C-5), 45.1 (CH₂N), 41.3 (C-6), 36.9 (CH), 25.8 (CH₂), 21.6, 21.5 (CH₃). ESIMS: *m*/*z =* 707.59 [M + H]⁺. Anal. calcd. for C₃₄H₆₆N₄O₁₁: C 57.77, H 9.41, N 7.93. Found C 57.86, H 9.56, N 7.74.

### Example 1w - 6,6'-Oideoxy-6,6'-di-(N'-methyl,N'-octylthioureido)-α,α'-treha/ose (compound TU14).

Synthesized from compound **1TU** and amine **A14** (→ **14**) according to Scheme 2. Yield: 116 mg (78%). *R_{f}* 0.54 (40:10:1 DCM-MeOH-H₂O). [α]_{D} +24.7 (c 1.0 in MeOH). ¹H NMR (500 MHz, CD₃OD): δ = 5.07 (d, 2 H, *J*_{1,2} = 3.8 Hz, H-1), 3.91 (m, 2 H, H-5), 3.80 (t, 2 H, *J*_{2,3} = *J*_{3,4} = 9.4 Hz, H-3), 3.56 (m, 2 H, *J*_{6a,6b} = 14.3 Hz, *J*_{5,6a} = 5.4 Hz, H-6a), 3.48 (dd, 2 H, H-2), 3.42 (dd, 2 H, *J*_{5,6b}= 3.2 Hz, H-6b), 3.29 (m, 4 H, CH₂N), 3.20 (t, 2 H, *J*_{4,5} = 9.4 Hz, H-4), 2.91 (s, 6 H, CH₃N), 1.58 (m, 4 H, CH₂), 1.35-0.94 (m, 26 H, CH₂, CH₂CH₃). ¹³C NMR (125.7 MHz, CD₃OD): δ = 159.6 (CO), 94.3 (C-1), 72.6 (C-3), 72.0 (C-2), 71.8 (C-4), 71.0 (C-5), 41.4 (C-6), 33.3-13.0 (CH₂, CH₂CH₃). ESIMS: *m*/*z* = 679.57 [M + H]⁺. Anal. calcd. for C₃₂H₆₂N₄O₁₁: C 56.62, H 9.21, N 8.25. Found C 56.67, H 9.22, N 8.11.

### Example 1x - 6,6'-Dideoxy-6,6'-di-(N',N'-didecylthioureido)-α,α'-trehalose (compound TU15).

Synthesized from compound **1TU** and amine **A15** (→ **15**) according to Scheme 2. Yield: 19%. *R_{f}* 0.50 (40:10:1 DCM-MeOH-H₂O). [α]_{D} +6.6 (c 1.0 in MeOH). ¹H NMR (500 MHz, CD₃OD): δ = 5.06 (d, 2 H, *J*_{1,2} = 3.8 Hz, H-1), 3.92 (m, 2 H, H-5), 3.81 (t, 2 H, *J*_{2,3} = *J*_{3,4} = 9.4 Hz, H-3), 3.55 (m, 2 H, *J*_{6a,6b} = 14.3 Hz, *J*_{5,6a} = 5.4 Hz, H-6a), 3.48 (dd, 2 H, H-2), 3.42 (dd, 2 H, *J*_{5,6b} = 3.2 Hz, H-6b), 3.29 (m, 10 H, CH₂N, H-4), 1.58 (m, 8 H, CH₂), 1.34-0.94 (m, 68 H, CH₂, CH₂CH₃). ¹³C NMR (125.7 MHz, CD₃OD): δ = 159.0 (CO), 94.2 (C-1), 72.6 (C-3), 72.1 (C-2), 71.9 (C-4), 70.9 (C-5), 41.4 (C-6), 31.6-13.0 (CH₂, CH₂CH₃). ESIMS: *m*/*z* = 988.02 [M + H]⁺. Anal. calcd. for C₅₄H₁₀₆N₄O₁₁: C 65.68, H 10.82, N 5.67. Found C 65.92, H 11.04, N 5.55.

### Example 1y- Methyl 6-deoxy-6-(N'-octylthioureido) α-D-glucopyranoside (compound GT1).

Synthesized from compound **1GT** and isothiocyanate **IT1** (→ **1**) according to Scheme 3. Column chromatography eluent: (100:10:1 DCM-MeOH-H₂O). Yield: 80%. *Rᵣ* 0.30 (100:10:1 DCM-MeOH-H₂O). [α]_{D} +17.23 (c 1.0 in 1:1 DCM-MeOH). ¹H NMR (500 MHz, CD₃OD): δ = 4.56 (d, 1 H, *J*_{1,2} = 3.7 Hz, H-1), 3.51 (m, 4 H, H-5, H-6a), 3.29 (s, 3 H, CH₃), 3.26 (t, 2 H, *J*_{2,3} = *J*_{3,4} = 9.4 Hz, H-3), 3.62 (bd, 2 H, *J*_{6a,6b} = 12.2 Hz, H-6b), 3.49 (bdd, 6 H, H-2, CH₂N), 3.07 (t, 2 H, *J*_{4',5'} = 9.4 Hz, H-4), 1.58 (m, 4 H, CH₂), 1.34-0.92 (m, 15 H, CH₂, CH₂CH₃). ¹³C NMR (125.7 MHz, CD₃OD): δ = 99.9 (C-1), 73.0 (C-3), 72.2 (C-2), 71.2 (C-4), 70.5 (C-5), 54.2 (CH₂N, C-6), 31.6-13.0 (CH₂, CH₂CH₃). ESIMS: *m*/*z* = 365.21 [M + H]⁺. Anal. calcd. for C₁₆H₃₂N₂O₅S: C 52.72, H 8.85, N 7.69, S 8.80. Found C 52.49, H 8.63, N 7.42, S 8.56.

### Example 1z - 6,6'-Dideoxy-6,6'-di-(N'-octylthioureido)sucrose (compound GT1).

Synthesized from compound **1ST** and isothiocyanate **IT1** (→ **1**) according to Scheme 4. Column chromatography eluent: 70:10:1 DCM-MeOH-H₂O. Yield: 82%. *R_{f}* 0.30 (60:10:1 DCM-MeOH-H₂O). [α]_{D} +13.57 (c 1.0 in 1:1 DCM-MeOH). ¹H NMR (500 MHz, CD₃OD): δ = 5.25 (d, 2 H, *J*_{1,2} = 3.7 Hz, H-1), 3.97 (d, 2 H, *J*_{5,6a} = 7.8 Hz, H-6a), 3.88 (m, 2 H, H-3', H-6'), 3.63 (t, 2 H, *J*_{2,3} = *J*_{3,4} = 9.4 Hz, H-3), 3.54 (m, 4 H, *J*_{6a,6b} = 12.2 Hz, H-6b, H-5, H-1'), 3.34 (dd, 2 H, H-2), 3.09 (m, 4 H, CH₂N), 1.48 (m, 4 H, CH₂), 1.24-0.80 (m, 30 H, CH₂, CH₂CH₃). ¹³C NMR (125.7 MHz, CD₃OD): δ = 104.2 (C-2'), 92.1 (C-1), 79.9 (C-5'), 76.4 (C-3'), 72.7 (C-4', C-6), 71.9 (C-3), 71.3 (C-5), 62.8 (C-2, C-1'), 46.5 (CH₂N), 31.6-13.2 (CH₂, CH₂CH₃). ESIMS: *m*/*z* = 683.42 [M + H]⁺. Anal. calcd. for C₃₀H₅₈N₄O₉S₂: C 52.76, H 8.56, N 8.20, S 9.39. Found C 52.45, H 8.41, N 7.97, S 9.11.

### Example 2 - Signaling Response of NFAT-GFP Mincle Reporter Cells to the Compounds.

### General methodology for evaluation of the capacity of glycolipids of the invention to promote Mincle-mediated GFP activation

The capacity of the compounds to act as Mincle agonists was evaluated using a reporter T-cell hybridoma that expresses Mincle and FcRγ as well as green fluorescent protein (GFP) under the control of the transcription factor nuclear factor of activated T-cells (NFAT) to detect ITAM-mediated signals. T-cell hybridoma Mincle and FcRy expressing NFAT-GFP reporter cells were obtained following reported protocols (Yamasaki et al., Nat. Immunol. 2008, 9, 1179-1188). Cells were cultured in 10% FBS RPMI 1640 Medium at 37°C, 5% CO₂.

The corresponding lyophilized glycolipids were dissolved in isopropyl alcohol (IPA) to a 2 mg/mL concentration and serially diluted using IPA. The multi-concentration compound collections were applied (20 µL) to 96-well cell culture plates. Plates were placed in a biosafety cabinet for 2 hours to evaporate to dryness. Mincle reporter cells were applied to the wells (200 µL/well) at a 3×10⁵ cells/mL density and incubated at 37°C, 5% CO₂ for 20-24 hours. After incubation and supernatant removal, FACS buffer (1xPBS, 0.1 % (w/v) BSA, 1 mM EDTA, 0.02% (w/v) NaN₃, pH 7.4) (200 µL) and propidium iodide (20 µL at 20 µg/mL) were added. Cells were resuspended by gentle pipetting and cellular GFP expression results were recorded using a Sony SA3800 spectral cell analyzer.

### Example 2a - Mincle agonist glycolipid fast screening evaluation assay.

**FIG. 1** shows the results on the Mincle mediated cell reporter activation along with GFP expression for compounds **TT1, TT2, TT3, TT4, TT5, TT6, TT7, TT8, TT9, TT10, TU2, TU3, TU5, TA1, GT1** and **ST1** compared to the results for trehalose-6,6-dimycolate (TDM; positive control) and isopropyl alcohol (IPA; negative control). The coating amount for TDM was 20 ng. The coating amount range for the candidate compounds was 250, 500, 1000 and 2000 ng. Several compounds generated a GFP positive Mincle reporter cell response, compatible with Mincle agonistic activity. Compounds **TT7, TT8, TT9** and **TU2** displayed a GFP expression value that surpassed the positive control TDM. Nevertheless, due to the considerable concentration difference with the control, this fast-screening comparative results need to be interpreted with caution.

### Example 2b - Compound concentration exploration analysis for GFP expression.

**FIG. 2** shows the results on the Mincle mediated cell reporter activation along with GFP expression for compounds (IPA), **TT1, TT2, TT3, TT4, TT5, TT6, TT7, TT8, TT9, TT10, TU2** and **TU3** compared to the results for trehalose-6,6-dimycolate (TDM; positive control) and isopropyl alcohol (IPA; negative control). The coating amount for TDM was 10 ng. The coating amount range for the candidate compounds was 62.5, 125, 250, 500, 1000 and 2000 ng.

The analysis conducted at lower concentrations (62.5 and 125 ng) displayed a decrease in the GFP positive cellular count in numerous compounds. However, compounds **TT4, TT5, TT7, TT8, TT9** and **TU3** did not show a meaningful disparity between these lower concentrations and the previous results.

### Example 2c - Fine structure-Mincle agonistic activity relationship analyses.

**FIGs. 3-5** show a comprehensive series of assays on the Mincle mediated cell reporter activation along with GFP expression for compounds **TT4, TT5, TT6, TT7, TT8, TU3** and **TU5 (****FIG. 4****), TU5, TT8, TU8, TT11, TU11, TT12** and **TU12 (****FIG. 3****),** and **TU5, TT13, TU13, TT14, TU14, TT15** and **TU15 (****FIG. 5****)** at concentrations comparable to that use for the positive control TDM (10 ng), in order to estimate structure-activity relationships. Isopropyl alcohol (IPA) was included as negative control. The coating amount range for the candidate compounds was 1, 5, 10 and 20 ng.

Compounds **TT7** and **TT8** at 10 ng presented similar Mincle reporter cells GFP activation to TDM at 10 ng however, an activation decrease can be observed when comparing these compounds and the positive control at 1 ng concentration. On the other hand, compounds **TT5** and **TU5** could not reach similar values at 20 ng coating but exhibited comparable results at 1 ng **(****FIG.3****).**

Compounds **TT8** and **TU8** showed similar GFP expression levels to the positive control and the previously examined **TU5** structure. Interestingly, it appears that TU8 presents a better GFP-positive cell profile than **TT8** at the lowest concentration **(****FIG. 4****).**

Compounds **TT15** and **TU15** showed close Mincle activation results compared with the positive control and the compound **TU5.** Furthermore, **TU15** displayed a higher activation rate than its counterpart **TT15 (****FIG. 5****).**

### Example 3 - In vitro and In vivo Inmmunomodulatory Activity of the Compounds.

### Materials

RPMI 1640, DMEM, newborn calf serum (NBCS), and red blood cell (RBC) lysis buffer were purchased from Gibco^{™} (Waltham, MA, USA). Fetal bovine serum (FBS) was purchased from HyClone (Logan, UT, USA). Recombinant mouse IL-2 were purchased from BioLegend (San Diego, CA, USA). Recombinant human IL-2 was purchased from eBioscience (San Diego, CA, USA). OVA257-264 peptide and trehalose-6,6-dibehenate (TDB) were purchased from InvivoGen (San Diego, CA, USA).

### Mice

Five- to ten-week-old C57BL/6 female mice were purchased from National Laboratory Animal Center (Taipei, Taiwan). All animals were housed under specific pathogen-free conditions.

### In vitro culture of human peripheral blood mononuclear cells (PBMCs)

Human peripheral blood mononuclear cells (PBMCs) from healthy volunteers were collected from whole blood through gradient-centrifugation with Histopaque-1077 (Sigma-Aldrich). Cells were maintained in RPMI 1640 medium supplemented with human recombinant IL-2 protein at 20ng/ml concentration.

### In vitro stimulation of Splenocytes, U937, THP-1, RAW246.7 cells

Stock solutions of the compounds were prepared in DMSO at 20mM concentration, and aliquots of these solutions were added to indicated medium to reach the desired target concentration. The final proportion of DMSO was <5% in all cases. Mouse splenocytes were isolated and stimulated with compounds for 24 h or 96 h. U937 and THP-1 cells were maintained in RPMI 1640 medium and need to be primed with PMA, 50ng/ml and 5ng/ml respectively, 48 h prior to the compound treatment. RAW264.7 cells were maintained in DMEM medium.

### OT-1 adjuvancy assay

The scheme of the OT-1 adoptive transfer mouse model for examining the proliferation of CD8+ T cells in adjuvanting OVA challenge is described in **Fig.11****.** Briefly, carboxyfluorescein succinimidyl ester (CFSE)-labeled splenocytes from OT-1 mice were intravenously transferred to C57BL/6 wildtype mice. 2 h later, the mice were immunized by intraperitoneal (i.p.) injection of OVA257-264 peptide (0.1 µg/mice) with indicated compound. Two days after the immunization, the proliferation of OT-1 CD8+ T cells were analyzed by flow cytometry.

### Flow cytometry

Single cell suspensions were stained with fixable viability dye eFluor^{®} 780 (eBioscience) for 30 mins at 4°C and Fc receptors were blocked with anti-CD16/32 (BioLegend) blocking antibody prior to surface staining with monoclonal antibodies. Antibodies used for OT-1 adjuvancy surface staining are as listed: mouse: anti-mouse CD45 (30-F11, BioLegend), anti-mouse CD3 (17A2, Biolegend), anti-mouse CD8 (53-6.7, Biolegend). For CFSE (Cayman) staining, cells were stained with 5 µM CFSE according to the manufacturer's protocol. Data were acquired on LSR II (BD Biosciences) and analyzed with FlowJo software (TreeStar).

### ELISA

Cytokines (mouse IL-1β, TNF-α, IFN-γ, IL-17; human IL-1β, TNF-α, IFN-γ, IL-6, IL-17) in culture supernatant were analyzed with ELISA kits from Biolegend, except for mouse IL-6 from Invitrogen. For determination of cytokine concentrations in vitro, supernatants from treated cells were collected after centrifugation and analyzed by capture ELISA according to the manufacturer's protocol.

### Statistics

Data were analyzed using the GraphPad Prism (GraphPad Software, San Diego, CA, USA). Statistical analysis was determined using the one-way analysis of variance (ANOVA).

### Example 3a - Immunostimulant function of compounds (Macrophage-like cells in vitro).

THP-1 and RAW264.7 cells were treated with the compounds of the invention alone to assess the immune-stimulating function of the compounds by measuring their ability to promote the secretion of IL-1β, IL-6, and TNF-α. In **Fig. 6A-C**, as a representative example, we found that compounds **TT8, TT15,** and **TU15** were effective in promoting the production of IL-1β, particularly for **TT15** with high IL-1β production. Compounds **TU5, TT15,** and **TU15** induced low-concentration production of TNF-α in THP-1 cells. We also found that all tested compounds induced low-concentration production of TNF-α in RAW264.7 cells. No IL-1β production was observed in RAW264.7 cells following treatment with the compounds. No IL-6 production was observed in either cell line following treatment with the compounds.

### Example 3b - In vitro immunostimulant function of compound TT15 in mouse splenocytes.

Mouse splenocytes were treated with compound **TT15** alone to assess its immune-stimulating function by measuring its ability to promote the secretion of IL-17, IL-1β, and TNF-α. In **Fig. 7****,** although **TT15** did not induce the production of IL-17 and IL-1β, it did induce low-concentration production of TNF-α, in contrast to the high-concentration production of TNF-α induced by the Mincle agonist TDB.

### Example 3c - In vitro immunostimulant function of compound TT15 in human macrophage-like cells and PBMCs.

Considering previous observations of higher immunostimulation by **TT15** in human cells as oppose to mouse cells *in vitro,* we further assessed the cytokine signature of **TT15** using human macrophage-like cell lines, U937 and THP-1, and PBMCs from volunteering donors. In **Fig. 8A-B**, we confirmed that, similar to its effects in THP-1 cells, **TT-15** promoted high-concentration IL-1β production in a dose-dependent manner in U937 cells, while it induced low-concentration of TNF-α, similar to that in THP-1 cells. Furthermore, **TT-15** was observed to induced a higher concentration of IL-1β compared to the same concentration of TDB. In **Fig. 9A-B**, a similar cytokine induction profile was observed in PBMCs from two donors, as well as the comparably better performance relative to TDB. As the secretion of IL-17 and IFNγ are the hallmarks of Mincle activation, we extended the incubation of **TT15** with PBMCs to 96 hours instead of the usual 24 hours incubation time and examined the effect. Accordingly, in **Fig. 10A-B**, we observed the secretion of IL-17 and IFNγ in PBMCs from two donors, as well as notably better induction by **TT15** compared to TDB.

### Example 3d - In vitro immunostimulant function of compound TT15 in human macrophage-like cells and PBMCs.

Compound **TT15** was injected in combination with the OVA₂₅₇₋₂₆₄ peptide into OT-1 mice (transgenic mice that express a TCR that is specific for a peptide derived from ovalbumin) to validate the Th1-biased immunomodulation property. As seen in **Fig. 11****,** compound **TT15** was observed to induce significant CD8⁺ T cell proliferation, indicating that compound **TT15** can promote Th1 immune response and has adjuvant functions.

## Claims

1. A compound of formula I: 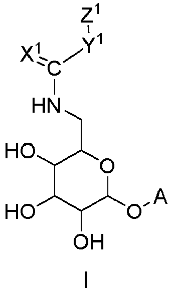 or a pharmaceutically acceptable salt thereof, wherein:
A is A¹, A², A³ or A⁴;
A¹ is selected from C₁-C₂₀ aliphatic, C₃-C₂₀ cycloaliphatic, C₅-C₁₄ aryl, 3- to 14-membered heterocyclyl comprising 1 to 3 heteroatoms selected from N, O, and S, wherein each A¹ is optionally substituted with one or more A^{a};
each A^{a} is independently selected from halogen, -N₃, -A^{b}, -OA^{b}, -SA^{b}, -NHA^{b}, -C(O)-A^{b}, -OC(O)A^{b}, -NHC(O)A^{b}, -C(O)NHA^{b}, -NHC(O)NHA^{b} and -NHC(S)NHA^{b}
each A^{b} is selected from H, C₁-C₂₀ aliphatic, C₃-C₂₀ cycloaliphatic, C₅-C₁₄ aryl, 3- to 14-membered heterocyclyl comprising 1 to 3 heteroatoms selected from N, O, and S and 4- to 14-membered heteroaryl comprising 1 to 3 heteroatoms selected from N, O, and S;
A², A³ and A⁴ are: 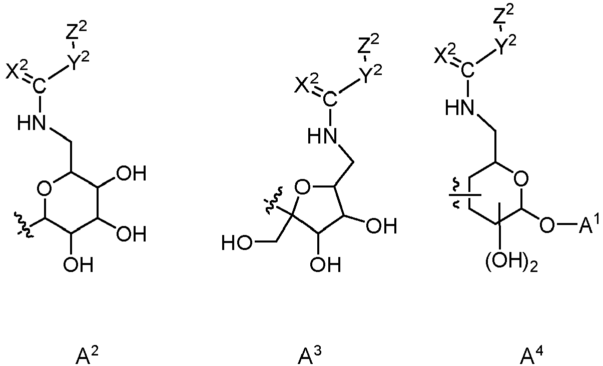
**X¹** and X² are independently selected from S and O;
**Y¹** and Y² are independently selected from -N(R^{a})-, -N(R^{a})-C(=O)- and -N(R^{a})-S(=O)₂-each R^{a} is independently selected from H, C₁-C₂₅ aliphatic, C₃-C₂₀ cycloaliphatic, C₅-C₁₄ aryl, 3- to 14-membered heterocyclyl comprising 1 to 3 heteroatoms selected from N, O, and S, wherein each R^{a} is optionally substituted with one or more R^{b};
each R^{b} is independently selected from halogen, -N₃, -R^{c}, -OR^{c}, -SR^{c}, -NHR^{c}, -C(O)-R^{c}, -OC(O)R^{c}, -NHC(O)R^{c}, -C(O)NHR^{c}, -NHC(O)NHR^{c} and NHC(S)NHR^{c};
each R^{c} is independently selected from H or optionally substituted C₁-C₂₀ aliphatic, C₃-C₂₀ cycloaliphatic, C₅-C₁₄ aryl, 3- to 14-membered heterocyclyl comprising 1 to 3 heteroatoms selected from N, O, and S, and 4- to 14-membered heteroaryl comprising 1 to 3 heteroatoms selected from N, O, and S;
**Z¹** and Z² are independently selected from C₄-C₂₅ aliphatic, C₃-C₂₀ cycloaliphatic, C₅-C₁₄ aryl, 3- to 14-membered heterocyclyl comprising 1 to 3 heteroatoms selected from N, O, and S, wherein Z¹ and Z² are optionally independently substituted with one or more Z³;
each Z³ is independently selected from halogen, -N₃, -R^{d}, -OC(O)R^{d}, -OR^{d}, -SR^{e}, - NHR^{e}, -C(O)-R^{e}, -NHC(O)R^{e}, -C(O)NHR^{e}, -NHC(O)NHR^{e} and NHC(S)NHR^{e};
each R^{d} is independently selected from optionally substituted C₂-C₂₀ aliphatic, C₃-C₂₀ cycloaliphatic, C₅-C₁₄ aryl, 3- to 14-membered heterocyclyl comprising 1 to 3 heteroatoms selected from N, O, and S, and 4- to 14-membered heteroaryl comprising 1 to 3 heteroatoms selected from N, O, and S;
each R^{e} is independently selected from H or optionally substituted C₁-C₂₀ aliphatic, C₃-C₂₀ cycloaliphatic, C₅-C₁₄ aryl, 3- to 14-membered heterocyclyl comprising 1 to 3 heteroatoms selected from N, O, and S, and 4- to 14-membered heteroaryl comprising 1 to 3 heteroatoms selected from N, O, and S;
for use as a vaccine adjuvant.

2. The compound for use, according to claim 1, wherein A is A¹.

3. The compound for use, according to claim 2, wherein A¹ is methyl.

4. The compound for use according to claim 1 wherein A is A².

5. The compound for use, according to claim 1, wherein A is A³ or A⁴.

6. The compound for use, according to claim 5, wherein A¹ of A⁴ is methyl.

7. The compound for use, according to any of the preceding claims, wherein each of Y¹ and Y² is N(R^{a})-, -N(R^{a})-C(=O)-, or -N(R^{a})-S(=O)₂-

8. The compound for use, according to claim 7, wherein R^{a} is C₁-C₂₅ aliphatic, optionally substituted with one or more R^{b}, preferably , R^{a} is Me.

9. The compound for use, according to any of the preceding claims, wherein Z¹ and Z² are each C₄-C₂₅ aliphatic optionally substituted with one or more Z³,

10. The compound for use, according to claim 9 wherein Z³ is selected from optionally substituted -R^{d}, -OR^{d}, -OC(O)R^{d} and -C(O)-R^{e}.

11. The compound for use, according to claim 10 wherein R^{d} is optionally substituted C₂-C₂₀ aliphatic and R^{e} is selected from H and optionally substituted C₁-C₂₀ aliphatic.

12. The compound for use, according to claim 1, wherein the compound is selected from:
| Name | Structure |
|---|---|
| TT1 | 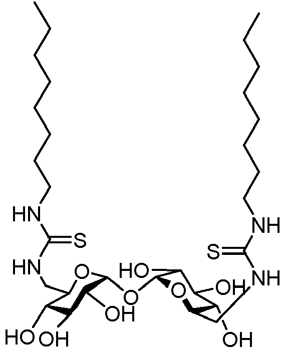 |
| TT2 | 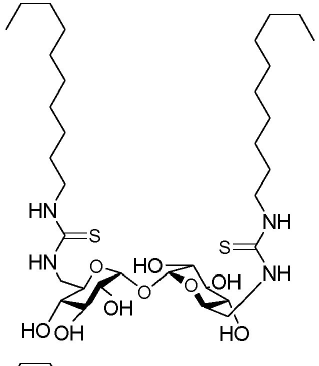 |
| TT3 | 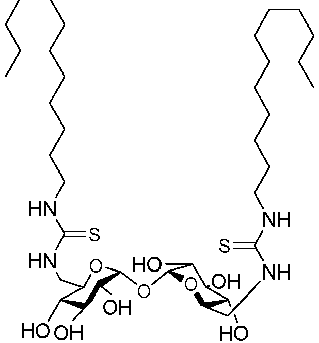 |
| TT4 | 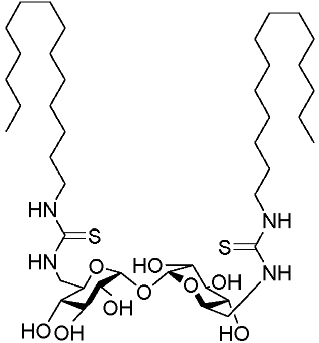 |
| TT5 | 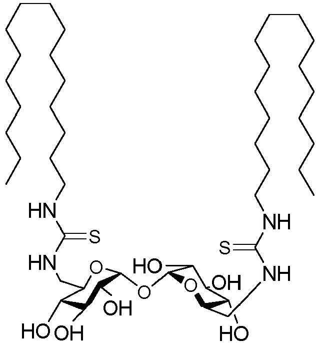 |
| TT6 | 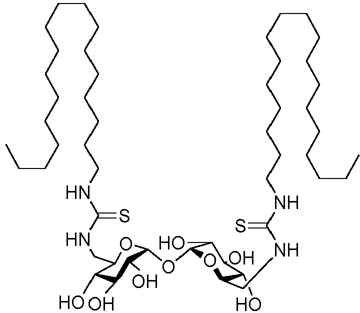 |
| TT7 | 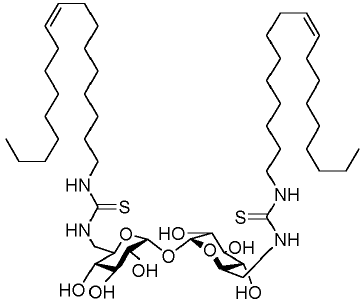 |
| TT8 | 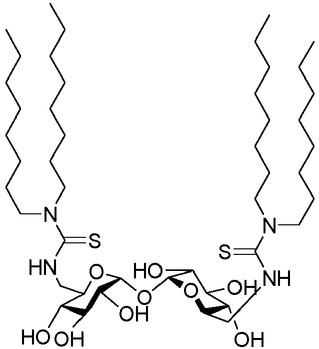 |
| TT9 | 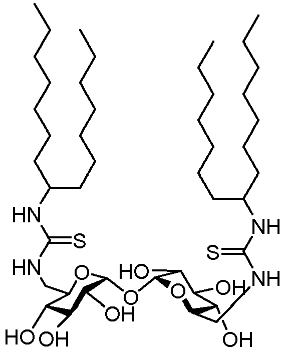 |
| TT10 | 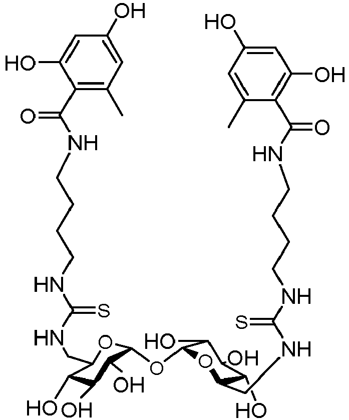 |
| TT11 | 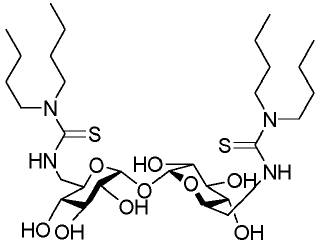 |
| TT12 | 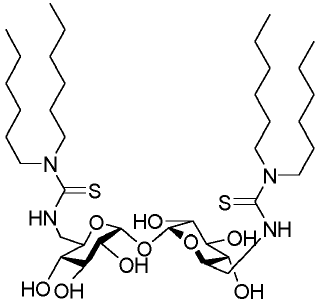 |
| TT13 | 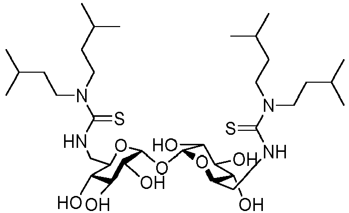 |
| TT14 | 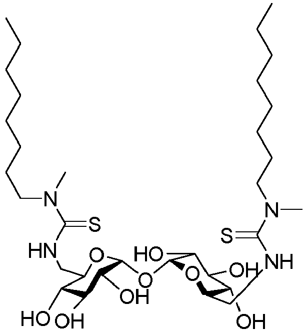 |
| TT15 | 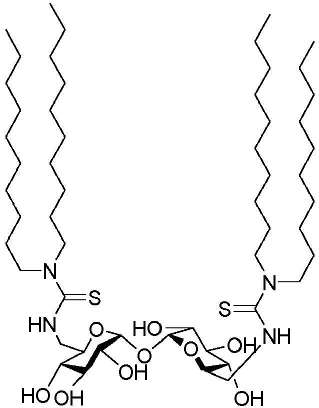 |
| TU2 | 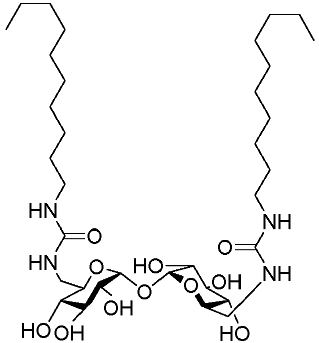 |
| TU3 | 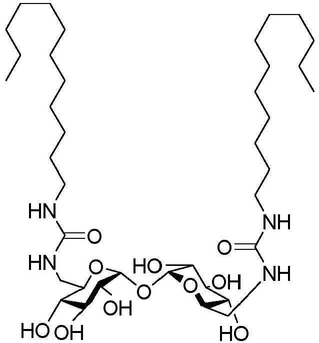 |
| TU5 | 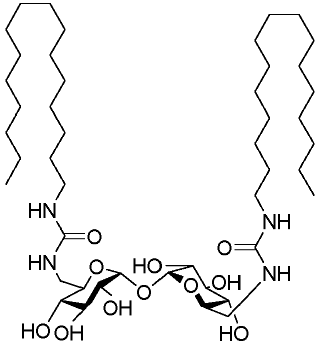 |
| TU8 | 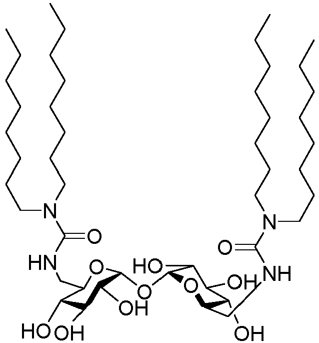 |
| TU11 | 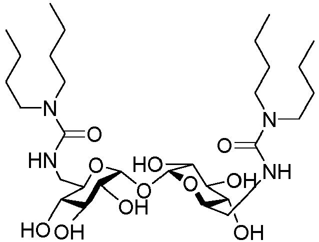 |
| TU12 | 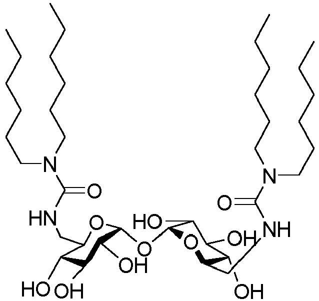 |
| TU13 | 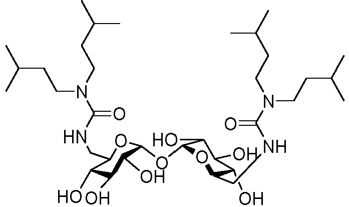 |
| TU14 | 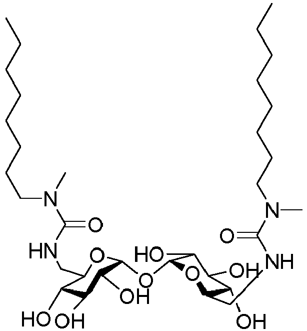 |
| TU15 | 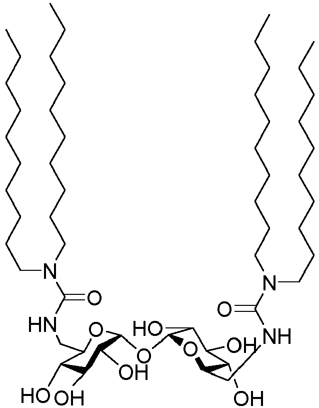 |
| GT1 | 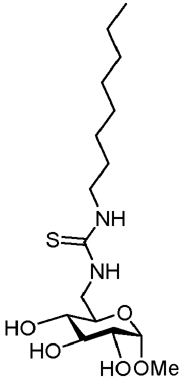 |
| GT5 | 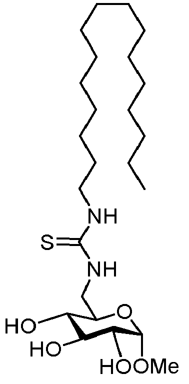 |
| GT8 | 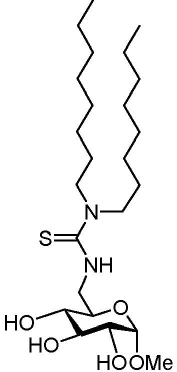 |
| GU5 | 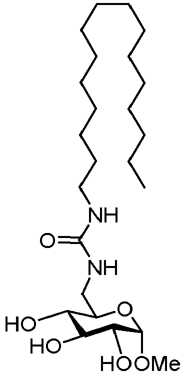 |
| GU8 | 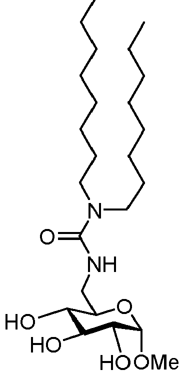 |
| ST1 | 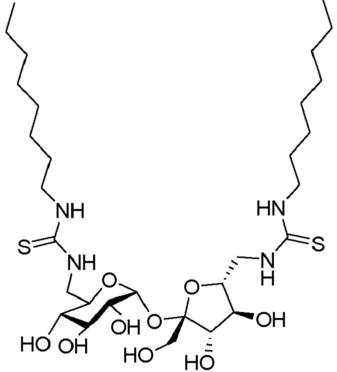 |
| ST5 | 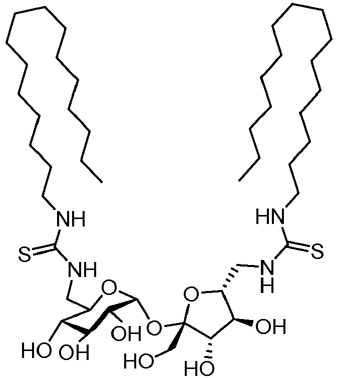 |
| ST8 | 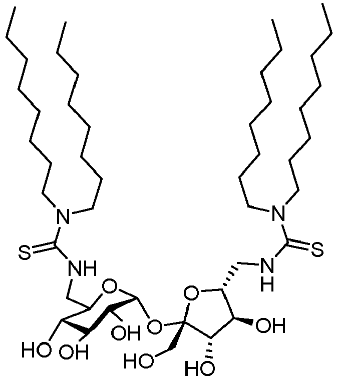 |
| SU5 | 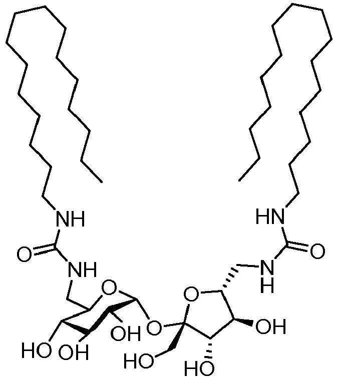 |
| SU8 | 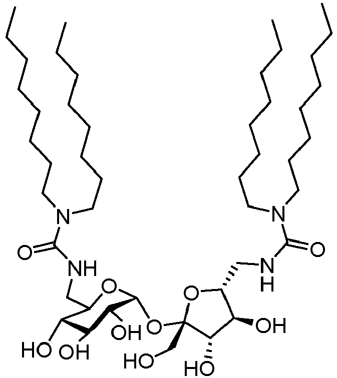 |
| MT5 | 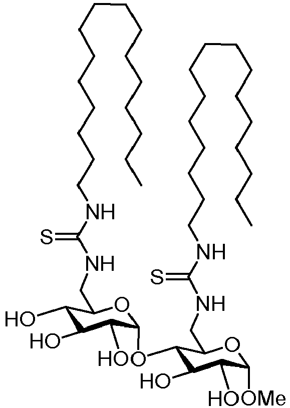 |
| MT8 | 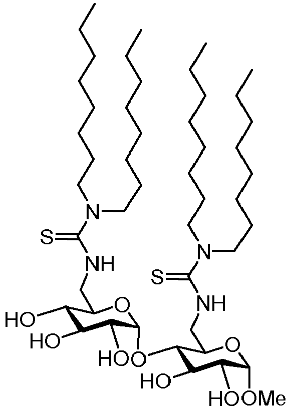 |
| MU5 | 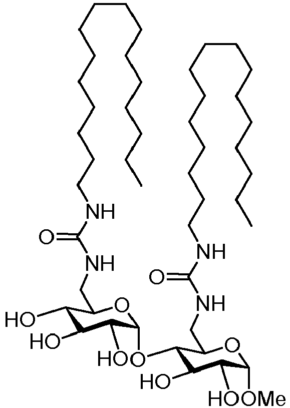 |
| MU8 | 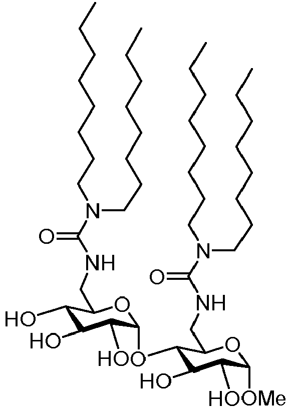 |

13. A compound of formula I 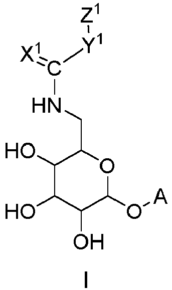 or a pharmaceutically acceptable salt thereof, wherein A, X¹, Y' and Z¹ are as defined in any of claims 1 to 12, provided that:
- when A is A², each X¹ and X² is O, each Y' and Y² is -N(R^{a})-, and each R^{a} is H, then each Z¹ and Z² is not selected from -(CH₂)₇CH₃ and -(CH₂)₈CH₃ and
- when A is methyl, X¹ is S, Y² is -N(R^{a})-, and R^{a} is H, then Z¹ is selected from C₆-C₂₅ aliphatic, C₃-C₂₀ cycloaliphatic, C₅-C₁₄ aryl, 3- to 14-membered heterocyclyl comprising 1 to 3 heteroatoms selected from N, O, and S, wherein Z¹ is optionally substituted with one or more Z³.

14. A method of preparing a compound of formula I as defined in claim 13 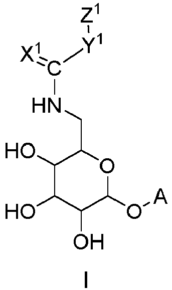 comprising contacting a compound of formula II: 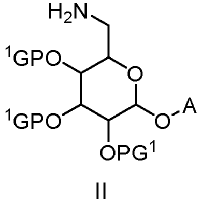 with a compound of formula III
X¹=C=N-Z¹ III
wherein A, X¹, Y¹, and Z¹ are as in the claim 14, and
each PG¹ is independently selected from H or a suitable oxygen protecting group that is removed in a next step or
comprising contacting a compound of formula IV: 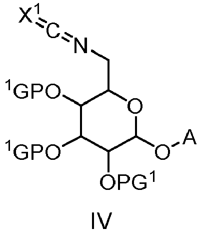
with a compound of formula V
HY¹-Z¹ V
wherein A, X¹, Y¹, and Z¹ are as in the preceding claims, and
each PG¹ is independently selected from a suitable oxygen protecting group that is removed in a next step.

15. A pharmaceutical composition comprising a therapeutically or prophylactically effective amount of a compound according to claim 13.

16. A pharmaceutical composition according to claim 15, further comprising pharmaceutically acceptable carriers, wherein the pharmaceutical composition is a vaccine.

17. Compound according to claim 13, or a pharmaceutical composition according to anyone of claims 15 to 16 for use as a medicament.

18. Compound according to claim 13, or a pharmaceutical composition according to anyone of claims 15 to 16, for use in the modulation of immune response in a subject.

19. Compound according to claim 13, or a pharmaceutical composition according to anyone of claims 15 to 16, for use in the treatment and/or prophylaxis of infectious diseases or in the treatment of cancer.
